# EUROPÄISCHE PATENTSCHRIFT

(11) **EP 2 619 188 B1**
(45) Veröffentlichungstag und Bekanntmachung des Hinweises auf die Patenterteilung: **06.05.2015**
(21) Anmeldenummer: 11757802.1
(22) Anmeldetag: 31.08.2011
(51) Int. Cl.: C07D 249/12, A61K 31/4196, A61P 9/00

(54) **SUBSTITUIERTE N-PHENETHYL-TRIAZOLONACETAMIDE UND IHRE VERWENDUNG**
SUBSTITUTED N-PHENETHYLTRIAZOLONEACETAMIDES AND USE THEREOF
N-PHÉNÉTHYL-TRIAZOLONACÉTAMIDES SUBSTITUÉS ET LEUR UTILISATION

(30) Priorität: 02.09.2010 DE 102010040187
(43) Veröffentlichungstag der Anmeldung: 31.07.2013
(73) Patentinhaber: Bayer Intellectual Property GmbH, 40789 Monheim (DE)
(72) Erfinder: FÜRSTNER, Chantal, 45478 Mülheim/Ruhr (DE); KELDENICH, Jörg, 42113 Wuppertal (DE); DELBECK, Martina, 42579 Heiligenhaus (DE); KOLKHOF, Peter, 42113 Wuppertal (DE); KRETSCHMER, Axel, 42113 Wuppertal (DE); POOK, Elisabeth, 42109 Wuppertal (DE); SCHMECK, Carsten, 45472 Mülheim/Ruhr (DE); TRÜBEL, Hubert, 42281 Wuppertal (DE)
(74) Vertreter: BIP Patents
(86) Internationale Anmeldenummer: PCT/EP2011/065000
(87) Internationale Veröffentlichungsnummer: WO 2012/028644

(56) Entgegenhaltungen:
- WO-A1-2006/117657
- WO-A1-2007/134862
- WO-A1-2011/023703
- DE-A1-102008 060 967

## Beschreibung

Die vorliegende Anmeldung betrifft neue substituierte *N*-Phenethyl-triazolonacetamide, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von kardiovaskulären Erkrankungen.

Der Flüssigkeitsgehalt des menschlichen Körpers unterliegt verschiedenen physiologischen Kontrollmechanismen, die auf seine Konstanterhaltung abzielen (Volumenhomöostase). Dabei werden sowohl die Volumenfüllung des Blutgefäßsystems als auch die Osmolarität des Blutplasmas kontinuierlich von entsprechenden Sensoren (Barorezeptoren und Osmorezeptoren) registriert. Die Informationen, die diese Sensoren an die zuständigen Zentren des Hirns liefern, regulieren das Trinkverhalten und steuern vermittels humoraler und nervaler Signale die Flüssigkeitsausscheidung über die Nieren. Eine zentrale Bedeutung kommt hierbei dem Peptidhormon Vasopressin zu [Schrier R.W., Abraham, W.T., New Engl. J. Med. 341, 577-585 (1999)].

Vasopressin wird in spezialisierten, endokrinen Neuronen im *Nucleus supraopticus* und *N. para-ventricularis* in der Wand des dritten Ventrikels (Hypothalamus) produziert und von dort entlang ihrer Nervenfortsätze in den Hypophysenhinterlappen (Neurohypophyse) verbracht. Dort wird das Hormon je nach Stimulus in die Blutbahn abgegeben. Ein Volumenverlust, z.B. infolge akuter Blutung, starkem Schwitzen, langem Dursten oder Diarrhöe, ist ein Stimulus für die verstärkte Ausschüttung des Hormons. Umgekehrt wird die Sekretion von Vasopressin durch eine Erhöhung des Intravasalvolumens, z.B. infolge gesteigerter Flüssigkeitszufuhr, gehemmt.

Vasopressin entfaltet seine Wirkung hauptsächlich über Bindung an drei Rezeptoren, die als V1a-, V1b- und V2-Rezeptoren klassifiziert werden und zur Familie der G-Protein-gekoppelten Rezeptoren gehören. V1a-Rezeptoren sind vorwiegend auf den Zellen der glatten Gefäßmuskulatur lokalisiert. Ihre Aktivierung ruft eine Vasokonstriktion hervor, wodurch der periphere Widerstand und Blutdruck ansteigen. Daneben sind V1a-Rezeptoren auch in der Leber nachweisbar. V1b-Rezeptoren (auch V3-Rezeptoren genannt) sind im zentralen Nervensystem nachweisbar. Zusammen mit dem Corticotropin-Releasing-Hormon (CRH) reguliert Vasopressin über den V1b-Rezeptor die basale und stressinduzierte Sekretion des Adrenocorticotropen Hormons (ACTH). V2-Rezeptoren finden sich im distalen Tubulusepithel und dem Epithel der Sammelrohre der Niere. Ihre Aktivierung macht diese Epithelien für Wasser durchlässig. Diesem Phänomen liegt der Einbau von Aquaporinen (speziellen Wasserkanälen) in die luminale Membran der Epithelzellen zugrunde.

Welche Bedeutung Vasopressin für die Rückresorption von Wasser aus dem Harn in der Niere hat, wird durch das Krankheitsbild des Diabetes insipidus deutlich, das durch einen Mangel des Hormons - z.B. infolge einer Hypophysenschädigung - hervorgerufen wird. Patienten, die an diesem Krankheitsbild leiden, scheiden bis zu 20 Liter Harn pro 24 Stunden aus, sofern ihnen das Hormon nicht substituiert wird. Dieses Volumen entspricht in etwa 10% des Primärharns. Wegen seiner großen Bedeutung für die Rückresorption von Wasser aus dem Harn wird Vasopressin auch synonym als Antidiuretisches Hormon (ADH) bezeichnet. Folgerichtig führt eine pharmakologische Hemmung der Vasopressin/ADH-Wirkung am V2-Rezeptor zu einer vermehrten Urinausscheidung. Im Gegensatz zu der Wirkung anderer Diuretika (Thiazide und Schleifendiuretika) jedoch bewirken V2-Rezeptor-Antagonisten eine vermehrte Wasserausscheidung, ohne die Ausscheidung von Elektrolyten maßgeblich zu steigern. Das bedeutet, dass durch V2-antagonistische Pharmaka die Volumenhomöostase wiederhergestellt werden kann, ohne dabei in die Elektrolyt-Homöostase einzugreifen. Daher erscheinen V2-antagonistisch wirksame Pharmaka besonders geeignet für die Behandlung aller krankhaften Zustände, die mit einer Überfrachtung des Organismus mit Wasser einhergehen, ohne dass parallel die Elektrolyte adäquat erhöht sind. Eine bedeutende Elektrolyt-Abnormalität ist klinisch-chemisch als Hyponatriämie (Natriumkonzentration < 135 mmol/L) messbar; sie stellt die wichtigste Elektrolyt-Abnormalität bei Krankenhauspatienten dar mit einer Häufigkeit von ca. 5% bzw. 250.000 Fällen pro Jahr allein in den USA. Bei einem Absinken der Plasma-Natriumkonzentration unter 115 mmol/L drohen komatöse Zustände und Tod.

Entsprechend der zugrunde liegenden Ursache unterscheidet man die hypovolämische, euvolämische und hypervolämische Hyponatriämie. Klinisch bedeutsam sind die Formen der Hypervolämie mit Ödembildung. Typische Beispiele hierfür sind das Syndrom der inadäquaten ADH/Vasopressin-Sekretion (SIAD) (z.B. nach Schädel-Hirn-Trauma oder als Paraneoplasie bei Carcinomen) und die hypervolämische Hyponatriämie bei Leberzirrhose, verschiedenen Nierenerkrankungen und Herzinsuffizienz [De Luca L. et al., Am. J. Cardiol. 96 (suppl.), 19L-23L (2005)]. Gerade Patienten mit Herzinsuffizienz weisen trotz ihrer relativen Hyponatriämie und Hypervolämie häufig erhöhte Vasopressin-Spiegel auf, was als die Folge einer generell gestörten neurohumoralen Regulation bei der Herzinsuffizienz angesehen wird [Francis G.S. et al., Circulation 82, 1724-1729 (1990)].

Die gestörte neurohumorale Regulation manifestiert sich im Wesentlichen in einer Erhöhung des Sympathicotonus und einer inadäquaten Aktivierung des Renin-Angiotensin-Aldosteron-Systems. Während die Hemmung dieser Komponenten durch Beta-Rezeptoren-Blocker einerseits und durch ACE-Inhibitoren bzw. Angiotensin-Rezeptor-Blocker andererseits heute fester Bestandteil der pharmakologischen Behandlung der Herzinsuffizienz ist, ist die inadäquate Steigerung der Vasopressin-Sekretion in der fortgeschrittenen Herzinsuffizienz derzeit noch nicht ausreichend therapierbar.

Neben der durch V2-Rezeptoren vermittelten Retention von Wasser und den damit verbundenen ungünstigen hämodynamischen Folgen im Sinne einer Nachlasterhöhung werden auch durch V1a-vermittelte Vasokonstriktion die Entleerung des linken Ventrikels, der Druck in den Pulmonalgefäßen und die Herzleistung negativ beeinflusst. Darüber hinaus wird aufgrund tierexperimenteller Daten dem Vasopressin auch eine direkte Hypertrophie-fördernde Wirkung am Herzmuskel beigemessen. Im Unterschied zur renalen Wirkung der Volumenexpansion, die über Aktivierung von V2-Rezeptoren vermittelt ist, wird die direkte Wirkung am Herzmuskel durch Aktivierung von V1a-Rezeptoren ausgelöst.

Aus diesen Gründen erscheinen Substanzen, die die Wirkung des Vasopressins am V2- und/oder am V1a-Rezeptor hemmen, zur Behandlung der Herzinsuffizienz geeignet. Vor allem Verbindungen mit kombinierter Aktivität an beiden Vasopressin-Rezeptoren (V1a und V2) sollten sowohl wünschenswerte renale als auch hämodynamische Effekte bewirken und somit ein besonders ideales Profil für die Behandlung von Patienten mit Herzinsuffizienz bieten. Die Bereitstellung derartig kombinierter Vasopressin-Antagonisten erscheint auch insofern sinnvoll, als ein allein über V2-Rezeptor-Blockade vermittelter Volumenentzug die Erregung von Osmorezeptoren und in der Folge eine weitere kompensatorische Steigerung der Vasopressin-Ausschüttung nach sich ziehen kann. Hierdurch könnten - bei Fehlen einer gleichzeitig den V1a-Rezeptor blockierenden Komponente - die schädlichen Wirkungen des Vasopressins, wie z.B. Vasokonstriktion und Herzmuskelhypertrophie, noch verstärkt werden [Saghi P. et al., Europ. Heart J. 26, 538-543 (2005)].

Aufgabe der vorliegenden Erfindung war daher die Bereitstellung neuer Verbindungen, die als potente selektive V1a-, V2- oder duale V1a/V2-Rezeptor-Antagonisten wirken und als solche zur Behandlung und/oder Prävention von Krankheiten, insbesondere zur Behandlung und/oder Prävention von kardiovaskulären Erkrankungen geeignet sind.

In WO 99/31099-A1 werden verschiedenartig substituierte 1,2,4-Triazolone als therapeutisch nutzbare Integrin-Rezeptor-Antagonisten offenbart. Die Verwendung von 5-Aryl-1,2,4-triazolonen als Arzneimittel mit neuroprotektiver Wirkung wurde in WO 99/54315-A2 beansprucht, und in WO 2006/117657-A1 werden 4,5-Diaryltriazolon-Derivate als anti-inflammatorische Agentien beschrieben. Aus WO 2005/105779-A1 sind 3-Heterocyclyl-4-phenyltriazole als Inhibitoren des Vasopressin-V1A-Rezeptors bekannt, und in WO 2007/134862-A1 werden amidisch verknüpfte 5-Aryl-1,2,4-triazolone als duale Vasopressin-Antagonisten offenbart.

Gegenstand der vorliegenden Erfindung sind Verbindungen der allgemeinen Formel (I) 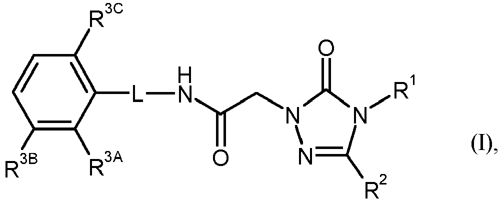 in welcher
- R¹: für (C₁-C₆)-Alkyl, (C₂-C₆)-Alkenyl oder (C₂-C₆)-Alkinyl steht, welche jeweils ein- oder zweifach, gleich oder verschieden, mit Resten ausgewählt aus der Reihe Fluor, Chlor, Cyano, Difluormethyl, Trifluormethyl, Oxo, Hydroxy, Difluormethoxy, Trifluormethoxy, (C₁-C₄)-Alkoxy, (C₃-C₆)-Cycloalkyl und Phenyl substituiert sein können,
wobei (C₃-C₆)-Cycloalkyl bis zu zweifach, gleich oder verschieden, mit Resten ausgewählt aus der Reihe Fluor, Trifluormethyl, (C₁-C₄)-Alkyl, Oxo, Hydroxy, Trifluormethoxy und (C₁-C₄)-Alkoly substituiert sein kann
und
wobei Phenyl bis zu dreifach, gleich oder verschieden, mit Resten ausgewählt aus der Reihe Halogen, Cyano, Difluormethyl, Trifluormethyl, (C₁-C₄)-Alkyl, Hydroxy, Hydroxymethyl, Difluormethoxy, Trifluormethoxy, (C₁-C₄)-Alkoₗy, (C₁-C₄)-Alkoxymethyl, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
oder
für (C₃-C₆)-Cycloalkyl steht, welches ein- oder zweifach, gleich oder verschieden, mit Resten ausgewählt aus der Reihe Fluor, Trifluormethyl, (C₁-C₄)-Alkyl, Oxo, Hydroxy, Trifluormethoxy und (C₁-C₄)-Alkoxy substituiert sein kann,
- R²: für Phenyl oder Thienyl steht, welche ein- oder zweifach, gleich oder verschieden, mit Resten ausgewählt aus der Reihe Halogen, Cyano, Difluormethyl, Trifluormethyl, (C₁-C₄)-Alkyl, Hydroxy, Trifluormethoxy und (C₁-C₄)-Alkoxy substituiert sein können,
- R^{3A}, R^{3B} und R^{3C}: unabhängig voneinander für Wasserstoff, Fluor, Chlor, Difluormethyl, Trifluormethyl, (C₁-C₄)-Alkyl, Difluormethoxy, Trifluormethoxy oder (C₁-C₄)-Alkoₗy stehen,
wobei jedoch mindestens einer der Reste R^{3A}, R^{3B}, R^{3C} von Wasserstoff verschieden ist,
und
- L: für eine Gruppe der Formel 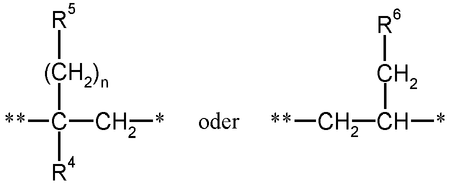 steht, worin
* die Verknüpfungsstelle mit dem angrenzenden N-Atom und
** die Verknüpfungsstelle mit dem Phenyl-Ring kennzeichnen,
n die Zahl 0, 1 oder 2 bedeutet,
R⁴ Wasserstoff oder Methyl bedeutet,
R⁵ eine Gruppe der Formel 1 -O-C(=O)-NR^{7A}R^{7B}, -NR⁸-C(=O)-NR^{7A}R^{7B}, -NR⁸-SO₂NR^{7A}R^{7B}, -NR⁸-C(=O)-R⁹, -NR⁸-SO₂-R¹⁰ oder -NR⁸-C(=O)-OR¹⁰ bedeutet, worin
R^{7A} und R^{7B} unabhängig voneinander Wasserstoff, (C₁-C₆)-Alkyl oder (C₃-C₆)-Cycloalkyl darstellen oder zusammen mit dem Stickstoffatom, an das sie beide gebunden sind, einen 4- bis 6-gliedrigen Heterocyclus bilden, der ein weiteres Ring-Heteroatom aus der Reihe N, O oder S enthalten kann und der ein- oder zweifach, gleich oder verschieden, mit Resten ausgewählt aus der Reihe Fluor, Trifluormethyl, (C₁-C₄)-Alkyl, Hydroxy und Oxo substituiert sein kann,
R⁸ Wasserstoff oder (C₁-C₄)-Alkyl darstellt,
R⁹ Wasserstoff, (C₁-C₆)-Alkyl oder (C₃-C₆)-Cycloalkyl darstellt, und
R¹⁰ (C₁-C₆)-Alkyl oder (C₃-C₆)-Cycloalkyl darstellt,
und
- R⁶: die oben angegebene Bedeutung von R⁵ hat oder Hydroxy bedeutet,

sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, *N*,*N*-Diisopropylethylamin, Monoethanolamin, Diethanolamin, Trisethanolamin, Dimethylaminoethanol, Diethylaminoethanol, Procain, Dicyclohexylamin, Dibenzylamin, *N*-Methylpiperidin, *N*-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper auf beispielsweise metabolischem oder hydrolytischem Wege zu erfindungsgemäßen Verbindungen umgesetzt werden.

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec*.-Butyl, *tert*.-Butyl, *n*-Pentyl, 2-Pentyl, 3-Pentyl, Neopentyl, *n*-Hexyl, 2-Hexyl und 3-Hexyl.
(C₂-C₆)-Alkenyl und (C₂-C₄)-Alkenyl stehen im Rahmen der Erfindung für einen geradkettigen oder verzweigten, eine Doppelbindung enthaltenden Alkenylrest mit 2 bis 6 bzw. 2 bis 4 Kohlenstoffatomen. Bevorzugt ist ein geradkettiger Alkenylrest mit 2 bis 4 Kohlenstoffatomen. Beispielhaft und vorzugsweise seien genannt: Vinyl, *n*-Prop-1-en-1-yl, Allyl, Isopropenyl, 2-Methyl-2-propen-1-yl, *n-*But-1-en-1-yl, *n*-But-2-en-1-yl, *n*-But-3-en-1-yl, *n*-Pent-1-en-1-yl, *n*-Pent-2-en-1-yl, *n*-Pent-3-en-1-yl, *n*-Pent-4-en-1-yl, 3-Methylbut-2-en-1-yl und 4-Methylpent-3-en-1-yl.
(C₂-C₆)-Alkinyl steht im Rahmen der Erfindung für einen geradkettigen oder verzweigten Alkinylrest mit 2 bis 6 Kohlenstoffatomen und einer Dreifachbindung. Bevorzugt ist ein geradkettiger oder verzweigter Alkinylrest mit 3 bis 6 Kohlenstoffatomen. Beispielhaft und vorzugsweise seien genannt: Ethinyl, *n*-Prop-1-in-1-yl, *n*-Prop-2-in-1-yl, *n*-But-2-in-1-yl, *n*-But-3-in-1-yl, *n*-Pent-2-in-1-yl, *n-*Pent-3-in-1-yl und *n*-Pent-4-in-1-yl.
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n-*Propoxy, Isopropoxy, *n*-Butoxy, *iso*-Butoxy, *sec*.-Butoxy und *tert*.-Butoxy.
(C₁-C₄)-Alkoxymethyl steht im Rahmen der Erfindung für einen geradkettigen oder verzweigten Alkoxyrest mit 1 bis 4 Kohlenstoffatomen, der über eine an das O-Atom gebundene MethylenGruppe [-CH₂-] mit dem Rest des Moleküls verknüpft ist. Beispielhaft und vorzugsweise seien genannt: Methoxymethyl, Ethoxymethyl, *n*-Propoxymethyl, Isopropoxymethyl, *n*-Butoxymethyl und *tert*.-Butoxymethyl.
(C₁-C₄)-Alkoxycarbonyl steht im Rahmen der Erfindung für einen geradkettigen oder verzweigten Alkoxyrest mit 1 bis 4 Kohlenstoffatomen, der über eine an das O-Atom gebundene Carbonyl-Gruppe [-C(=O)-] mit dem Rest des Moleküls verknüpft ist. Beispielhaft und vorzugsweise seien genannt: Methoxycarbonyl, Ethoxycarbonyl, *n*-Propoxycarbonyl, Isopropoxycarbonyl, *n*-Butoxy-carbonyl und *tert*.-Butoxycarbonyl.
Mono-(C₁-C₄)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n-*Butylamino und *tert*.-Butylamino.
Di-(C₁-C₄-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N*-Dimethylamino, *N,N-*Diethylamino, *N*-Ethyl-*N*-methylainino, *N*-Methyl-*N*-*n*-propylamino, *N*-Isopropyl-*N*-methylamino, *N*-Isopropyl-*N*-*n*-propylainino, *N*,*N*-Diisopropylamino, *N*-*n*-Butyl-*N*-methylamino, *N,N-*Di-*n*-butyl-amino und *N*-*tert*.-Butyl-*N*-methylamino.
Mono- bzw. Di-(C₁-C₄)-alkylaminocarbonyl steht im Rahmen der Erfindung für eine Amino-Gruppe, die über eine Carbonyl-Gruppe [-C(=O)-] mit dem Rest des Moleküls verknüpft ist und die einen geradkettigen oder verzweigten bzw. zwei gleiche oder verschiedene geradkettige oder verzweigte *N-*Alkylsubstituenten mit jeweils 1 bis 4 Kohlenstoffatomen aufweist. Beispielhaft und vorzugsweise seien genannt: Methylaminocarbonyl, Ethylaminocarbonyl, *n*-Propylaminocarbonyl, Isopropylaminocarbonyl, *n*-Butylaminocarbonyl, *tert*.-Butylaminocarbonyl, *N*,*N*-Dimethylaminocarbonyl, *N,N-*Diethylaminocarbonyl, *N*-Ethyl-*N*-methylaminocarbonyl, *N*-Methyl-*N*-*n*-propylaminocarbonyl, *N*,*N*-Diisopropylaminocarbonyl, *N*-*n*-Butyl-*N*-methylaminocarbonyl und *N-tert.*-Butyl-*N-*methylaminocarbonyl.
(C₃-C₆)-Cycloalkyl und (C₃-C₅)-Cycloalkyl stehen im Rahmen der Erfindung für eine monocyclische, gesättigte Cycloalkylgruppe mit 3 bis 6 bzw. 3 bis 5 Kohlenstoffatomen. Beispielhaft und vorzugsweise seien genannt: Cyclopropyl, Cyclobutyl, Cyclopentyl und Cyclohexyl.
Ein 4- bis 6-gliedriger Heterocyclus steht im Rahmen der Erfindung für einen monocyclischen, gesättigten Heterocyclus mit insgesamt 4 bis 6 Ringatomen, der ein Ring-Stickstoffatom enthält, über das er mit dem Rest des Moleküls verknüpft ist, und der darüber hinaus ein weiteres Ring-Heteroatom aus der Reihe N, O oder S enthalten kann. Beispielhaft und vorzugsweise seien genannt: Azetidinyl, Pyrrolidinyl, Pyrazolidinyl, 1,3-Oxazolidinyl, 1,3-Thiazolidinyl, Piperidinyl, Piperazinyl, Morpholinyl und Thiomorpholinyl. Bevorzugt sind Azetidinyl, Pyrrolidinyl, Piperidinyl und Morpholinyl.
Halogen schließt im Rahmen der Erfindung Fluor, Chlor, Brom und Iod ein. Bevorzugt sind Chlor, Fluor oder Brom, besonders bevorzugt Fluor oder Chlor.
Ein Oxo-Substituent steht im Rahmen der Erfindung für ein Sauerstoffatom, das über eine Doppelbindung an ein Kohlenstoffatom gebunden ist.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei oder drei gleichen oder verschiedenen Substituenten ist bevorzugt. Besonders bevorzugt ist die Substitution mit einem oder mit zwei gleichen oder verschiedenen Substituenten. Ganz besonders bevorzugt ist die Substitution mit einem Substituenten.

Eine bevorzugte Ausführungsform der vorliegenden Erfindung umfasst Verbindungen der Formel (I), in welcher
- R¹: für (C₁-C₄)-Alkyl oder (C₂-C₄)-Alkenyl steht, welche jeweils ein- oder zweifach, gleich oder verschieden, mit Resten ausgewählt aus der Reihe Fluor, Trifluormethyl, Hydroxy, Trifluormethoxy und (C₁-C₄)-Alkoxy substituiert sein können,
oder
für Benzyl steht, welches im Phenylring ein- oder zweifach, gleich oder verschieden, mit Resten ausgewählt aus der Reihe Fluor, Chlor, Trifluormethyl, (C₁-C₄)-Alkyl, Trifluormethoxy und (C₁-C₄)-Alkoₗy substituiert sein kann,
oder
für (C₃-C₅)-Cycloalkyl steht,
sowie ihre Salze, Solvate und Solvate der Salze.

Eine gleichfalls bevorzugte Ausführungsform der vorliegenden Erfindung umfasst Verbindungen der Formel (I), in welcher
- R²: für Phenyl oder Thienyl steht, welche mit einem Rest ausgewählt aus der Reihe Fluor, Chlor, Cyano, Methyl, Difluormethyl, Trifluormethyl, Methoxy und Trifluormethoxy substituiert sind,
sowie ihre Salze, Solvate und Solvate der Salze.

Eine weitere bevorzugte Ausführungsform der vorliegenden Erfindung umfasst Verbindungen der Formel (I), in welcher
- R^{3A}, R^{3B} und R^{3C}: unabhängig voneinander für Wasserstoff, Fluor, Chlor, Methyl, Difluormethyl, Trifluormethyl, Methoxy oder Trifluormethoxy stehen, wobei jedoch mindestens einer der Reste R^{3A}, R^{3B}, R^{3C} von Wasserstoff verschieden ist,
sowie ihre Salze, Solvate und Solvate der Salze.

Eine weitere bevorzugte Ausführungsform der vorliegenden Erfindung umfasst Verbindungen der Formel (I), in welcher
- R^{3C}: für Wasserstoff steht,
sowie ihre Salze, Solvate und Solvate der Salze.

Eine weitere bevorzugte Ausführungsform der vorliegenden Erfindung umfasst Verbindungen der Formel (I), in welcher
- L: für eine Gruppe der Formel 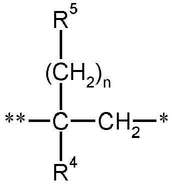 steht, worin
* die Verknüpfungsstelle mit dem angrenzenden N-Atom und
** die Verknüpfungsstelle mit dem Phenyl-Ring kennzeichnen,
n die Zahl 0 oder 1 bedeutet,
R⁴ Wasserstoff oder Methyl bedeutet,
und
- R⁵: eine Gruppe der Formel -O-C(=O)-NR^{7A}R^{7B}, -NH-C(=O)-NR^{7A}R^{7B}, -NH-C(=O)-R⁹, -NH-SO₂-R¹⁰ oder -NH-C(=O)-OR¹⁰ bedeutet, worin
R^{7A} und R^{7B} unabhängig voneinander Wasserstoff oder (C₁-C₄)-Alkyl darstellen,
R⁹ Wasserstoff oder (C₁-C₄)-Alkyl darstellt, und
R¹⁰ (C₁-C₄)-Alkyl darstellt,
sowie ihre Salze, Solvate und Solvate der Salze.

Eine weitere bevorzugte Ausführungsform der vorliegenden Erfindung umfasst Verbindungen der Formel (I), in welcher
- L: für eine Gruppe der Formel 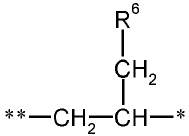 steht, worin
* die Verknüpfungsstelle mit dem angrenzenden N-Atom und
** die Verknüpfungsstelle mit dem Phenyl-Ring kennzeichnen, und
R⁶ Hydroxy oder eine Gruppe der Formel -O-C(=O)-NR^{7A}R^{7B} bedeutet, worin R^{7A} und R^{7B} unabhängig voneinander Wasserstoff oder (C₁-C₄)-Alkyl darstellen,
sowie ihre Salze, Solvate und Solvate der Salze.

Besonders bevorzugt im Rahmen der vorliegenden Erfindung sind Verbindungen der Formel (I), in welcher
- R¹: für (C₁-C₄)-Alkyl oder (C₂-C₄)-Alkenyl steht, welche jeweils ein- oder zweifach, gleich oder verschieden, mit Resten ausgewählt aus der Reihe Fluor, Trifluormethyl, Hydroxy, Methoxy und Ethoxy substituiert sein können,
oder
für Benzyl steht, welches im Phenylring mit einem Rest ausgewählt aus der Reihe Fluor, Chlor, Methyl, Trifluormethyl und Methoxy substituiert sein kann,
oder
für Cyclopropyl steht,
- R²: für Phenyl oder Thienyl steht, welche mit einem Rest ausgewählt aus der Reihe Fluor und Chlor substituiert sind,
- R^{3A} und R^{3B}: unabhängig voneinander für Wasserstoff, Fluor, Chlor, Methyl, Trifluormethyl, Methoxy oder Trifluormethoxy stehen,
wobei jedoch mindestens einer der Reste R^{3A} und R^{3B} von Wasserstoff verschieden ist,
- R^{3C}: für Wasserstoff steht,
und
- L: für eine Gruppe der Formel 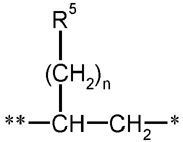 steht, worin
* die Verknüpfungsstelle mit dem angrenzenden N-Atom und
** die Verknüpfungsstelle mit dem Phenyl-Ring kennzeichnen,
n die Zahl 0 oder 1 bedeutet, und
R⁵ eine Gruppe der Formel -O-C(=O)-NHR^{7B}, -NH-C(=O)-NHR^{7B}, -NH-C(=O)-R⁹, -NH-SO₂-R¹⁰ oder -NH-C(=O)-OR¹⁰ bedeutet, worin
R^{7B} Wasserstoff oder (C₁-C₄)-Alkyl darstellt,
R⁹ Wasserstoff oder (C₁-C₄)-Alkyl darstellt, und
R¹⁰ (C₁-C₄)-Alkyl darstellt,
sowie ihre Salze, Solvate und Solvate der Salze.

Ganz besonders bevorzugt im Rahmen der vorliegenden Erfindung sind Verbindungen der Formel (I), in welcher
- R¹: für 3,3,3-Trifluor-2-hydroxypropyl, 3,3,3-Trifluorpropyl oder 3,3,3-Trifluorprop-1-en-1-yl steht,
- R²: für *p*-Chlorphenyl steht,
- R^{3A} und R^{3B}: unabhängig voneinander für Wasserstoff, Chlor oder Trifluormethyl stehen, wobei jedoch mindestens einer der Reste R^{3A} und R^{3B} von Wasserstoff verschieden ist,
- R^{3C}: für Wasserstoff steht,
und
- L: für eine Gruppe der Formel 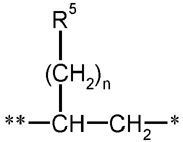 steht, worin
* die Verknüpfungsstelle mit dem angrenzenden N-Atom und
** die Verknüpfungsstelle mit dem Phenyl-Ring kennzeichnen,
n die Zahl 0 oder 1 bedeutet, und
R⁵ eine Gruppe der Formel -O-C(=O)-NH₂, -NH-C(=O)-NH₂ oder -NH-SO₂-R¹⁰ bedeutet, worin
R¹⁰ Methyl oder Ethyl darstellt,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt. Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man eine Verbindung der Formel (II) 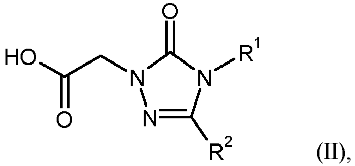 in welcher R¹ und R² die oben angegebenen Bedeutungen haben,

in einem inerten Lösungsmittel unter Aktivierung der Carbonsäure-Funktion mit einer Verbindung der Formel (III) 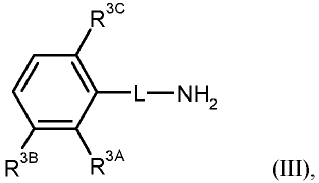 in welcher L, R^{3A}, R^{3B} und R^{3C} die oben angegebenen Bedeutungen haben,
kuppelt und die so erhaltenen Verbindungen der Formel (I) gegebenenfalls in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

Inerte Lösungsmittel für den Verfahrensschritt (II) + (III) → (I) sind beispielsweise Ether wie Diethylether, Diisopropylether, *tert*.-Butylmethylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Heptan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder dipolar-aprotische Lösungsmittel wie Aceton, Methylethylketon, Ethylacetat, Acetonitril, Pyridin, Dimethylsulfoxid (DMSO), *N*,*N*-Dimethylformamid (DMF), *N*,*N*-Dimethylacetamid (DMA), *N*,*N*'-Dimethylpropylenharnstoff (DMPU) oder *N*-Methylpyrrolidinon (NMP). Ebenso ist es möglich, Gemische solcher Lösungsmittel zu verwenden. Bevorzugt sind Acetonitril, Dichlormethan, Dimethylformamid oder Gemische dieser Lösungsmittel.

Als Aktivierungs-/Kondensationsmittel für die Kupplungsreaktion (II) + (III) → (I) eignen sich beispielsweise Carbodiimide wie *N*,*N*'-Diethyl-, *N*,*N*'-Dipropyl-, *N*,*N*'-Diisopropyl-, *N*,*N*'-Dicyclohexylcarbodiimid (DCC) oder *N*-(3-Dimethylaminoisopropyl)-*N*'-ethylcarbodiimid-Hydrochlorid (EDC), Phosgen-Derivate wie *N*,*N*'-Carbonyldiimidazol (CDI) oder Isobutylchlorformiat, 1,2-Oxazolium-Verbindungen wie 2-Ethyl-5-phenyl-1,2-oxazolium-3-sulfat oder 2-*tert*.-Butyl-5-methyl-isoxazolium-perchlorat, Acylamino-Verbindungen wie 2-Ethoxy-1-ethoxycarbonyl-1,2-dihydrochinolin, α-Chlorenamine wie 1-Chlor-2-methyl-1-dimethylamino-1-propen, Phosphor-Verbindungen wie Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat oder Benzotriazol-1-yloxy-tris(pyrrolidino)phosphonium-hexafluorophosphat (PyBOP), oder Uronium-Verbindungen wie *O*-(Benzotriazol-1-yl)-*N*,*N*,*N*',*N*'-tetramethyluronium-tetrafluoroborat (TBTU), *O*-(Benzotriazol-1-yl)-*N*,*N*,*N*',*N*'-tetramethyluronium-hexafluorophosphat (HBTU), 2-(2-Ovo-1-(2H)-pyridyl)-1,1,3,3-tetramethyluronium-tetrafluoroborat (TPTU), *O*-(7-Azabenzotriazol-1-yl)-*N*,*N*,*N*',*N*'-tetramethyluronium-hexafluorophosphat (HATU) oder *O*-(1*H*-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder tertiäre Aminbasen wie Triethylamin, *N*-Methylmorpholin, *N*-Methylpiperidin, *N,N-*Diisopropylethylamin, Pyridin oder 4-*N*,*N*-Dimethylaminopyridin. Bevorzugt wird EDC in Kombination mit HOBt und *N*,*N*-Diisopropylethylamin verwendet.

Die Kupplung (II) + (III) → (I) wird im Allgemeinen in einem Temperaturbereich von -20°C bis +60°C, bevorzugt bei 0°C bis +40°C durchgeführt. Die Umsetzung kann bei normalem, bei erhöhtem oder bei erniedrigtem Druck erfolgen (z.B. von 0.5 bis 5 bar). Im Allgemeinen arbeitet man bei Normaldruck.

Die Herstellung der erfindungsgemäßen Verbindungen kann durch das folgende Syntheseschema veranschaulicht werden:

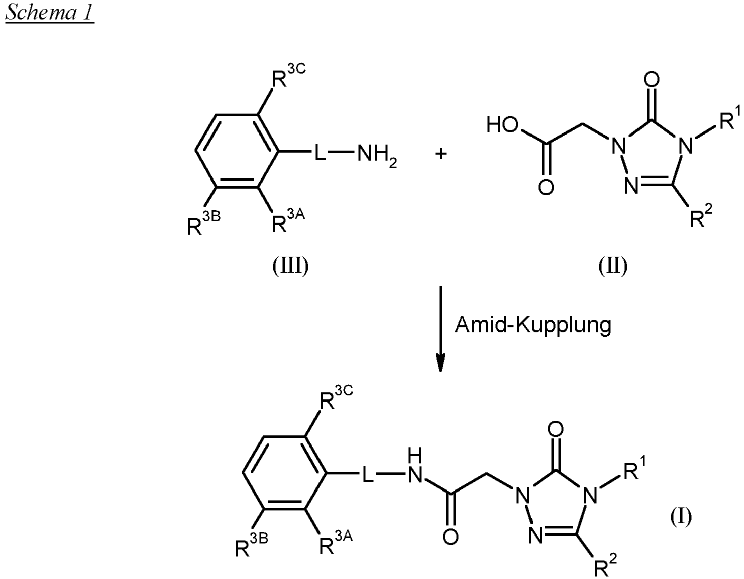

Die Verbindungen der Formel (II) können ihrerseits durch baseninduzierte Alkylierung von 2,4-Dihydro-3*H*-1,2,4-triazol-3-onen der Formel (IV) mit einem Haloessigsäureester der Formel (V) zu den N²-substituierten Verbindungen der Formel (VI) und nachfolgende Ester-Hydrolyse erhalten werden (siehe Schema 2): 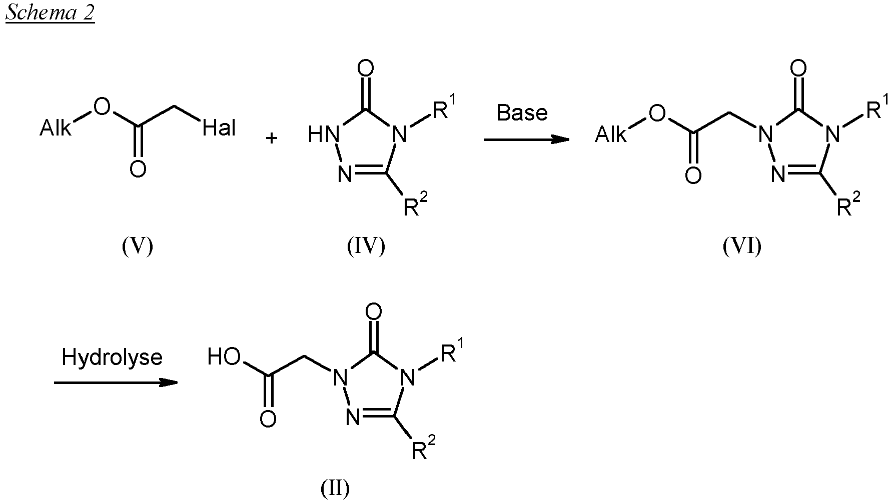 [Alk = Alkyl, Hal = Halogen].

Die Verbindungen der Formel (VI) können auf alternativem Wege auch aus literaturbekannten *N-*(Alkoxycarbonyl)arylthioamiden der Formel (VIII) [siehe z.B. M. Arnswald, W.P. Neumann, J. Org. Chem. 58 (25), 7022-7028 (1993); E.P. Papadopoulos, J. Org. Chem. 41 (6), 962-965 (1976)] durch Umsetzung mit einem Hydrazinoessigsäureester der Formel (VII) und nachfolgende Derivatisierung an N-4 des Triazolons (IX) hergestellt werden (siehe Schema 3):

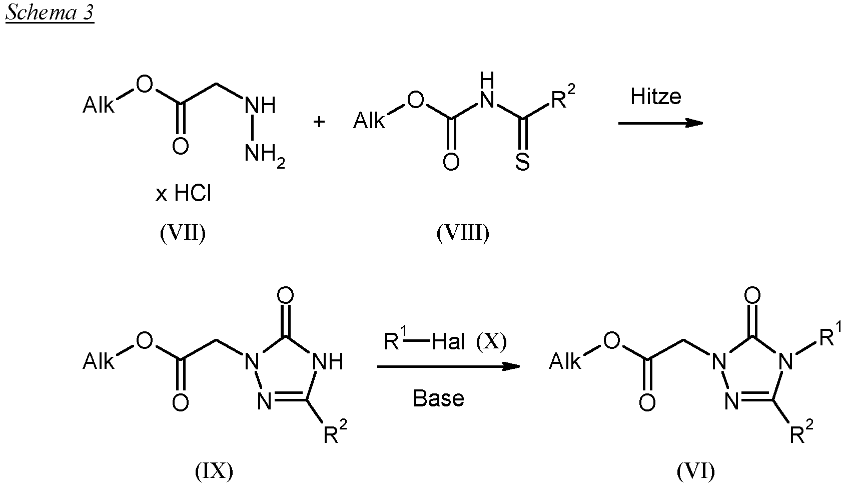

Die Verbindungen der Formel (IV) können ausgehend von Carbonsäurehydraziden der Formel (XI) durch Umsetzung mit Isocyanaten der Formel (XII) oder Nitrophenylcarbamaten der Formel (XIII) und nachfolgende baseninduzierte Cyclisierung der intermediären Hydrazincarboxamide (XIV) hergestellt werden (siehe Schema 4):

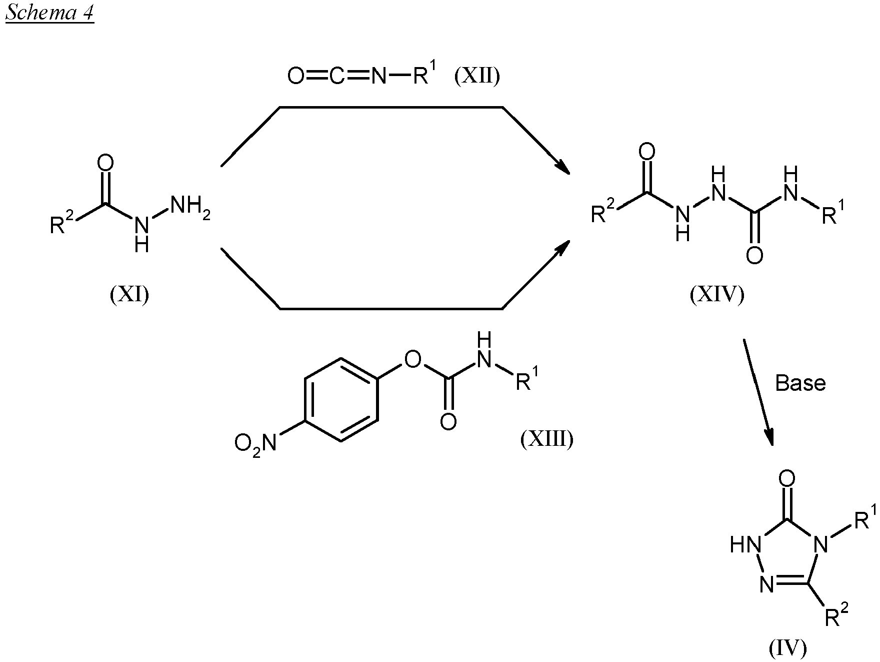

Gemäß einer besonderen Verfahrensvariante lassen sich Verbindungen der Formel (VI) gegebenenfalls auch dadurch herstellen, dass bei den in Schema 2 und 4 beschriebenen Verfahren anstelle des Restes R¹ zunächst eine temporäre Schutzgruppe (PG), wie beispielsweise Allyl oder 4-Methoxybenzyl, eingesetzt wird; nach deren Abspaltung zu Verbindungen der Formel (IX) können die gewünschten Verbindungen der Formel (VI) dann durch entsprechende N⁴-Alkylierung erhalten werden (siehe Schema 5): 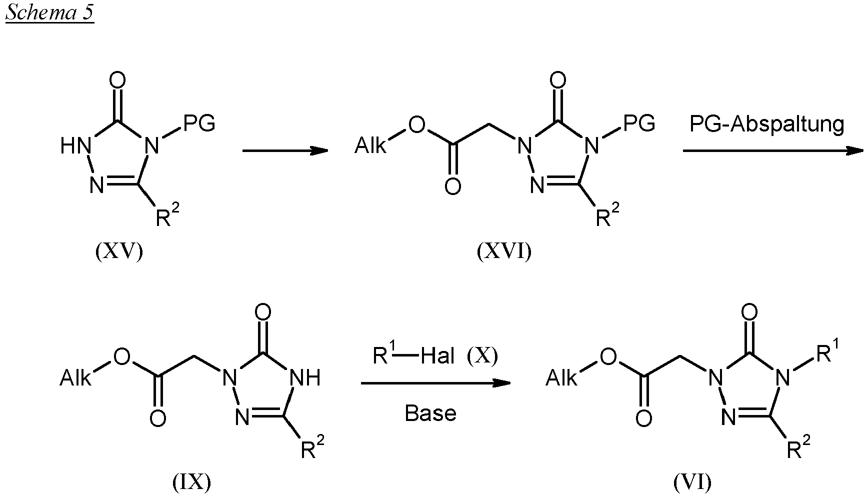 [PG = Schutzgruppe, z.B. Allyl oder 4-Methoxybenzyl].

Die Einführung und Abspaltung der Schutzgruppe PG erfolgt hierbei nach literaturüblichen Methoden [siehe z.B. T.W. Greene und P.G.M. Wuts, Protective Groups in Organic Synthesis, Wiley, New York, 1999]. So wird die Allyl-Gruppe vorzugsweise mit Hilfe von Ameisensäure in Gegenwart des Tetrakis(triphenylphosphin)palladium(0)-Katalysators und einer Amin-Base wie Triethylamin entfernt. Die Abspaltung der *p*-Methoxybenzyl-Schutzgruppe erfolgt bevorzugt mit Hilfe starker Säuren, wie beispielsweise Trifluoressigsäure, oder auf oxidativem Wege, z.B. durch Behandlung mit 2,3-Dichlor-5,6-dicyano-1,4-benzochinon (DDQ) oder Ammoniumcer(IV)nitrat.

Analoge Transformationen PG → R¹ können gegebenenfalls auch auf anderen Verfahrensstufen durchgeführt werden.

Weitere erfindungsgemäße Verbindungen der Formel (I) können, falls zweckmäßig, auch durch Umwandlungen von funktionellen Gruppen einzelner Reste und Substituenten, insbesondere den unter R¹, R², R⁵ und R⁶ aufgeführten, hergestellt werden, wobei von anderen, nach obigen Verfahren erhaltenen Verbindungen der Formel (I) oder Vorstufen hiervon ausgegangen wird. Diese Umwandlungen werden nach üblichen, dem Fachmann geläufigen Methoden durchgeführt und umfassen beispielsweise Reaktionen wie nukleophile oder elektrophile Substitutionsreaktionen, nukleophile oder elektrophile Additionsreaktionen, Eliminierungsreaktionen, Oxidation, Reduktion, Hydrierung, Alkylierung, Acylierung, Sulfonylierung, Aminierung, Hydroxylierung, Veretherung, Veresterung, Esterspaltung und -hydrolyse, insbesondere die Bildung von Carbonamiden, Sulfonamiden, Carbamaten, Harnstoffen und Schwefelsäurediamiden, sowie die Einführung und Entfernung temporärer Schutzgruppen [vgl. auch die im nachfolgenden Experimentellen Teil detailliert beschriebene Herstellung der Ausführungsbeispiele].

Eine Trennung der erfindungsgemäßen Verbindungen in die entsprechenden Enantiomere und/ oder Diastereomere kann, je nach Zweckmäßigkeit, auch bereits auf der Stufe einzelner Intermediate, wie oben aufgeführt, erfolgen, wobei diese Intermediate dann in separierter Form entsprechend den zuvor beschriebenen Verfahrensschritten weiter umgesetzt werden. Eine solche Auftrennung der Stereoisomeren läßt sich nach üblichen, dem Fachmann bekannten Methoden durchführen; vorzugsweise werden chromatographische Verfahren, insbesondere die HPLC-Chromatographie an achiraler bzw. chiraler Phase, verwendet.

Die Verbindungen der Formeln (III), (V), (VII), (VIII), (X), (XI), (XII) und (XIII) sind entweder kommerziell erhältlich oder als solche in der Literatur beschrieben, oder sie können, ausgehend von kommerziell erhältlichen Verbindungen, nach allgemein üblichen, literaturbekannten Methoden hergestellt werden. Zahlreiche detaillierte Vorschriften sowie Literaturangaben zur Herstellung dieser Substanzen befinden sich auch im nachfolgenden Experimentellen Teil im Abschnitt zur Herstellung der Ausgangsverbindungen und Intermediate.

Die erfindungsgemäßen Verbindungen besitzen wertvolle pharmakologische Eigenschaften und können zur Vorbeugung und/oder Behandlung von verschiedenen Erkrankungen und krankheitsbedingten Zuständen beim Menschen und bei Säugetieren allgemein verwendet werden.

Die erfindungsgemäßen Verbindungen stellen potente selektive V1a-, V2- oder duale V1a/V2-Rezeptor-Antagonisten dar, die die Vasopressin-Aktivität *in vitro* und *in vivo* inhibieren. Darüber hinaus wirken die erfindungsgemäßen Verbindungen auch als Antagonisten am verwandten Oxytozin-Rezeptor.

Die erfindungsgemäßen Verbindungen sind insbesondere für die Prävention und/oder Behandlung von kardiovaskulären Erkrankungen geeignet. In diesem Zusammenhang seien als Zielindikationen beispielhaft und vorzugsweise genannt: akute und chronische Herzinsuffizienz, arterielle Hypertonie, koronare Herzerkrankung, stabile und instabile Angina pectoris, myokardiale Ischämie, Myokardinfarkt, Schock, Arteriosklerose, atriale und ventrikuläre Arrhythmien, transitorische und ischämische Attacken, Hirnschlag, entzündliche kardiovaskuläre Erkrankungen, periphere und kardiale Gefäßerkrankungen, periphere Durchblutungsstörungen, arterielle pulmonale Hypertonie, Spasmen der Koronararterien und peripherer Arterien, Thrombosen, thromboembolische Erkrankungen, Ödembildung wie zum Beispiel pulmonales Ödem, Hirnödem, renales Ödem oder Herzinsuffizienzbedingtes Ödem, sowie Restenosen wie nach Thrombolysetherapien, percutan-transluminalen Angioplastien (PTA), transluminalen Koronarangioplastien (PTCA), Herztransplantationen und Bypass-Operationen.

Im Sinne der vorliegenden Erfindung umfasst der Begriff Herzinsuffizienz auch spezifischere oder verwandte Krankheitsformen wie Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Weiterhin eignen sich die erfindungsgemäßen Verbindungen zur Verwendung als Diuretikum für die Behandlung von Ödemen und bei Elektrolytstörungen, insbesondere bei der hypervolämischen und euvolämischen Hyponaträmie.

Die erfindungsgemäßen Verbindungen sind weiter geeignet für die Prävention und/oder Behandlung der polyzystischen Nierenkrankheit (PCKD) und des Syndroms der inadäquaten ADH-Sekretion (SIADH).

Außerdem können die erfindungsgemäßen Verbindungen für die Prävention und/oder Behandlung der Leberzirrhose, des Aszites, von Diabetes mellitus und diabetischen Folgeerkrankungen, wie z.B. Neuropathie und Nephropathie, von akutem und chronischem Nierenversagen sowie von chronischer Niereninsuffizienz verwendet werden.

Ferner eignen sich die erfindungsgemäßen Verbindungen für die Prävention und/oder Behandlung zentralnervöser Störungen wie Angstzuständen und Depressionen, von Glaukom sowie von Krebs, insbesondere von Lungentumoren.

Darüber hinaus können die erfindungsgemäßen Verbindungen für die Prävention und/oder Behandlung von entzündlichen Erkrankungen, asthmatischen Erkrankungen, chronisch-obstruktiven Atemwegserkrankungen (COPD), Schmerzzuständen, Prostatahypertrophien, Inkontinenz, Blasenentzündung, hyperaktiver Blase, Erkrankungen der Nebenniere wie zum Beispiel Phäochromozytom und Nebennierenapoplexie, Erkrankungen des Darms wie zum Beispiel Morbus Crohn und Diarrhoe, von Menstruationsstörungen wie zum Beispiel Dysmenorrhöen oder von Endometriose eingesetzt werden.

Aufgrund ihres Wirkprofils eignen sich die erfindungsgemäßen Verbindungen insbesondere zur Behandlung und/oder Prävention von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, die mindestens eine der erfindungsgemäßen Verbindungen sowie einen oder mehrere weitere Wirkstoffe enthalten, insbesondere zur Behandlung und/oder Prävention der zuvor genannten Erkrankungen. Als hierfür geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Diuretika, insbesondere Schleifendiuretika sowie Thiazide und Thiazid-ähnliche Diuretika;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Calcium-Sensitizer, wie beispielhaft und vorzugsweise Levosimendan;
- NO- und Häm-unabhängige Aktivatoren der Guanylatcyclase, wie insbesondere Cinaciguat sowie die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der Guanylatcyclase, wie insbesondere Riociguat sowie die in WO 00/06568, WO 00/06569, WO 02/42301 und WO 03/095451 beschriebenen Verbindungen;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib;
- den Energiestoffwechsel des Herzens beeinflussende Verbindungen, wie beispielhaft und vorzugsweise Etomoxir, Dichloracetat, Ranolazine oder Trimetazidine;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten und Rho-Kinase-Inhibitoren; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugs-weise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Vasopeptidase-Inhibitor bzw. Inhibitor der neutralen Endopeptidase (NEP), wie beispielhaft und vorzugsweise Omapatrilat oder AVE-7688, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon, Canrenon oder Kalium-Canrenoat, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib, Dalcetrapib, Anacetrapib, BAY 60-5521 oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 10 mg/kg, vorzugsweise etwa 0.01 bis 1 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- Ac: Acetyl
- Alk: Alkyl
- Boc: *tert*.-Butoxycarbonyl
- Bsp.: Beispiel
- ca.: *circa*, ungefähr
- CI: chemische Ionisation (bei MS)
- DCI: direkte chemische Ionisation (bei MS)
- DME: 1,2-Dimethoxyethan
- DMF: *N*,*N*-Dimethylformamid
- DMPU: 1,3-Dimethyltetrahydro-2(1*H*)-pyrimidinon
- DMSO: Dimethylsulfoxid
- d. Th.: der Theorie (bei Ausbeute)
- EDC: *N*'-(3-Dimethylaminopropyl)-*N*-ethylcarbodimid-Hydrochlorid
- ee: Enantiomerenüberschuss
- eq.: Äquivalent(e)
- ESI: Elektrospray-Ionisation (bei MS)
- ges.: gesättigt
- h: Stunde(n)
- Hal: Halogen
- HOBt: 1-Hydroxy-1*H*-benzotriazol-Hydrat
- HPLC: Hochdruck-, Hochleistungsflüssigchromatographie
- konz.: konzentriert
- LC/MS: Flüssigchromatographie-gekoppelte Massenspektrometrie
- LDA: Lithiumdiisopropylamid
- LiHMDS: Lithiumhexamethyldisilazan
- min: Minute(n)
- MS: Massenspektrometrie
- MTBE: Methyl-*tert*.-butylether
- NMR: Kernresonanzspektrometrie
- OAc: Acetat
- *p*: para
- Ph: Phenyl
- quant.: quantitativ (bei Ausbeute)
- rac: racemisch / Racemat
- RT: Raumtemperatur
- Rₜ: Retentionszeit (bei HPLC)
- THF: Tetrahydrofuran
- UV: Ultraviolett-Spektrometrie
- v/v: Volumen zu Volumen-Verhältnis (einer Lösung)
- zus.: zusammen

### LC/MS- und HPLC-Methoden:

### Methode 1 (LC/MS):

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 2 (LC/MS):

Instrument: Micromass Quattro Micro MS mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 3 (LC/MS):

Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 4 (LC/MS):

Instrument: Waters Acquity SQD UPLC System; Säule: Waters Acquity UPLC HSS T3 1.8µ 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 5 (chirale präparative HPLC):

Chirale stationäre Kieselgel-Phase basierend auf dem Selektor Poly(*N*-methacryloyl-L-leucin-(+)-3-pinanmethylamid); Säule: 600 mm x 30 mm; Temperatur: 24°C; UV-Detektion: 265 nm; Fluss: 80 ml/min; Elutionsmittel:
Methode 5a: 0-13.1 min Isohexan/Ethylacetat 25:75 (v/v), 13.11-19.1 min 100% Ethylacetat, 19.11-23.5 min Isohexan/Ethylacetat 25:75 (v/v);
Methode 5b: 100% Ethylacetat.

### Methode 6 (chirale analytische HPLC):

Chirale stationäre Kieselgel-Phase basierend auf dem Selektor Poly(*N*-methacryloyl-L-leucin-(+)-3-pinanmethylamid); Säule: 250 mm x 4.6 mm; Temperatur: 24°C; UV-Detektion: 265 nm; Fluss: 2 ml/min; Elutionsmittel:
Methode 6a: Isohexan/Ethylacetat 1:4 (v/v);
Methode 6b: 100% Ethylacetat.

### Methode 7 (präparative HPLC):

Säule: YMC ODS C18, 10 µm, 250 mm x 30 mm; Eluent A: 0.1% Ameisensäure in Wasser, Eluent B: Acetonitril; Fluss: 50 ml/min; Programm: 0-6 min 10% B, 6-27 min Gradient bis 95% B, 27-43 min 95% B, 43-45 min Gradient bis 10% B, 45-50 min 10% B.

### Methode 8 (präparative HPLC):

Säule: Grom-Sil 120 ODS-4HE, 10 µm, 250 mm x 30 mm; Eluent A: 0.1% Ameisensäure in Wasser, Eluent B: Acetonitril; Fluss: 50 ml/min; Programm: 0-3 min 10% B, 3-27 min Gradient bis 95% B, 27-34 min 95% B, 34-38 min 10% B.

### Methode 9 (präparative HPLC):

Säule: Grom-Sil 120 ODS-4HE, 10 µm, 250 mm x 30 mm; Eluent A: 0.1% Ameisensäure in Wasser, Eluent B: Acetonitril; Fluss: 50 ml/min; Programm: 0-6 min 5% B, 6-34 min Gradient bis 95% B, 34-38 min 95% B, 38-45 min 5% B.

### Methode 10 (chirale präparative HPLC):

Chirale stationäre Kieselgel-Phase basierend auf dem Selektor Poly(*N*-methacryloyl-D-leucin-dicyclopropylmethylamid); Säule: 670 mm x 40 mm; Fluss: 80 ml/min; Temperatur: 24°C; UV-Detektion: 260 nm; Elutionsmittel:
Methode 10a: Isohexan/Essigsäureethylester 20:80 (v/v);
Methode 10b: Isohexan/Essigsäureethylester 15:85 (v/v).

### Methode 11 (chirale analytische HPLC):

Chirale stationäre Kieselgel-Phase basierend auf dem Selektor Poly(*N*-methacryloyl-D-leucin-dicyclopropylmethylamid); Säule: 250 mm x 4.6 mm; Eluent: 100% Essigsäureethylester; Fluss: 2 ml/min; Temperatur: 24°C; UV-Detektion: 265 nm.

### Methode 12 (präparative HPLC):

Säule: Grom-Sil 120 ODS-4HE, 10 µm, 250 mm x 30 mm; Eluent A: 0.1% Ameisensäure in Wasser, Eluent B: Methanol; Fluss: 50 ml/min; Programm: 0-6 min 20% B, 6-27 min Gradient bis 98% B, 27-53 min 98% B, 53-54 min Gradient bis 20% B, 54-61 min 20% B.

### Methode 13 (chirale präparative HPLC):

Stationäre Phase: Daicel Chiralpak AS-H, 5 µm; Säule: 250 mm x 20 mm; Eluent: Isohexan/ Methanol/n-Propanol 95:2.5:2.5 (v/v/v); Fluss: 20 ml/min; Temperatur: RT; UV-Detektion: 230 nm.

### Methode 14 (chirale analytische HPLC):

Stationäre Phase: Daicel Chiralpak AS-H, 5 µm; Säule: 250 mm x 4 mm; Eluent: Isohexan/ Methanol/Ethanol 92:4:4 (v/v/v); Fluss 1 ml/min; UV-Detektion: 220 nm.

### Methode 15 (chirale präparative HPLC):

Chirale stationäre Mercapto-Kieselgel-Phase basierend auf dem Selektor Poly(*N*-methacryloyl-L-isoleucin-3-pentylamid); Säule: 430 mm x 40 mm; Eluent: 100% Ethylacetat; Fluss: 80 ml/min; Temperatur: 24°C; UV-Detektion: 265 nm.

### Methode 16 (chirale analytische HPLC):

Chirale stationäre Mercapto-Kieselgel-Phase basierend auf dem Selektor Poly(*N*-methacryloyl-L-isoleucin-3-pentylamid); Säule: 250 mm x 4.6 mm; Eluent: 100% Ethylacetat; Fluss: 2 ml/min; Temperatur: 24°C; UV-Detektion: 265 nm.

### Methode 17 (chirale präparative HPLC):

Stationäre Phase: Daicel Chiralpak AD-H, 10 µm; Säule: 250 mm x 20 mm; Temperatur: RT; UV-Detektion: 230 nm; Fluss: 20 ml/min; Elutionsmittel:
Methode 17a: Isohexan/Isopropanol 60:40 (v/v);
Methode 17b: Isohexan/Isopropanol 70:30 (v/v);
Methode 17c: Isohexan/Ethanol 75:25 (v/v).

### Methode 18 (chirale analytische HPLC):

Stationäre Phase: Daicel Chiralpak AD-H, 5 µm; Säule: 250 mm x 4.6 mm; Temperatur: 30°C; UV-Detektion: 230 nm; Fluss: 1.0 ml/min; Elutionsmittel:
Methode 18a: Isohexan/Isopropanol 50:50 (v/v);
Methode 18b: Isohexan/Ethanol 70:30 (v/v).

### Methode 19 (präparative HPLC):

Säule: Reprosil C18, 10 µm, 250 mm x 30 mm; Eluent A: 0.1% Ameisensäure in Wasser, Eluent B: Methanol; Fluss: 50 ml/min; Programm: 0-6 min 30% B, 6-33 min Gradient bis 95% B, 33-42 min 95% B, 42-43 min Gradient bis 30% B, 43-50 min 30% B.

### Methode 20 (präparative HPLC):

Säule: Reprosil C18, 10 µm, 250 mm x 40 mm; Eluent A: 0.1% Ameisensäure in Wasser, Eluent B: Acetonitril; Fluss: 50 ml/min; Programm: 0-6 min 10% B, 6-40 min Gradient bis 95% B, 40-53 min 95% B, 53-54 min Gradient bis 10% B, 54-57 min 10% B.

### Methode 21 (LC/MS):

Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A 4.5 min 5% A → 6 min 5% A; Fluss: 2 ml/min; Ofen: 40°C; UV-Detektion: 210 nm.

### Methode 22 (LC/MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 23 (präparative HPLC):

Säule: Reprosil C18, 10 µm, 250 mm x 30 mm; Eluent A: 0.1% Ameisensäure in Wasser, Eluent B: Acetonitril; Fluss: 50 ml/min; Programm: 0-6 min 10% B, 6-27 min Gradient bis 95% B, 27-38 min 95% B, 38-39 min Gradient bis 10% B, 39-40 min 10% B.

### Ausgangsverbindungen und Intermediate:

### Beispiel 1A

### Ethyl-N-({2-[(4-chlorphenyl)carbonyl]hydrazinyl}carbonyl)glycinat

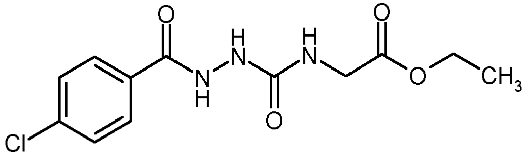

Eine Suspension von 12.95 g (75.9 mmol) 4-Chlorbenzohydrazid in 50 ml trockenem THF wurde bei 50°C vorgelegt und tropfenweise mit einer Lösung von 10.0 g (77.5 mmol) Ethyl-2-isocyanatoacetat in 100 ml trockenem THF versetzt. Zunächst bildete sich eine Lösung, dann fiel ein Niederschlag aus. Nach Ende der Zugabe wurde die Mischung noch 2 h bei 50°C weiter gerührt und dann über Nacht bei RT stehen gelassen. Die Kristalle wurden durch Filtration isoliert, mit wenig Diethylether gewaschen und im Hochvakuum getrocknet. Es wurden 21.43 g (89% d. Th) der Titelverbindung erhalten.
LC/MS [Methode 1]: Rₜ = 1.13 min; m/z = 300 (M+H)⁺
¹H-NMR (DMSO-d₆, 400 MHz): δ = 1.19 (t, 3H), 3.77 (d, 2H), 4.09 (q, 2H), 6.88 (br. s, 1H), 7.57 (d, 2H), 7.91 (d, 2H), 8.21 (s, 1H), 10.29 (s, 1H).

### Beispiel 2A

### [3-(4-Chlorphenyl)-5-oxo-1,5-dihydro-4H-1,2,4-triazol-4-yl]essigsäure

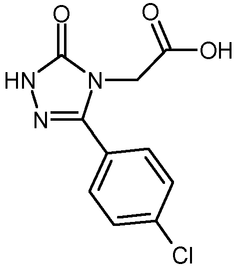

21.43 g (67.9 mmol) der Verbindung aus Beispiel 1A wurden mit 91 ml 3 N Natronlauge versetzt und über Nacht unter Rückfluss erhitzt. Nach Abkühlen auf RT wurde die Mischung durch langsame Zugabe von ca. 20%-iger Salzsäure auf pH 1 eingestellt. Der ausgefallene Feststoff wurde durch Filtration isoliert, mit Wasser gewaschen und bei 60°C im Vakuum getrocknet. Es wurden 17.55 g der Titelverbindung in ca. 88% Reinheit (90% d. Th.) erhalten.
LC/MS [Methode 1]: Rₜ = 0.94 min; m/z = 254 (M+H)⁺
¹H-NMR DMSO-d₆, 400 MHz): δ = 4.45 (s, 2H), 7.65-7.56 (m, 4H), 12.09 (s, 1H), 13.25 (br. s, 1H).

### Beispiel 3A

### 5-(4-Chlorphenyl)-4-(3,3,3-trifluor-2-oxopropyl)-2,4-dihydro-3H-1,2,4-triazol-3-on (Keton-Form) bzw. 5-(4-Chlorphenyl)-4-(3,3,3-trifluor-2,2-dihydroxypropyl)-2,4-dihydro-3H-1,2,4-triazol-3-on (Hydrat-Form)

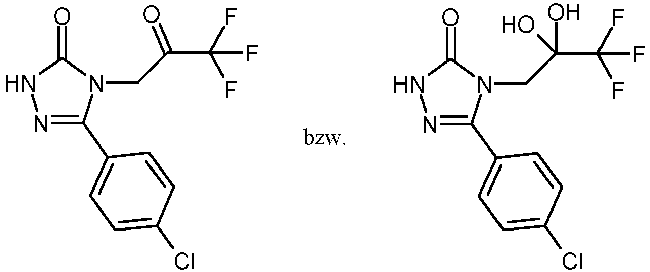

5.0 g (16.36 mmol) der Verbindung aus Beispiel 2A wurden unter Argon in 200 ml Pyridin gelöst und mit 17.18 g (81.8 mmol) Trifluoressigsäureanhydrid versetzt. Dabei stieg die Temperatur auf ca. 35°C an. Nach 30 min wurde das Pyridin am Rotationsverdampfer entfernt und der Rückstand mit 1.5 Liter 0.5 N Salzsäure versetzt. Diese Mischung wurde auf 70°C erwärmt und dann in der Wärme filtriert. Der Feststoff wurde mit etwas Wasser gewaschen. Das gesamte Filtrat wurde dreimal mit Ethylacetat extrahiert. Die vereinigten organischen Phasen wurden nacheinander mit Wasser, ges. Natriumhydrogencarbonat-Lösung und ges. Natriumchlorid-Lösung gewaschen, über Natriumsulfat getrocknet und am Rotationsverdampfer vom Lösungsmittel befreit. Der Rückstand wurde im Hochvakuum getrocknet. Es wurden 3.56 g (68% d. Th.) der Titelverbindung in der Hydrat-Form erhalten.
LC/MS [Methode 1]: Rₜ = 1.51 min; m/z = 306 (M+H)⁺ und 324 (M+H)⁺ (Keton- bzw. Hydrat-Form)
¹H-NMR (DMSO-d₆, 400 MHz): δ = 3.98 (s, 2H), 7.61 (d, 2H), 7.68 (br. s, 2H), 7.72 (d, 2H), 12.44 (s, 1H).

### Beispiel 4A

### 5-(4-Chlorphenyl)-4-(3,3,3-trifluor-2-hydroxypropyl)-2,4-dihydro-3H-1,2,4-triazol-3-on

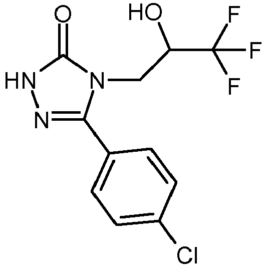

3.56 g (11.0 mmol) der Verbindung aus Beispiel 3A wurden in 100 ml Methanol gelöst und unter Eiskühlung mit 3.75 g (99.5 mmol) Natriumborhydrid versetzt. Nach 1.5 h wurden langsam 200 ml 1 M Salzsäure zugegeben. Das Methanol wurde am Rotationsverdampfer entfernt, der Rückstand mit 500 ml Wasser verdünnt und dreimal mit Ethylacetat extrahiert. Die vereinigten organischen Phasen wurden mit ges. Natriumhydrogencarbonat-Lösung und dann mit ges. Natriumchlorid-Lösung gewaschen, über Natriumsulfat getrocknet und am Rotationsverdampfer vom Lösungsmittel befreit. Der Rückstand wurde im Hochvakuum getrocknet. Es wurden 3.04 g (90% d. Th.) der Titelverbindung erhalten.
LC/MS [Methode 2]: Rₜ = 1.80 min; m/z = 308 (M+H)⁺
¹H-NMR (DMSO-d₆, 400 MHz): δ = 3.77 (dd, 1H), 3.92 (dd, 1H), 4.34-4.23 (m, 1H), 6.85 (d, 1H), 7.62 (d, 2H), 7.75 (d, 2H), 12.11 (s, 1H).

### Beispiel 5A

### {3-(4-Chlorphenyl)-5-oxo-4-(3,3,3-trifluor-2-hydroxypropyl)-4,5-dihydro-1H-1,2,4-triazol-1-yl}-essigsäuremethylester (Racemat)

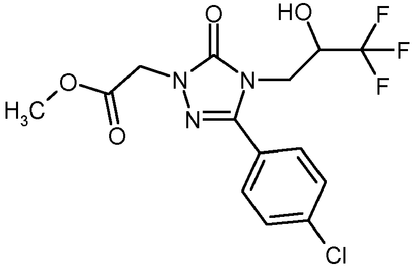

3.04 g (9.9 mmol) der Verbindung aus Beispiel 4A wurden in 100 ml Acetonitril gelöst und mit 1.07 g (9.9 mmol) Chloressigsäuremethylester, 2.73 g (19.8 mmol) Kaliumcarbonat sowie einer kleinen Spatelspitze Kaliumiodid versetzt. Die Reaktionsmischung wurde 1 h unter Rückfluss erhitzt, dann auf RT abkühlen gelassen und filtriert. Das Filtrat wurde am Rotationsverdampfer von den flüchtigen Komponenten befreit und der Rückstand im Hochvakuum getrocknet. Es wurden 3.70 g der Titelverbindung in ca. 90% Reinheit (89% d. Th.) erhalten.
LC/MS [Methode 3]: Rₜ = 1.10 min; m/z = 380 (M+H)⁺
¹H-NMR (DMSO-d₆, 400 MHz): δ = 3.70 (s, 3H), 3.84 (dd, 1H), 3.99 (dd, 1H), 4.16-4.35 (m, 1H), 4.72 (s, 2H), 6.91 (d, 1H), 7.64 (d, 2H), 7.78 (d, 2H).

Die racemische Verbindung aus Beispiel 5A wurde durch präparative HPLC an chiraler Phase in die Enantiomere aufgetrennt [Probenvorbereitung: 3.6 g Racemat gelöst in 54 ml Ethylacetat/Isohexan (1:1 v/v), in drei Portionen über die Säule getrennt; Säule: chirale Kieselgel-Phase basierend auf dem Selektor Poly(*N*-methacryloyl-L-isoleucin-3-pentylamid), 430 mm x 40 mm; Eluent: Stufengradient Isohexan/Ethylacetat 1:1 → Ethylacetat → Isohexan/Ethylacetat 1:1; Fluss: 50 ml/ min; Temperatur: 24°C; UV-Detektion: 260 nm]. Auf diese Weise wurden 1.6 g des zuerst eluierenden Enantiomers 1 (Beispiel 6A) sowie 1.6 g des später eluierenden Enantiomers 2 (Beispiel 7A) erhalten:

### Beispiel 6A

### {3-(4-Chlorphenyl)-5-oxo-4-[(2S)-3,3,3-trifluor-2-hydroxypropyl]-4,5-dihydro-1H-1,2,4-triazol-1-yl}essigsäuremethylester (enantiomer 1)

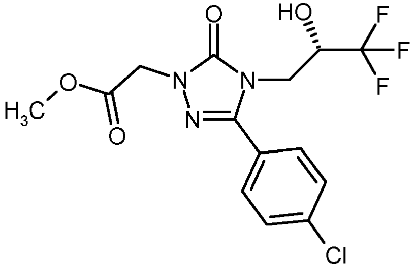

Zuerst eluierendes Enantiomer aus der Racemat-Trennung von Beispiel 5A.

Rₜ = 3.21 min [Säule: chirale Kieselgel-Phase basierend auf dem Selektor Poly(*N*-methacryloyl-L-isoleucin-3-pentylamid), 250 mm x 4.6 mm; Eluent: Isohexan/Ethylacetat 1:1; Fluss: 1 ml/min; UV-Detektion: 260 nm].

### Beispiel 7A

### {3-(4-Chlorphenyl)-5-oxo-4-[(2R)-3,3,3-trifluor-2-hydroxypropyl]-4,5-dihydro-1H-1,2,4-triazol-1-yl}essigsäuremethylester (Enantiomere 2)

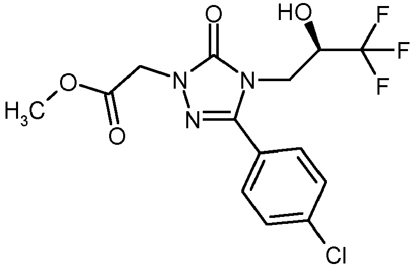

Zuletzt eluierendes Enantiomer aus der Racemat-Trennung von Beispiel 5A.

Rₜ = 4.48 min [Säule: chirale Kieselgel-Phase basierend auf dem Selektor Poly(*N*-methacryloyl-L-isoleucin-3-pentylamid), 250 mm x 4.6 mm; Eluent: Isohexan/Ethylacetat 1:1; Fluss: 1 ml/min; UV-Detektion: 260 nm].

### Beispiel 8A

### {3-(4-Chlorphenyl)-5-oxo-4-(2S)-3,3,3-trifluor-2-hydroxypropyl]-4,5-dihydro-1H-1,2,4-triazol-1-yl}essigsäure

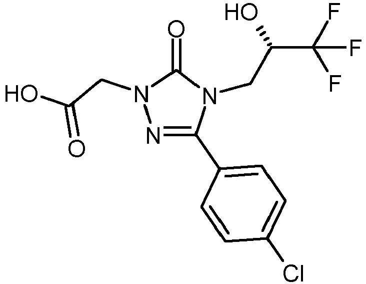

Die enantiomerenreine Verbindung aus Beispiel 6A (1.6 g, 4.21 mmol) wurde in 77 ml Methanol gelöst und mit 17 ml einer 1 M Lösung von Lithiumhydroxid in Wasser versetzt. Die Mischung wurde 1 h bei RT gerührt und dann am Rotationsverdampfer eingeengt. Der Rückstand wurde mit 100 ml Wasser verdünnt und mit 1 N Salzsäure auf pH 1-2 angesäuert. Das ausgefallene Produkt wurde abfiltriert, nacheinander mit Wasser und Cyclohexan gewaschen und trocken gesaugt. Nach weiterer Trocknung im Hochvakuum wurden 1.1 g (71% d. Th.) der Titelverbindung erhalten.
[α]_{D}²⁰ = +3.4° (Methanol, c = 0.37 g/100 ml)
LC/MS [Methode 1]: Rₜ = 1.51 min; m/z = 366 (M+H)⁺
¹H-NMR (400 MHz, DMSO-d₆): δ = 3.84 (dd, 1H), 4.00 (dd, 1H), 4.25 (m, 1H), 4.58 (s, 2H), 6.91 (d, 1H), 7.63 (d, 2H), 7.78 (d, 2H), 13.20 (br. s, 1H).

### Beispiel 9A

### {3-(4-Chlorphenyl)-5-oxo-4-[(2R)-3,3,3-trifluor-2-hydroxypropyl]-4,5-dihydro-1H-1,2,4-triazol-1-yl}essigsäure

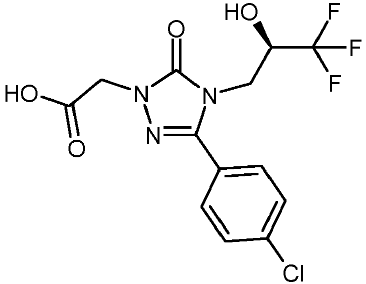

Die Titelverbindung wurde analog zu Beispiel 8A ausgehend von Beispiel 7A erhalten.
[α]_{D}²⁰ = -4.6° (Methanol, c = 0.44 g/100 ml)
LC/MS [Methode 1]: Rₜ = 1.53 min; m/z = 366 (M+H)⁺
¹H-NMR (400 MHz, DMSO-d₆): δ = 3.84 (dd, 1H), 4.00 (dd, 1H), 4.25 (m, 1H), 4.58 (s, 2H), 6.91 (d, 1H), 7.63 (d, 2H), 7.78 (d, 2H), 13.20 (br. s, 1H).

### Beispiel 10A

### Ethyl-3-amino-2-[2-(trifluormethyl)phenyl]propanoat

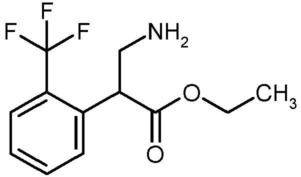

1.035 g (2.54 mmol) Ethyl-3-{[(benzyloxy)carbonyl]amino}-2-[2-(trifluormethyl)phenyl]propanoat [zur Herstellung siehe Beispiel 193A in WO 2007/134862] wurden in 24 ml Ethanol gelöst und in Gegenwart von 100 mg 10%-igem Palladium auf Kohle für 3 h unter Normaldruck hydriert. Der Katalysator wurde anschließend abfiltriert und das Filtrat am Rotationsverdampfer vom Lösungsmittel befreit. Der Rückstand entsprach der Titelverbindung.
Ausbeute: 700 mg (96% d. Th., 91% Reinheit laut LC/MS)
LC/MS [Methode 3]: Rₜ = 0.72 min; m/z = 262 (M+H)⁺
¹H-NMR (400 MHz, DMSO-d₆): δ [ppm] = 1.12 (t, 3H), 1.73 (br. s, 2H), 2.82 (dd, 1H), 3.14 (dd, 1H), 3.94 (dd, 1H), 4.01-4.16 (m, 2H), 7.49 (t, 1H), 7.58 (d, 1H), 7.67 (t, 1H), 7.73 (d, 1H).

### Beispiel IIA

### 3-Amino-2-[2-(trifluormethyl)phenyl]propan-1-ol-Hydrochlorid

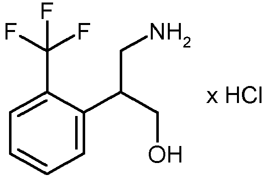

Eine Lösung von 700 mg (2.44 mmol) Ethyl-3-amino-2-[2-(trifluormethyl)phenyl]propanoat (Beispiel 10A) in 10 ml Diethylether wurde langsam zu einer auf 0°C vorgekühlten Lithiumaluminiumhydrid-Lösung (1 M in Diethylether, 3.9 ml, 3.9 mmol) getropft. Nach erfolgter Zugabe wurde die Reaktionsmischung 1 h unter Rückfluss erhitzt und dann wieder auf 0°C abgekühlt. Ein paar Tropfen Wasser wurden zugegeben, bis keine Wasserstoffentwicklung mehr zu beobachten war. Die Reaktionsmischung wurde danach filtriert und das Filtrat mit 1 ml einer 4 N Lösung von Chlorwasserstoff in Dioxan versetzt. Der ausgefallene Feststoff wurde durch Filtration isoliert, mit wenig Diethylether gewaschen und im Hochvakuum getrocknet. Man erhielt 390 mg (63% d. Th.) der Titelverbindung.
LC/MS [Methode 2]: Rₜ = 0.91 min; m/z = 220 (M+H)⁺
¹H-NMR (400 MHz, DMSO-d₆): δ [ppm] = 3.08 (br. t, 1H), 3.33-3.44 (m, 2H), 3.57-3.69 (m, 2H), 5.34 (t, 1H), 7.46-7.55 (m, 1H), 7.64-7.71 (m, 2H), 7.73 (d, 1H), 7.93 (br. s, 3H).

### Beispiel 12A

### tert.-Butyl-{3-hydroxy-2-[2-(trifluormethyl)phenyl]propyl}carbamat

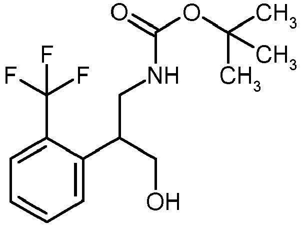

Eine Mischung von 310 mg (1.21 mmol) der Verbindung aus Beispiel 11A in 9.3 ml Dioxan und 9.3 ml einer 5%-igen wässrigen Natriumbicarbonat-Lösung wurde mit 265 mg (1.21 mmol) *Di-tert.-*butyldicarbonat versetzt und bei RT bis zur vollständigen Umsetzung gerührt. Die Mischung wurde anschließend dreimal mit Ethylacetat extrahiert. Die vereinigten organischen Phasen wurden über Natriumsulfat getrocknet und am Rotationsverdampfer vom Lösungsmittel befreit. Der Rückstand (440 mg) entsprach der Titelverbindung und wurde ohne weitere Reinigung in der Folgereaktion eingesetzt.
LC/MS [Methode 3]: Rₜ = 1.16 min; m/z = 342 (M+Na)⁺.

### Beispiel 13A

### 3-Amino-2-[2-(trifluormethyl)phenyl]propylcarbamat-Hydrochlorid

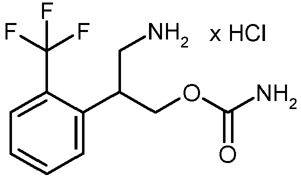

Eine Lösung von 387 mg (1.21 mmol) der Verbindung aus Beispiel 12A in 19.3 ml Acetonitril wurde auf -15°C gekühlt und mit 148 µl (1.70 mmol) Chlorsulfonylisocyanat versetzt. Nach 5 min wurden 10 ml Wasser zugegeben und die Reaktionsmischung über Nacht bei 60°C weiter gerührt. Nach Abkühlen auf RT wurden 10 ml einer gesättigten wässrigen Natriumbicarbonat-Lösung zugesetzt. Die Mischung wurde viermal mit Ethylacetat extrahiert. Die vereinigten organischen Phasen wurden über Natriumsulfat getrocknet und am Rotationsverdampfer vom Lösungsmittel befreit. Der Rückstand wurde mit 5 ml einer 4 N Lösung von Chlorwasserstoff in Dioxan versetzt und 5 min verrührt. Die flüchtigen Bestandteile wurden danach erneut am Rotationsverdampfer entfernt. Der Rückstand (300 mg, ca. 90% Reinheit) entsprach der Titelverbindung und wurde ohne weitere Reinigung weiter umgesetzt.
LC/MS [Methode 4]: Rₜ = 0.42 min; m/z = 262 (M+H)⁺
¹H-NMR (400 MHz, DMSO-d₆): δ [ppm] = 3.04-3.16 (m, 1H), 3.23-3.34 (m, 1H), 3.53-3.64 (m, 1H), 4.13 (dd, 1H), 4.24 (dd, 1H), 6.41-6.74 (br. s, 2H), 7.53 (t, 1H), 7.66-7.78 (m, 3H), 8.05 (br. s, 3H).

### Beispiel 14A

### tert.-Butyl-[2-(2-chlorphenyl)-3-hydroxypropyl]carbamat

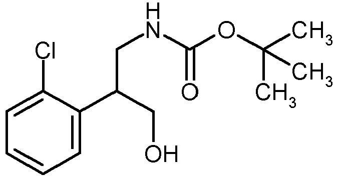

Eine Lösung von 300 mg (1.62 mmol) 3-Amino-2-(2-chlorphenyl)propan-1-ol [zur Herstellung siehe Beispiel 9h in Arch. Pharm. 1968, 301 (10), 750-762] in 14 ml Dichlormethan wurde mit 705 mg (3.23 mmol) Di-*tert*.-butyldicarbonat versetzt und 3 h bei RT gerührt. Die Mischung wurde danach mit 100 ml Ethylacetat verdünnt und nacheinander mit 1 M Salzsäure (zweimal), ges. Natriumbicarbonat-Lösung und ges. Natriumchlorid-Lösung gewaschen. Die organische Phase wurde über Natriumsulfat getrocknet und am Rotationsverdampfer vom Lösungsmittel befreit. Der Rückstand wurde durch präparative HPLC (Methode 7) gereinigt. Man erhielt 382 mg (83% d. Th.) der Titelverbindung als farbloses Öl.
LC/MS [Methode 4]: Rₜ = 0.98 min; m/z = 286 (M+H)⁺.

### Beispiel 15A

### 3-Amino-2-(2-chlorphenyl)propylcarbamat

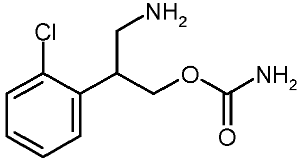

Eine Lösung von 344 mg (1.20 mmol) der Verbindung aus Beispiel 14A in 86 ml Acetonitril wurde auf -15°C gekühlt und mit 314 µl (3.61 mmol) Chlorsulfonylisocyanat versetzt. Nach 5 min wurden 10 ml Wasser zugegeben und die Reaktionsmischung über Nacht bei 60°C weiter gerührt. Nach Abkühlen auf RT wurden 10 ml einer gesättigten wässrigen Natriumbicarbonat-Lösung zugesetzt. Die Mischung wurde viermal mit Ethylacetat extrahiert. Die vereinigten organischen Phasen wurden über Natriumsulfat getrocknet und am Rotationsverdampfer vom Lösungsmittel befreit. Der Rückstand (118 mg, ca. 85% Reinheit, ca. 37% d. Th.) entsprach der Titelverbindung und wurde ohne weitere Reinigung weiter umgesetzt.
LC/MS [Methode 2]: Rₜ = 0.92 min; m/z = 229 (M+H)⁺
¹H-NMR (400 MHz, DMSO-d₆): δ [ppm] = 2.78-2.90 (m, 2H), 3.31 (br. s, 2H), 3.39-3.51 (m, 1H), 4.17 (dd, 1H), 4.24 (dd, 1H), 6.25-6.60 (br. s, 2H), 7.25 (dt, 1H), 7.32 (dt, 1H), 7.39 (dd, 1H), 7.43 (dd, 1H).

### Beispiel 16A

### Methyl-3-(1,3-dioxo-1,3-dihydro-2H-isoindol-2-yl)-2-[3-(trifluormethyl)phenyl]propanoat

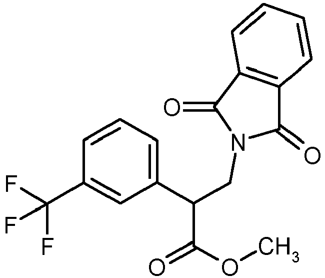

Zu einer auf -20°C vorgekühlten Lösung von 4.91 ml (35 mmol) Diisopropylamin in 50 ml THF wurde innerhalb von ca. 5 min eine Lösung von *n*-Butyllithium (1.6 M in Hexan, 18.75 ml, 30 mmol) zugetropft. Die so erhaltene LDA-Lösung wurde auf -78°C abgekühlt und mit 15.1 ml DMPU (1,3-Dimethyltetrahydro-2(1*H*)-pyrimidinon, 125 mmol) versetzt. Nach 20 min bei -78°C wurde eine Lösung von 5.45 g (25 mmol) [3-(Trifluormethyl)phenyl]essigsäuremethylester in 35 ml THF langsam hinzugetropft. Nach weiteren 20 min bei -78°C wurde eine Lösung von 7.20 g (30 mmol) *N*-Brommethylphthalimid in 50 ml THF tropfenweise hinzugefügt. Die Reaktionsmischung wurde zunächst 1 h bei -78°C, dann ohne Kältebad über Nacht bei RT weiter gerührt. Nach Zugabe von 100 ml 1 N Salzsäure wurde die Mischung dreimal mit Ethylacetat extrahiert. Die vereinigten organischen Phasen wurden mit ges. Natriumchlorid-Lösung gewaschen, über Natriumsulfat getrocknet und am Rotationsverdampfer vom Lösungsmittel befreit. Der Rückstand wurde in 50 ml DMSO gelöst und in mehreren Portionen durch präparative HPLC [Methode 8] gereinigt. Man erhielt so 2.20 g (22% d. Th.) der Titelverbindung.
LC/MS [Methode 3]: Rₜ = 1.32 min; m/z = 378 (M+H)⁺
¹H-NMR (400 MHz, DMSO-d₆): δ [ppm] = 3.33 (s, 3H), 4.07 (dd, 1H), 4.19 (dd, 1H), 4.33 (dd, 1H), 7.50-7.57 (m, 1H), 7.58-7.66 (m, 3H), 7.75-7.87 (m, 4H).

### Beispiel 17A

### tert.-Butyl-{3-hydroxy-2-[3-(trifluormethyl)phenyl]propyl} carbamat

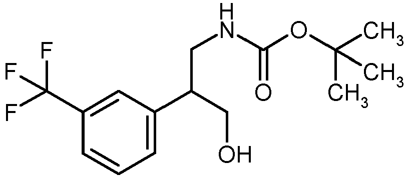

550 mg (1.46 mmol) der Verbindung aus Beispiel 16A wurden in 19 ml eines Gemisches von 2-Propanol und Wasser (6:1 v/v) vorgelegt und mit 276 mg (7.29 mmol) Natriumborhydrid versetzt. Die Reaktionsmischung wurde über Nacht bei RT gerührt und dann durch Zugabe von Eisessig auf pH 5 gestellt. Das resultierende Gemisch wurde auf 80°C erwärmt und über Nacht bei dieser Temperatur weiter gerührt. Eine analytische Probe zeigte laut LC/MS das freie 3-Amino-2-[3-(trifluormethyl)phenyl]propan-1-ol als Hauptkomponente [LC/MS (Methode 3): Rₜ = 1.02 min, m/z = 220 (M+H)⁺]. Nach leichtem Abkühlen wurde die Reaktionsmischung am Rotationsverdampfer von den flüchtigen Bestandteilen befreit. Nach jeweils zweimaliger Co-Evaporation mit Methanol und mit Toluol wurde der Rückstand im Hochvakuum getrocknet. Er wurde anschließend in 6 ml einer 1:1-Mischung von Acetonitril und Natriumbicarbonat-Lösung (5% in Wasser) gelöst und mit 402 µl (1.75 mmol) Di-*tert*.-butyldicarbonat versetzt. Diese Lösung wurde über Nacht bei RT gerührt und dann dreimal mit Ethylacetat extrahiert. Die vereinigten organischen Phasen wurden über Natriumsulfat getrocknet und am Rotationsverdampfer von den flüchtigen Bestandteilen befreit. Der Rückstand wurde durch präparative HPLC [Methode 8] gereinigt. Man erhielt so 351 mg (75% d. Th.) der Titelverbindung.
LC/MS [Methode 3]: Rₜ = 1.18 min; m/z = 220 (M+H-C₄H₈-CO₂)⁺
¹H-NMR (400 MHz, DMSO-d₆): δ [ppm] = 1.30 (s, 9H), 2.94-3.05 (m, 1H), 3.17-3.31 (m, 2H), 3.54-3.66 (m, 2H), 4.68 (t, 1H), 6.77 (t, 1H), 7.49-7.58 (m, 4H).

### Beispiel 18A

### 3-Amino-2-[3-(trifluormethyl)phenyl]propylcarbamat-Hydrochlorid

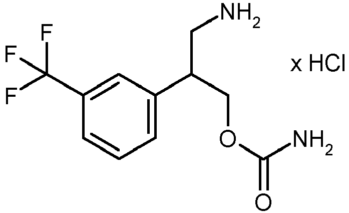

Eine Lösung von 350 mg (1.10 mmol) der Verbindung aus Beispiel 17A in 50 ml Acetonitril wurde auf -15°C abgekühlt und tropfenweise mit einer Lösung von 191 µl (2.19 mmol) Chlorsulfonylisocyanat in 10 ml Acetonitril versetzt. Nach 5 min wurden 50 ml Wasser zugegeben und die Mischung 4 h auf 60°C erhitzt. Nach Abkühlen auf RT wurden 50 ml einer ges. wässrigen Natriumbicarbonat-Lösung zugesetzt und die Mischung dreimal mit Ethylacetat extrahiert. Die vereinigten organischen Phasen wurden über Natriumsulfat getrocknet und am Rotationsverdampfer von den flüchtigen Bestandteilen befreit. Der Rückstand wurde mit 5 ml einer 4 N Lösung von Chlorwasserstoff in Dioxan versetzt und 5 min verrührt. Die flüchtigen Bestandteile wurden danach erneut am Rotationsverdampfer entfernt. Der Rückstand (385 mg) entsprach der rohen Titelverbindung in ca. 60% Reinheit laut LC/MS (ca. 70% d. Th.); dieses Material wurde ohne Reinigung weiter umgesetzt.
LC/MS [Methode 4]: Rₜ = 0.52 min; m/z = 263 (M+H)⁺.

### Beispiel 19A

### 3-[(tert.-Butoxycarbonyl)amino]-2-[3-(trifluormethyl)phenyl]propyl-ethylcarbamat

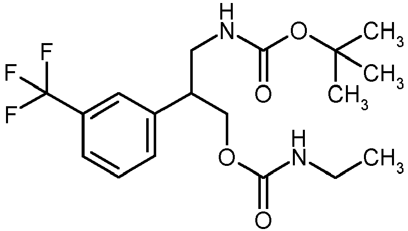

Eine Lösung von 134 mg (0.42 mmol) der Verbindung aus Beispiel 17A in 2.5 ml Pyridin wurde mit 5 mg (42 µmol) 4-*N,N-*Dimethylaminopyridin und 66 µl (0.84 mmol) Ethylisocyanat versetzt. Die Mischung wurde über Nacht bei RT gerührt. Danach wurden weitere 66 µl Ethylisocyanat hinzugefügt und die Mischung für 24 h auf 50°C erhitzt. Nach Abkühlen auf RT wurden 0.5 ml Ammoniaklösung (35% in Wasser) zugegeben. Die flüchtigen Bestandteile wurden am Rotationsverdampfer entfernt. Der Rückstand wurde mit wenig Acetonitril und 1 N Salzsäure versetzt und die Lösung durch präparative HPLC [Methode 9] aufgetrennt. Man erhielt 110 mg (67% d. Th.) der Titelverbindung.
LC/MS [Methode 4]: Rₜ = 1.15 min; m/z = 391 (M+H)⁺
¹H-NMR (400 MHz, DMSO-d₆): δ [ppm] = 0.95 (t, 3H), 1.29 (s, 9H), 2.94 (quin, 2H), 3.17-3.28 (m, 3H), 4.11-4.24 (m, 2H), 6.87 (br. t, 1H), 7.06 (t, 1H), 7.50-7.62 (m, 4H).

### Beispiel 20A

### 3-Amino-2-[3-(trifluormethyl)phenyl]propyl-ethylcarbamat-Hydrochlorid

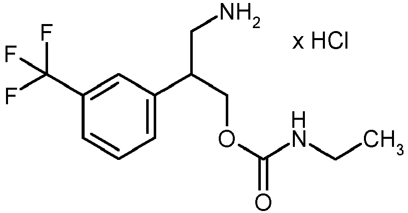

105 mg (0.27 mmol) der Verbindung aus Beispiel 19A wurden in 2 ml einer 4 N Lösung von Chlorwasserstoff in Dioxan gelöst und bei 60°C bis zur vollständigen Umsetzung gerührt (ca. 2 h). Die flüchtigen Bestandteile wurden anschließend am Rotationsverdampfer entfernt und der Rückstand im Hochvakuum getrocknet. Man erhielt 91 mg der Titelverbindung in ca. 83% Reinheit laut LC/MS (ca. 86% d. Th.); dieses Material wurde ohne zusätzliche Reinigung weiter umgesetzt.
LC/MS [Methode 4]: Rₜ = 0.65 min; m/z = 291 (M+H)⁺.

### Beispiel 21A

### tert.-Butyl-[(2,3-dichlorphenyl)(phenylsulfonyl)methyl]carbamat

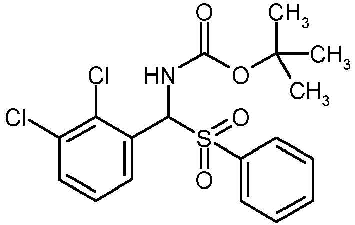

2.23 g (19.0 mmol) *tert*.-Butylcarbamat und 6.25 g (38.1 mmol) Benzolsulfinsäure-Natriumsalz wurden bei RT in 55 ml Methanol/Wasser (1:2) vorgelegt und mit 5.00 g (28.6 mmol) 2,3-Dichlorbenzaldehyd, dann mit 1.43 ml (37.9 mmol) Ameisensäure versetzt. Die Reaktionsmischung wurde 2 Tage bei RT gerührt. Der ausgefallene Feststoff wurde abgesaugt und mit Wasser sowie zweimal mit Diethylether gewaschen. Aus dem etherischen Filtrat wurden nach Entfernung des Lösungsmittels am Rotationsverdampfer 3.47 g 2,3-Dichlorbenzaldehyd zurückgewonnen. Der abgesaugte Feststoff wurde im Hochvakuum getrocknet. Man erhielt 2.22 g (19% d. Th.) der Titelverbindung.
¹H-NMR (400 MHz, DMSO-d₆): δ [ppm] = 8.93 (d, 1H), 7.96 (d, 1H), 7.84 (d, 2H), 7.76 (d, 2H), 7.63-7.71 (m, 2H), 7.51 (t, 1H), 6.60 (d, 1H), 1.21 (s, 9H).

### Beispiel 22A

### tert.-Butyl-[(E)-(2,3-dichlorphenyl)methyliden]carbamat

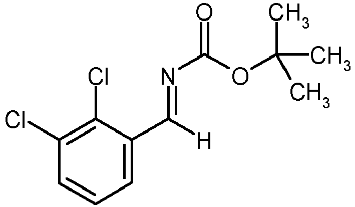

4.42 g (32.0 mmol) Kaliumcarbonat wurden im Hochvakuum heiß getrocknet und dann unter Argonatmosphäre auf RT abgekühlt. Dazu wurden 52 ml wasserfreies THF und 2.22 g (5.33 mmol) der Verbindung aus Beispiel 21A gegeben. Die Reaktionsmischung wurde anschließend 16 h bei Rückflusstemperatur gerührt. Nach Abkühlen auf RT wurde das Reaktionsgemisch über Celite filtriert. Der Feststoff wurde mit etwas THF nachgewaschen. Die vereinigten Filtrate wurden am Rotationsverdampfer vom Lösungsmittel befreit und der Rückstand im Hochvakuum getrocknet. Man erhielt 1.38 g (94% d. Th.) der Titelverbindung.
MS: m/z = 274 (M+H)⁺
¹H-NMR (400 MHz, DMSO-d₆): δ [ppm] = 9.11 (s, 1H), 8.01 (d, 1H), 7.92 (d, 1H), 7.52 (t, 1H), 1.52 (s, 9H).

### Beispiel 23A

### tert.-Butyl-[1-(2,3-dichlorphenyl)-2-nitroethyl]carbamat

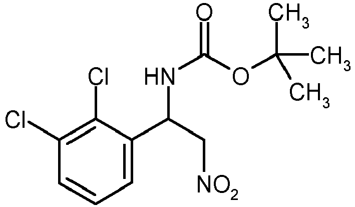

10.1 ml (186 mmol) Nitromethan wurden mit 263 µl (1.51 mmol) *N,N-*Diisopropylethylamin versetzt und die gelbe Lösung 1 h bei RT gerührt. Anschließend wurden 1.38 g (5.03 mmol) der Verbindung aus Beispiel 22A hinzugefügt und die Mischung bei RT über Nacht gerührt. Alle flüchtigen Bestandteile wurden am Rotationsverdampfer entfernt. Der Rückstand wurde durch präparative HPLC [Methode 12] gereinigt. Man erhielt 865 mg (51% d. Th.) der Titelverbindung.
LC/MS [Methode 4]: Rₜ = 1.17 min; m/z = 333 (M-H)⁻
¹H-NMR (400 MHz, DMSO-d₆): δ [ppm] = 8.07 (d, 1H), 7.64 (d, 1H), 7.50 (d, 1H), 7.44 (t, 1H), 5.74 (t, 1H), 4.87 (d, 1H), 4.62 (t, 1H), 1.34 (s, 9H).

### Beispiel 24A

### tert.-Butyl-[2-amino-1-(2,3-dichlorphenyl)ethyl]carbamat

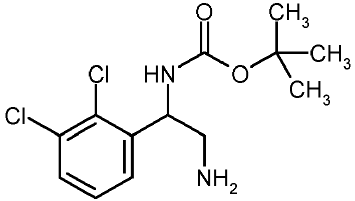

Eine Lösung von 440 mg (1.31 mmol) der Verbindung aus Beispiel 23A in 100 ml Methanol wurde in einer mit einer Raney-Nickel-Kartusche ausgestatteten Durchfluss-Hydrierapparatur (H-Cube, Firma Thales Nano, Budapest, Modell HC-2-SS) bei einem Durchfluss von 1 ml/min, einer Temperatur von 40°C und bei Normaldruck hydriert. Nach beendeter Umsetzung wurde die Lösung am Rotationsverdampfer vom Methanol befreit und der Rückstand kurz im Hochvakuum getrocknet. Es wurden 370 mg (91% d. Th.) der Titelverbindung erhalten.
LC/MS [Methode 4]: Rₜ = 0.76 min; m/z = 305 (M+H)⁺
¹H-NMR (400 MHz, DMSO-d₆): δ [ppm] = 7.55 (br. d, 1H), 7.51 (dd, 1H), 7.31-7.39 (m, 2H), 4.81-4.89 (m, 1H), 2.72 (dd, 1H), 2.59 (d, 1H), 1.66 (br. s, 2H), 1.36 (s, 9H).

### Beispiel 25A

### 1-(2-Chlorphenyl)-2-nitroethanamin-Hydrochlorid

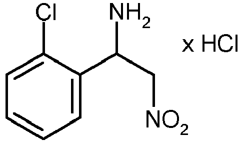

Eine Lösung von 1.037 g (3.45 mmol) *tert*.-Butyl-[1-(2-chlorphenyl)-2-nitroethyl]carbamat [siehe z.B. Tetrahedron Lett. 2009, 50 (9), 1016] in 10 ml Dichlormethan wurde mit 10 ml einer 4 N Lösung von Chlorwasserstoff in Dioxan versetzt und 2 h bei RT gerührt. Anschließend wurden die flüchtigen Bestandteile am Rotationsverdampfer entfernt. Der Rückstand wurde mit 5 ml Dichlormethan versetzt, erneut am Rotationsverdampfer vom Lösungsmittel befreit und dann im Hochvakuum getrocknet. Man erhielt 898 mg der Titelverbindung (quant., noch ca. 8% Dioxan laut ¹H-NMR enthaltend).
LC/MS [Methode 4]: Rₜ = 0.34 min; m/z = 200 (M+H)⁺
¹H-NMR (400 MHz, DMSO-d₆): δ [ppm] = 5.18 (dd, 1H), 5.31 (dd, 1H), 5.38 (dd, 1H), 7.44-7.53 (m, 2H), 7.56-7.63 (m, 1H), 7.82-7.91 (m, 1H), 9.10 (br. s, 3H).

### Beispiel 26A

### N-[1-(2-Chlorphenyl)-2-nitroethyl]methansulfonamid

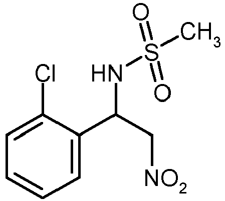

Eine Lösung von 300 mg (ca. 93% Reinheit, 1.18 mmol) der Verbindung aus Beispiel 25A in 7.2 ml Pyridin wurde bei RT mit 182 µl (2.35 mmol) Methansulfonsäurechlorid versetzt und 1 h gerührt. Das Pyridin wurde am Rotationsverdampfer entfernt und der Rückstand über präparative HPLC [Methode 20] gereinigt. Man erhielt 221 mg (65% d. Th.) der Titelverbindung.
LC/MS [Methode 4]: Rₜ = 0.82 min; m/z = 279 (M+H)⁺
¹H-NMR (400 MHz, DMSO-d₆): δ [ppm] = 2.68 (s, 3H), 4.68 (dd, 1H), 4.91 (dd, 1H), 5.57 (td, 1H), 7.37-7.48 (m, 2H), 7.52 (dd, 1H), 7.70 (dd, 1H), 8.47 (d, 1H).

### Beispiel 27A

### N-[2-Amino-1-(2-chlorphenyl)ethyl]methansulfonamid

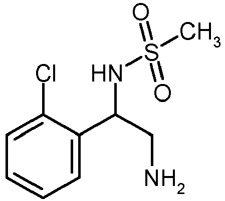

Eine Lösung von 221 mg (0.79 mmol) der Verbindung aus Beispiel 26A in 45 ml Methanol wurde in einer mit einer Raney-Nickel-Kartusche ausgestatteten Durchfluss-Hydrierapparatur (H-Cube, Firma Thales Nano, Budapest, Modell HC-2-SS) bei einem Durchfluss von 1 ml/min, einer Temperatur von 45°C und bei Normaldruck hydriert. Nach beendeter Umsetzung wurde die Lösung am Rotationsverdampfer vom Methanol befreit und der Rückstand kurz im Hochvakuum getrocknet. Es wurden 186 mg (94% d. Th.) der Titelverbindung erhalten.
LC/MS [Methode 4]: Rₜ = 0.33 min; m/z = 249 (M+H)⁺
¹H-NMR (400 MHz, DMSO-d₆): δ [ppm] = 2.60 (dd, 1H), 2.77 (dd, 1H), 2.81 (s, 3H), 3.50-4.40 (br. s, 3H), 4.69 (dd, 1H), 7.25-7.32 (m, 1H), 7.33-7.44 (m, 2H), 7.54 (dd, 1H).

### Beispiel 28A

### N-[1-(2-Chlorphenyl)-2-nitroethyl]ethansulfonamid

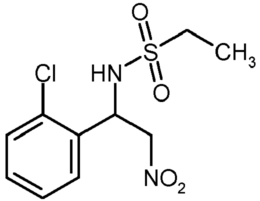

Eine Lösung von 295 mg (ca. 93% Reinheit, 1.16 mmol) der Verbindung aus Beispiel 25A in 7.0 ml Pyridin wurde bei RT mit 219 µl (2.31 mmol) Ethansulfonsäurechlorid versetzt und 1 h gerührt. Das Pyridin wurde dann am Rotationsverdampfer entfernt und der Rückstand über präparative HPLC [Methode 20] gereinigt. Man erhielt 230 mg (ca. 90% Reinheit laut LC/MS, 61% d. Th.) der Titelverbindung.
LC/MS [Methode 4]: Rₜ = 0.88 min; m/z = 293 (M+H)⁺
¹H-NMR (400 MHz, DMSO-d₆): δ [ppm] = 1.02 (t, 3H), 2.68 (dq, 1H), 2.82 (dq, 1H), 4.67 (dd, 1H), 4.89 (dd, 1H), 5.55 (td, 1H), 7.36-7.48 (m, 2H), 7.49-7.53 (m, 1H), 7.69-7.74 (m, 1H), 8.45 (d, 1H).

### Beispiel 29A

### N-[2-Amino-1-(2-chlorphenyl)ethyl]ethansulfonamid

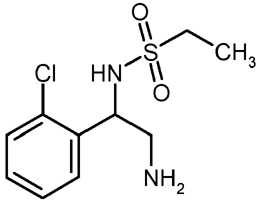

Analog zu Beispiel 27A wurden aus 230 mg (0.79 mmol) der Verbindung aus Beispiel 28A 190 mg (ca. 90% Reinheit laut LC/MS, 83% d. Th.) der Titelverbindung erhalten.
LC/MS [Methode 4]: Rₜ = 0.43 min; m/z = 263 (M+H)⁺
¹H-NMR (400 MHz, DMSO-d₆): δ [ppm] = 1.11 (t, 3H), 2.62 (dd, 1H), 2.69-2.84 (m, 2H), 2.93 (dq, 1H), ca. 3.35-4.50 (br. s, 3H), 4.69 (dd, 1H), 7.29 (dt, 1H), 7.33-7.46 (m, 2H), 7.56 (dd, 1H).

### Beispiel 30A

### 1-[1-(2-Chlorphenyl)-2-nitroethyl]harnstoff

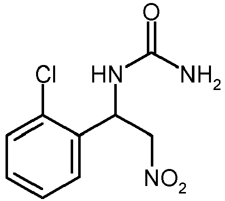

300 mg (ca. 93% Reinheit, 1.18 mmol) der Verbindung aus Beispiel 25A wurden bei RT in 12 ml Wasser/Methanol (1:1 v/v) vorgelegt und mit 287 mg (3.53 mmol) Kaliumcyanat versetzt. Die Reaktionsmischung wurde 1 h auf 40°C erwärmt. Nach Abkühlung auf RT wurde das Gemisch direkt mittels präparativer HPLC [Methode 8] in seine Komponenten aufgetrennt. Man erhielt so 120 mg (41% d. Th.) der Titelverbindung.
LC/MS [Methode 4]: Rₜ = 0.67 min; m/z = 244 (M+H)⁺
¹H-NMR (400 MHz, DMSO-d₆): δ [ppm] = 4.73 (dd, 1H), 4.88 (dd, 1H), 5.71 (br. s, 2H), 5.77 (dt, 1H), 6.99 (d, 1H), 7.35 (dt, 1H), 7.40 (dt, 1H), 7.48 (dd, 1H), 7.51 (dd, 1H).

### Beispiel 31A

### 1-[2-Amino-1-(2-chlorphenyl)ethyl]harnstoff

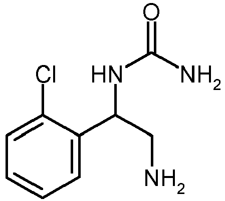

Analog zu Beispiel 27A wurden aus 120 mg (0.49 mmol) der Verbindung aus Beispiel 30A 94 mg (72% d. Th.) der Titelverbindung erhalten.
LC/MS [Methode 4]: Rₜ = 0.24 min; m/z = 214 (M+H)⁺
¹H-NMR (400 MHz, DMSO-d₆): δ [ppm] = 2.64 (dd, 1H), 2.79 (dd, 1H), 3.32 (br. s, 2H), 4.93 (td, 1H), 5.57 (br. s, 2H), 6.70 (d, 1H), 7.21-7.27 (m, 1H), 7.29-7.41 (m, 3H).

### Beispiel 32A

### Methyl-{3-(4-chlorphenyl)-5-oxo-4-[(1E)-3,3,3-trifluorprop-1-en-1-yl]-4,5-dihydro-1H-1,2,4-triazol-1-yl}acetat

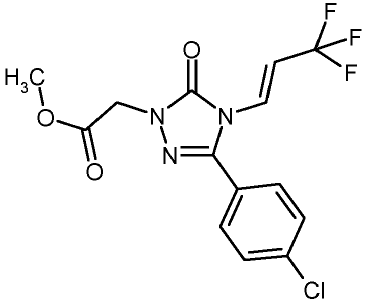

280 mg (0.74 mmol) der Verbindung aus Beispiel 7A wurden bei RT zusammen mit 108 mg (0.89 mmol) 4-*N,N*-Dimethylaminopyridin in 5.3 ml Pyridin vorgelegt, portionsweise mit 0.31 ml (1.84 mmol) Trifluormethansulfonsäureanhydrid versetzt und 12 h gerührt. Das Pyridin wurde anschließend am Rotationsverdampfer entfernt. Der Rückstand wurde in Acetonitril und 1 N Salzsäure aufgenommen und über präparative HLPC [Methode 9] gereinigt. Es wurden 230 mg (86% d. Th.) der Titelverbindung erhalten.
LC/MS [Methode 4]: Rₜ = 1.14 min; m/z = 362 (M+H)⁺
¹H-NMR (400 MHz, DMSO-d₆): δ [ppm] = 3.72 (s, 3H), 4.78 (s, 2H), 6.85 (dd, 1H), 7.18 (d, 1H), 7.68 (s, 4H).

### Beispiel 33A

### {3-(4-Chlorphenyl)-5-oxo-4-[(1E)-3,3,3-trifluorprop-1-en-1-yl]-4,5-dihydro-1H-1,2,4-triazol-1-yl}-essigsäure

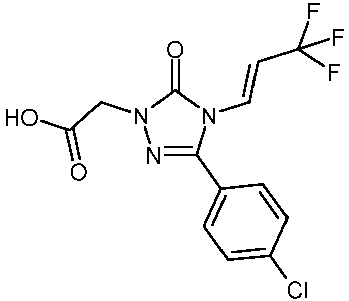

260 mg (0.72 mmol) der Verbindung aus Beispiel 32A wurden in 5 ml Methanol gelöst und mit 2.87 ml (2.87 mmol) einer 1 M Lösung von Lithiumhydroxid in Wasser versetzt. Die Mischung wurde 1 h bei RT gerührt, danach mit 1 N Salzsäure angesäuert und mit DMSO verdünnt. Diese Lösung wurde dann direkt über präparative HLPC [Methode 9] gereinigt. Es wurden 215 mg (86% d. Th.) der Titelverbindung erhalten.
LC/MS [Methode 4]: Rₜ = 1.03 min; m/z = 348 (M+H)⁺
¹H-NMR (400 MHz, DMSO-d₆): δ [ppm] = 4.64 (s, 2H), 6.79-6.92 (m, 1H), 7.19 (dd, 1H), 7.68 (s, 4H), 13.31 (br. s, 1H).

### Beispiel 34A

### 2-Amino-2-[3-(trifluormethyl)phenyl]propanamid

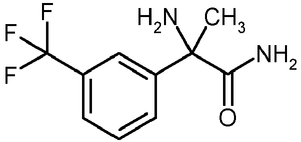

138 ml Wasser, 108 ml 25%-ige wässrige Ammoniaklösung und 173 ml Ethanol wurden vorgelegt. Zu dieser Mischung wurden 108 g (574 mmol) 1-[3-(Trifluormethyl)phenyl]ethanon, 30 g (574 mmol) Natriumcyanid und 31 g (631 mmol) Ammoniumchlorid gegeben. Die Mischung wurde für 20 h in einem Autoklaven bei 70°C gerührt. Danach wurde das Ethanol am Rotationsverdampfer abgezogen und der Rückstand viermal mit je 500 ml Diethylether extrahiert. Die vereinigten organischen Phasen wurden mit Magnesiumsulfat und Aktivkohle versetzt und über Kieselgur abgesaugt. Das Filtrat wurde am Rotationsverdampfer eingeengt und der Rückstand durch Chromatographie an 2 kg Kieselgel 60 gereinigt (Laufmittel: Cyclohexan/Ethylacetat 3:1 bis 1:1).

Das so erhaltene Zwischenprodukt 2-Amino-2-[3-(trifluormethyl)phenyl]propannitril [56 g, 46% d. Th.; ¹H-NMR (400 MHz, CDCl₃): δ [ppm] = 1.78 (s, 3H), 2.14 (br. s, 2H), 7.55 (t, 1H), 7.63 (d, 1H), 7.88 (d, 1H), 7.96 (s, 1H)] wurde unter Eiskühlung langsam mit 500 ml konzentrierter Salzsäure versetzt. Die Suspension wurde über Nacht bei RT gerührt. Anschließend wurde am Rotationsverdampfer das Volumen auf ca. 150 ml reduziert. Es wurden 250 ml Aceton zugegeben und dann alle flüchtigen Komponenten am Rotationsverdampfer entfernt. Der verbliebene feste Brei wurde unter Eiskühlung mit 125 ml konzentrierter Ammoniaklösung versetzt. Es wurde 30 min im Eisbad gerührt. Die entstandenen Kristalle wurden abgesaugt, gut abgepresst und zweimal mit je 50 ml Eiswasser, dann mit Pentan gewaschen. Das Produkt wurde im Hochvakuum getrocknet. Es wurden 43 g (32% d. Th.) der Titelverbindung erhalten.
MS (ESIpos): m/z = 233 (M+H)⁺
¹H-NMR (400 MHz, CDCl₃): δ [ppm] = 1.82 (s, 3H), 1.85 (br. s, 2H), 5.54 (br. s, 1H), 7.26 (br. s, 1H), 7.48 (t, 1H), 7.55 (d, 2H), 7.75 (d, 1H), 7.83 (s, 1H).

### Beispiel 35A

### 2-[3-(Trifluormethyl)phenyl]propan-1,2-diamin-Dihydrochlorid

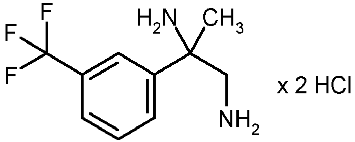

Eine Suspension von 500 mg (2.15 mmol) der Verbindung aus Beispiel 34A in 20 ml Diethylether wurde auf 0°C gekühlt und langsam mit 3.72 ml Lithiumaluminiumhydrid-Lösung (1 M in Diethylether, 3.72 mmol) versetzt. Nach 15 min wurde das Kühlbad entfernt und die Reaktionsmischung über Nacht bei RT gerührt. Unter Eiskühlung wurden 5 ml einer 5%-igen wässrigen Kaliumnatriumtartrat-Lösung langsam zugetropft. Die Mischung wurde mit 40 ml Diethylether und 40 ml 5%-iger Kaliumnatriumtartrat-Lösung verdünnt und dann ausgeschüttelt. Die wässrige Phase wurde noch zweimal mit Diethylether extrahiert. Die vereinigten organischen Phasen wurden über Natriumsulfat getrocknet, mit 3 ml einer 4 N Lösung von Chlorwasserstoff in Dioxan versetzt und dann am Rotationsverdampfer von den flüchtigen Bestandteilen befreit. Der Rückstand (153 mg) enthielt laut LC/MS die Titelverbindung zu ca. 35% und wurde als solches, ohne weitere Reinigung, weiter umgesetzt.
LC/MS [Methode 2]: Rₜ = 0.23 min; m/z = 219 (M+H)⁺.

### Beispiel 36A

### tert.-Butyl-{2-hydroxy-2-[2-(trifluormethyl)phenyl]ethyl}carbamat

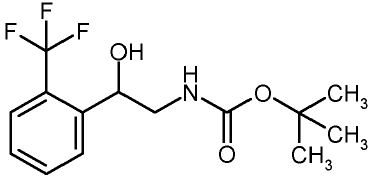

Eine Lösung von 430 mg (2.1 mmol) 2-Hydroxy-2-[(2-trifluormethyl)phenyl]ethanamin in 20 ml Dichlormethan wurde bei RT mit 963 µl (4.19 mmol) Di-*tert*.-butyldicarbonat versetzt und 3 h gerührt. Die Reaktionsmischung wurde danach mit 100 ml Ethylacetat verdünnt und je zweimal mit 1 M Salzsäure, ges. Natriumbicarbonat-Lösung und ges. Natriumchlorid-Lösung gewaschen. Die organische Phase wurde über Natriumsulfat getrocknet und am Rotationsverdampfer vom Lösungsmittel befreit. Der Rückstand wurde über präparative HPLC [Methode 9] gereinigt. Man erhielt 470 mg (73% d. Th.) der Titelverbindung.
LC/MS [Methode 4]: Rₜ = 1.05 min; m/z = 306 (M+H)⁺
¹H-NMR (400 MHz, DMSO-d₆): δ [ppm] = 1.31 (s, 9H), 3.04-3.18 (m, 2H), 4.64-4.73 (m, 1H), 5.59 (d, 1H), 6.79 (t, 1H), 7.52-7.65 (m, 4H).

### Beispiel 37A

### 2-Amino-1-[2-(trifluormethyl)phenyl]ethylcarbamat

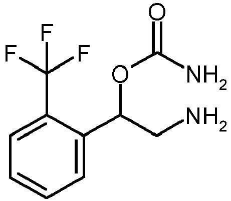

Eine Lösung von 420 mg (1.38 mmol) der Verbindung aus Beispiel 36A in 100 ml Acetonitril wurde auf -15°C gekühlt und tropfenweise mit einer Lösung von 168 µl (1.93 mmol) Chlorsulfonylisocyanat in 10 ml Acetonitril versetzt. Nach 5 min wurden 50 ml Wasser zugegeben und die Reaktionsmischung über Nacht bei 60°C weiter gerührt. Nach Abkühlung auf RT wurden 50 ml einer gesättigten wässrigen Natriumbicarbonat-Lösung zugesetzt. Die Mischung wurde dreimal mit Ethylacetat extrahiert. Die vereinigten organischen Phasen wurden über Natriumsulfat getrocknet und am Rotationsverdampfer vom Lösungsmittel befreit. Der Rückstand (321 mg, ca. 90% Reinheit, 85% d. Th.) entsprach der Titelverbindung und wurde ohne zusätzliche Reinigung weiter umgesetzt.
LC/MS [Methode 4]: Rₜ = 0.49 min; m/z = 249 (M+H)⁺.

### Beispiel 38A

### tert.-Butyl-{2-hydroxy-2-[3-(trifluormethyl)phenyl]ethyl}carbamat

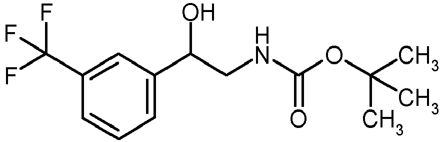

Eine Lösung von 85 mg (0.41 mmol) 2-Hydroxy-2-[(3-trifluormethyl)phenyl]ethanamin [zur Herstellung siehe J. Med. Chem. 1968, 11, 1258-1262] in 3.2 ml Dioxan wurde bei RT mit 3.2 ml einer 5%-igen wässrigen Natriumbicarbonat-Lösung und 91 mg (0.41 mmol) Di-*tert*.-butyldicarbonat versetzt und bis zur vollständigen Umsetzung gerührt. Die Reaktionsmischung wurde danach dreimal mit Ethylacetat extrahiert. Die vereinigten organischen Phasen wurden über Natriumsulfat getrocknet und am Rotationsverdampfer vom Lösungsmittel befreit. Der Rückstand (88 mg, 70% d. Th.) entsprach der Titelverbindung.
LC/MS [Methode 4]: Rₜ = 1.03 min; m/z = 304 (M-H)⁻
¹H-NMR (400 MHz, DMSO-d₆): δ [ppm] = 1.31 (s, 9H), 3.04-3.19 (m, 2H), 4.61-4.74 (m, 1H), 5.59 (d, 1H), 6.79 (t, 1H), 7.50-7.67 (m, 4H).

### Beispiel 39A

### 2-Amino-1-[3-(trifluormethyl)phenyl]ethylcarbamat-Hydrochlorid

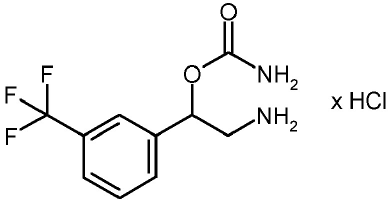

Analog zu Beispiel 37A wurden 85 mg (0.28 mmol) der Verbindung aus Beispiel 38A umgesetzt. Das so erhaltene 2-Amino-1-[3-(trifluormethyl)phenyl]ethylcarbamat wurde für 5 min mit 2 ml einer 4 N Lösung von Chlorwasserstoff in Dioxan verrührt. Die flüchtigen Bestandteile wurden am Rotationsverdampfer entfernt und der Rückstand im Hochvakuum getrocknet. Es wurden 73 mg (85% d. Th.) der Titelverbindung erhalten.
LC/MS [Methode 2]: Rₜ = 1.08 min; m/z = 249 (M+H)⁺
¹H-NMR (400 MHz, DMSO-d₆): δ [ppm] = 3.16-3.29 (m, 2H), 5.82 (dd, 1H), 6.79 (br. s, 2H), 7.63-7.78 (m, 4H), 8.18 (br. s, 3H).

### Beispiel 40A

### 2-Amino-1-(2-chlorphenyl)ethylcarbamat

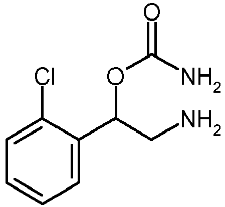

Analog zu Beispiel 37A wurden aus 114 mg (0.42 mmol) *tert*.-Butyl-[2-(2-chlorphenyl)-2-hydroxy-ethyl]carbamat 46 mg (51% d. Th.) der Titelverbindung erhalten.
LC/MS [Methode 2]: Rₜ = 0.88 min; m/z = 215 (M+H)⁺
¹H-NMR (400 MHz, DMSO-d₆): δ [ppm] = 2.73 (dd, 1H), 2.86 (dd, 1H), 5.71 (dd, 1H), 6.65 (br. s, 2H), 7.28-7.33 (m, 1H), 7.34-7.41 (m, 2H), 7.43 (dd, 1H).

### Beispiel 41A

### 2-Amino-1-(2,3-dichlorphenyl)ethanon-Hydrochlorid

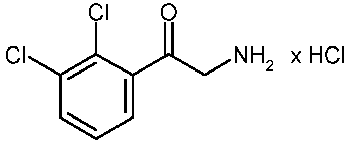

1.0 g (3.73 mmol) 2-Brom-1-(2,3-dichlorphenyl)ethanon [zur Herstellung siehe z.B. US-Patent 5,831,132] wurde in 4 ml Acetonitril bei RT mit 411 mg (4.33 mmol) Natriumdiformylamid versetzt und die Mischung über Nacht bei RT gerührt. Anschließend wurde die Mischung auf 70°C erhitzt und warm filtriert. Der zurückgebliebene Feststoff wurde mit 2 ml heißem Acetonitril gewaschen. Die kombinierten Filtrate wurden am Rotationsverdampfer vom Lösungsmittel befreit. Der dunkelbraune ölige Rückstand wurde mit 10 ml einer 5%-igen ethanolischen Chlorwasserstoff-Lösung versetzt und über Nacht bei RT gerührt. Die flüchtigen Bestandteile wurden am Rotationsverdampfer entfernt und der verbliebene gelbe Feststoff in 20 ml siedendem Diethylether verrührt. Nach Abkühlung auf RT wurde der Feststoff durch Filtration isoliert, mit Diethylether gewaschen und im Hochvakuum getrocknet. Man erhielt so 410 mg (46% d. Th.) der Titelverbindung.
LC/MS [Methode 21]: Rₜ = 0.78 min; m/z = 204 (M+H)⁺
¹H-NMR (400 MHz, DMSO-d₆): δ [ppm] = 4.51 (br. s, 2H), 7.57 (t, 1H), 7.88 (dd, 1H), 7.92 (dd, 1H), 8.52 (br. s, 3H).

### Beispiel 42A

### 2-Amino-1-(2,3-dichlorphenyl)ethanol

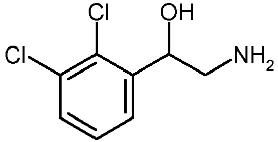

300 mg (1.25 mmol) der Verbindung aus Beispiel 41A wurden in 2 ml Methanol unter Argon vorgelegt. 189 mg (5.0 mmol) Natriumborhydrid wurden zugegeben und die Mischung über Nacht gerührt. Nach langsamer Zugabe von 1 N Salzsäure bis zum Ende der Gasentwicklung wurde das Methanol am Rotationsverdampfer entfernt. Der wässrige Rückstand wurde durch Zugabe von Natriumbicarbonat-Lösung alkalisch gestellt und dreimal mit Ethylacetat extrahiert. Die vereinigten organischen Phasen wurden über Natriumsulfat getrocknet und am Rotationsverdampfer vom Lösungsmittel befreit. Der Rückstand (189 mg, 74% d. Th.) entsprach der Titelverbindung.
LC/MS [Methode 4]: Rₜ = 0.48 min; m/z = 206 (M+H)⁺
¹H-NMR (400 MHz, DMSO-d₆): δ [ppm] = 1.60-1.99 (br. s, 2H), 2.45-2.52 (dd, 1H), 2.77 (dd, 1H), 4.83 (dd, 1H), 5.62 (br. s, 1H), 7.34-7.40 (m, 1H), 7.53 (d, 2H).

### Beispiel 43A

### tert.-Butyl-[2-(2,3-dichlorphenyl)-2-hydroxyethyl]carbamat

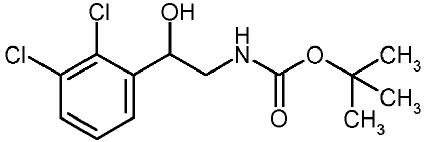

Eine Lösung von 126 mg (0.61 mmol) der Verbindung aus Beispiel 42A in 5 ml Acetonitril und 5 ml Dichlormethan wurde bei RT mit 281 µl (1.22 mmol) Di-*tert*.-butyldicarbonat versetzt und über Nacht gerührt. Die flüchtigen Bestandteile wurden am Rotationsverdampfer entfernt und der Rückstand durch präparative HPLC [Methode 9] gereinigt. Man erhielt 117 mg (62% d. Th.) der Titelverbindung.
LC/MS [Methode 4]: Rₜ = 1.05 min; m/z = 306 (M+H)⁺
¹H-NMR (400 MHz, DMSO-d₆): δ [ppm] = 1.33 (s, 9H), 3.03-3.21 (m, 2H), 4.97-5.05 (m, 1H), 5.65 (d, 1H), 6.80 (t, 1H), 7.37 (t, 1H), 7.52 (d, 2H).

### Beispiel 44A

### 2-Amino-1-(2,3-dichlorphenyl)ethylcarbamat

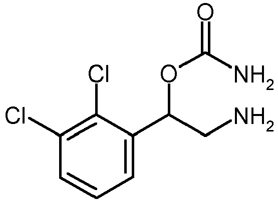

Analog zu Beispiel 37A wurden aus 117 mg (0.38 mmol) der Verbindung aus Beispiel 43A 62 mg (63% d. Th.) der Titelverbindung erhalten.
LC/MS [Methode 4]: Rₜ = 0.52 min; m/z = 263 (M+H)⁺
¹H-NMR (400 MHz, DMSO-d₆): δ [ppm] = 1.57 (br. s, 2H), 2.72 (dd, 1H), 2.85 (dd, 1H), 5.69 (dd, 1H), 6.44-6.97 (2 br. s, 2H), 7.35 (dd, 1H), 7.40 (t, 1H), 7.58 (dd, 1H).

### Beispiel 45A

### (2R)-2-Amino-3-[3-(trifluormethyl)phenyl]propan-1-ol-Hydrochlorid

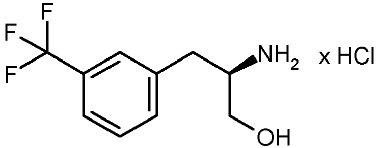

Eine Suspension von 500 mg (2.14 mmol) (2*R*)-2-Amino-3-[(3-trifluormethyl)phenyl]propancarbonsäure in 20 ml Diethylether wurde bei 0°C langsam mit 4.29 ml Lithiumaluminiumhydrid-Lösung (1 M in Diethylether, 4.29 mmol) versetzt. Die Reaktionsmischung wurde 15 min bei 0°C gerührt und dann über Nacht unter Rückfluss erhitzt. Nach Abkühlung auf 0°C wurden 5 ml einer 5%-igen wässrigen Natriumaluminiumtartrat-Lösung zugetropft. Die Mischung wurde mit 40 ml Diethylether und 40 ml 5%-iger Natriumaluminiumtartrat-Lösung verdünnt und dann ausgeschüttelt. Die wässrige Phase wurde noch zweimal mit Diethylether extrahiert. Die vereinigten organischen Phasen wurden über Natriumsulfat getrocknet, mit 3 ml einer 4 N Lösung von Chlorwasserstoff in Dioxan versetzt und am Rotationsverdampfer von den flüchtigen Bestandteilen befreit. Der Rückstand wurde im Hochvakuum getrocknet. Es wurden 410 mg (67% d. Th., ca. 90% Reinheit laut LC/MS) der Titelverbindung erhalten.
LC/MS [Methode 1]: Rₜ = 0.59 min; m/z = 220 (M+H)⁺
¹H-NMR (400 MHz, DMSO-d₆): δ [ppm] = 2.91-3.08 (m, 2H), 3.30-3.45 (m, 2H), 3.48-3.58 (m, 1H), 5.40 (br. s, 1H), 7.51-7.65 (m, 3H), 7.67 (s, 1H), 8.16 (br. m, 3H).

### Beispiel 46A

### (2S)-2-Amino-3-[3-(trifluormethyl)phenyl]propan-1-ol-Hydrochlorid

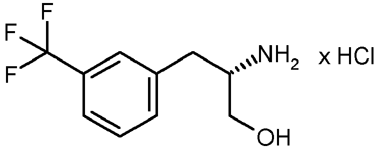

Analog zu Beispiel 45A wurden 500 mg (2*S*)-2-Amino-3-[(3-trifluormethyl)phenyl]propancarbonsäure umgesetzt. Man erhielt 517 mg (89% d. Th.) der Titelverbindung.
LC/MS [Methode 1]: Rₜ = 0.59 min; m/z = 220 (M+H)⁺
¹H-NMR (400 MHz, DMSO-d₆): δ [ppm]= 2.91-3.06 (m, 2H), 3.34-3.43 (m, 2H), 3.49-3.58 (m, 1H), 5.37 (br. s, 1H), 7.51-7.65 (m, 3H), 7.67 (s, 1H), 8.07-8.24 (br. m, 3H).

### Beispiel 47A

### (2R)-2-Amino-3-[3-(trifluormethyl)phenyl]propylcarbamat-Hydrochlorid

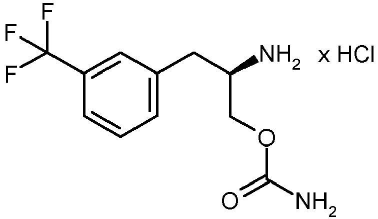

Eine Lösung von 100 mg (0.31 mmol) (2*R*)-*tert*.-Butyl-{1-hydroxy-3-[3-(trifluormethyl)phenyl]-propan-2-yl}carbamat (zur Herstellung siehe US-Patent Application 2008/0242694, Beispiel [0440]) in 2 ml Acetonitril wurde bei -15°C langsam mit 38 µl (0.44 mmol) Chlorsulfonylisocyanat versetzt. Die Reaktionsmischung wurde 1 h bei RT gerührt, dann mit 3 ml Wasser versetzt und über Nacht unter Rückfluss erhitzt. Nach Abkühlung auf RT wurde die Mischung direkt durch präparative HPLC [Methode 9] aufgereinigt. Man erhielt eine erste Fraktion (21 mg) der Titelverbindung (zum Teil als Formiat-Salz) sowie eine Fraktion (17 mg) der entsprechenden noch Bocgeschützten Verbindung. Die letztere Fraktion wurde 30 min mit 3 ml einer 4 N Lösung von Chlorwasserstoff in Dioxan verrührt und dann am Rotationsverdampfer und schließlich im Hochvakuum von den flüchtigen Bestandteilen befreit. Der Rückstand (16 mg) entsprach der reinen Titelverbindung. Beide Fraktionen der Titelverbindung wurden vereinigt und zur Herstellung nachfolgender Verbindungen eingesetzt.
LC/MS [Methode 1]: Rₜ = 0.76 min; m/z = 263 (M+H)⁺
¹H-NMR (400 MHz, DMSO-d₆): δ [ppm] = 2.91-3.11 (m, 2H), 3.63-3.75 (m, 1H), 3.90 (dd, 1H), 4.05 (dd, 1H), 6.64 (br. s, 2H), 7.55-7.70 (m, 4H), 8.19 (br. s, 3H).

### Beispiel 48A

### (2S)-2-Amino-3-[3-(trifluormethyl)phenyl]propylcarbamat-Hydrochlorid

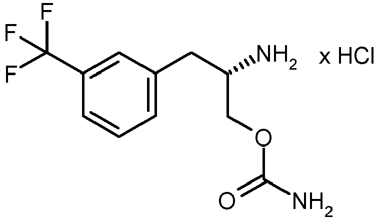

Eine Lösung von 160 mg (0.50 mmol) (2*S*)-*tert*.-Butyl-{1-hydroly-3-[3-(trifluormethyl)phenyl]-propan-2-yl}carbamat (zur Herstellung siehe US-Patent Application 2008/0242694, Beispiel [0464]) in 7.6 ml Acetonitril wurde bei -15°C langsam mit 51 µl (0.70 mmol) Chlorsulfonylisocyanat versetzt. Die Reaktionsmischung wurde 1 h bei RT gerührt, dann mit 1 ml Wasser versetzt und über Nacht auf 60°C erhitzt. Eine analytische Probe zeigte laut LC/MS komplette Umsetzung zum Zielprodukt. Nach Abkühlung auf RT wurden die flüchtigen Bestandteile am Rotationsverdampfer und schließlich im Hochvakuum entfernt. Der Rückstand (80 mg, 53% d. Th.) entsprach der Titelverbindung.
LC/MS [Methode 3]: Rₜ = 0.67 min; m/z = 263 (M+H)⁺.

### Beispiel 49A

### 2-[(5-Chlor-2-thienyl)carbonyl]-N-(2-methoxyethyl)hydrazincarboxamid

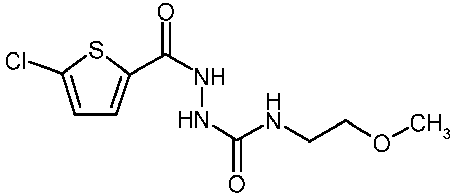

3.1 g (17.55 mmol) 5-Chlorthiophen-2-carbohydrazid wurden in 30 ml trockenem THF bei 50°C weitgehend suspendiert. Anschließend wurden 1.81 g (17.90 mmol) 1-Isocyanato-2-methoxyethan, in 30 ml THF gelöst, zugetropft. Es wurde 2.5 h bei 50°C gerührt. Nach dem Abkühlen auf RT wurde das Lösungsmittel am Rotationsverdampfer entfernt und der Rückstand mit Diethylether verrührt.

Die Kristalle wurden abgesaugt, mit Diethylether nachgewaschen und im Hochvakuum getrocknet. Es wurden 4.87 g (100% d. Th.) der Titelverbindung erhalten.
¹H-NMR (400 MHz, DMSO-d₆): δ = 3.14-3.21 (m, 2H), 3.28-3.36 (m, 5H), 6.52 (br. s, 1H), 7.22 (d, 1H), 7.70 (d, 1H), 7.97 (s, 1H), 10.24 (s, 1H).

### Beispiel 50A

### 5-(5-Chlor-2-thienyl)-4-(2-methoxyethyl)-2,4-dihydro-3H-1,2,4-triazol-3-on

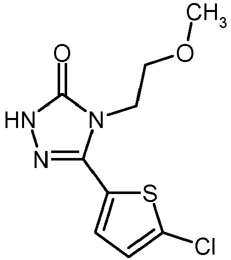

4.85 g (17.46 mmol) der Verbindung aus Beispiel 49A wurden in 17 ml (52.39 mmol) 3 M Natronlauge gelöst und 168 h unter Rückfluss erhitzt. Dabei wurden nach 16, 40, 64 und 88 h jeweils weitere 1.05 g (26.19 mmol, insgesamt 104.76 mmol) festes Natriumhydroxid zugegeben. Der Ansatz wurde danach mit 1 M Salzsäure auf pH 10 gestellt und das Gemisch zweimal mit je 30 ml Ethylacetat extrahiert. Die vereinigten organischen Phasen wurden über Natriumsulfat getrocknet, filtriert und am Rotationsverdampfer vom Lösungsmittel befreit. Der Rückstand wurde im Hochvakuum getrocknet. Es wurden 2.44 g (54% d. Th.) der Titelverbindung erhalten.
¹H-NMR (400 MHz, DMSO-d₆): δ = 3.20 (s, 3H), 3.53 (t, 2H), 3.92 (t, 2H), 7.24 (d, 1H), 7.51 (d, 1H), 12.04 (s, 1H).

### Beispiel 51A

### Ethyl-[3-(5-chlor-2-thienyl)-4-(2-methoxyethyl)-5-oxo-4,5-dihydro-1H-1,2,4-triazol-1-yl]acetat

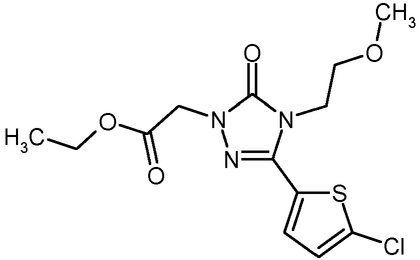

2.4 g (9.24 mmol) der Verbindung aus Beispiel 50A wurden mit 2.55 g (18.48 mmol) Kaliumcarbonat in 48 ml Acetonitril suspendiert. Anschließend wurde mit 1.08 ml (10.17 mmol) Chloressigsäureethylester versetzt und 4.5 h bei 80°C unter Rückfluss erhitzt. Danach wurden nochmals 113 mg (0.92 mmol) Chloressigsäureethylester zugegeben und weitere 2 h bei 80°C gerührt. Die Suspension wurde dann über eine Schicht Kieselgel filtriert und mit Ethylacetat nachgewaschen, das Filtrat am Rotationsverdampfer eingeengt und der Rückstand im Hochvakuum getrocknet. Es wurden 3.24 g (100% d. Th.) der Titelverbindung erhalten.
LC/MS [Methode 22]: Rₜ = 2.42 min; m/z = 346 (M+H)⁺
¹H-NMR (400 MHz, DMSO-d₆): δ = 1.21 (t, 3H), 3.30 (s, 3H), 3.55 (t, 2H), 3.99 (t, 2H), 4.15 (q, 2H), 4.65 (s, 2H), 7.27 (d, 1H), 7.58 (d, 1H).

### Beispiel 52A

### [3-(5-Chlor-2-thienyl)-4-(2-methoxyethyl)-5-oxo-4,5-dihydro-1H-1,2,4-triazol-1-yl]essigsäure

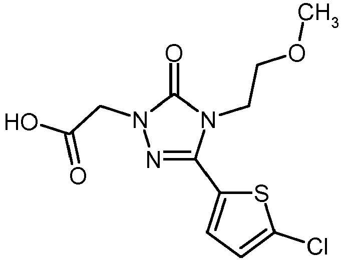

3.2 g (9.25 mmol) der Verbindung aus Beispiel 51A wurden in 28 ml Methanol gelöst. Anschließend wurden 2.82 ml einer 20%-igen wässrigen Kaliumhydroxid-Lösung zugegeben. Es wurde 2 h bei RT gerührt. Der Methanol-Anteil wurde dann am Rotationsverdampfer auf die Hälfte reduziert. Anschließend wurde mit Wasser verdünnt und einmal mit 15 ml Ethylacetat extrahiert. Die wässrige Phase wurde mit 920 µl konzentrierter Salzsäure angesäuert und zweimal mit je 15 ml Ethylacetat extrahiert. Die vereinigten organischen Phasen wurden über Natriumsulfat getrocknet, filtriert und am Rotationsverdampfer vom Lösungsmittel befreit. Nach Trocknen des Rückstands im Hochvakuum erhielt man 2.34 g (80% d. Th.) der Titelverbindung.
LC/MS [Methode 22]: Rₜ = 2.05 min; m/z = 318 (M+H)⁺
¹H-NMR (400 MHz, DMSO-d₆): δ = 3.20 (s, 3H), 3.55 (t, 2H), 3.99 (t, 2H), 4.53 (s, 2H), 7.27 (d, 1H), 7.58 (d, 1H), 13.14 (br. s, 1H).

### Beispiel 53A

### N-{2-Amino-2-[3-(trifluormethyl)phenyl]propyl}-2-{3-(4-chlorphenyl)-5-oxo-4-[(2S)-3,3,3-trifluor-2-hydroxypropyl]-4,5-dihydro-1H-1,2,4-triazol-1-yl}acetamid-Hydroformiat (Diastereomerengemisch)

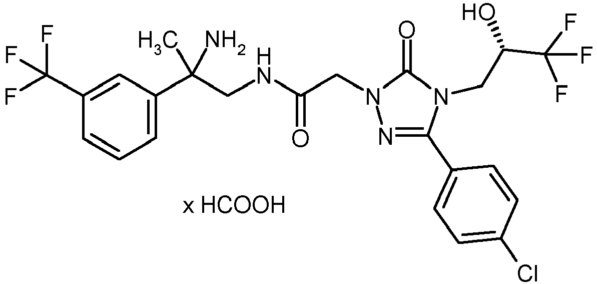

Eine Mischung von 166 mg (0.45 mmol) der Verbindung aus Beispiel 8A, 131 mg (0.68 mmol) EDC und 92 mg (0.68 mmol) HOBt in 4 ml DMF wurde zunächst 10 min bei RT verrührt und dann zu einer Lösung von 152 mg der Verbindung aus Beispiel 35A (Reinheit ca. 35%) und 158 µl (0.91 mmol) *N,N-*Diisopropylethylamin in 2 ml DMF eingetropft. Die Reaktionsmischung wurde 5 min bei RT gerührt, dann mit 2 ml 1 N Salzsäure versetzt und direkt durch präparative HPLC [Methode 9] aufgetrennt. Es wurden 84 mg (30% d. Th.) der reinen Titelverbindung erhalten.
LC/MS [Methode 4]: Rₜ = 0.88 min; m/z = 566 (M+H)⁺
¹H-NMR (400 MHz, DMSO-d₆): δ [ppm] = 1.39 (s, 3H), 3.35-3.50 (m, 2H), 3.82 (dd, 1H), 3.92-4.00 (m, 1H), 4.28 (br. s, 1H), 4.35-4.49 (m, 2H), 7.51-7.67 (m, 4H), 7.74 (d, 2H), 7.79 (d, 1H), 7.90 (s, 1H), 8.08 (t, 1H), 8.18 (s, 1H).

### Beispiel 54A

### N-[2-Amino-2-(2,3-dichlorphenyl)ethyl]-2-{3-(4-chlorphenyl)-5-oxo-4-[(2S)-3,3,3-trifluor-2-hydroxypropyl]-4,5-dihydro-1H-1,2,4-triazol-1-yl}acetamid-Hydrochlorid (Diastereomer 1)

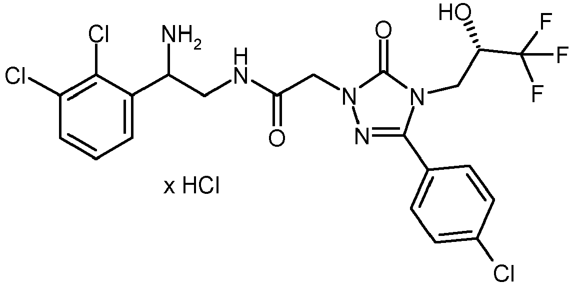

Eine Lösung von 147 mg (0.23 mmol) der Verbindung aus Beispiel 19 in 5 ml Dichlormethan wurde mit 5 ml einer 4 N Lösung von Chlorwasserstoff in Dioxan versetzt und 2 h bei RT gerührt. Anschließend wurden die flüchtigen Bestandteile am Rotationsverdampfer entfernt. Der Rückstand wurde erneut mit 5 ml Dichlormethan versetzt, wiederum am Rotationsverdampfer vom Lösungsmittel befreit und dann über präparative HPLC [Methode 8] gereinigt. Die produkthaltige Fraktion wurde mit 10 ml 1 M Salzsäure versetzt und dann am Rotationsverdampfer von allen flüchtigen Bestandteilen befreit. Der Rückstand wurde im Hochvakuum getrocknet. Man erhielt 127 mg (96% d. Th.) der Titelverbindung.
LC/MS [Methode 4]: Rₜ = 0.85 min; m/z = 552 (M+H)⁺
¹H-NMR (400 MHz, DMSO-d₆): δ [ppm] = 3.47-3.59 (m, 1H), 3.60-3.71 (m, 1H), 3.83 (dd, 1H), 3.97 (dd, 1H), 4.22-4.35 (m, 1H), 4.37-4.50 (m [AB], 2H), 4.80-4.91 (m, 1H), 6.92 (br. d, 1H), 7.51 (t, 1H), 7.64 (d, 2H), 7.67-7.80 (m, 4H), 8.45 (t, 1H), 8.67 (br. s, 3H).

### Beispiel 55A

### N-[2-Amino-2-(2,3-dichlorphenyl)ethyl]-2-{3-(4-chlorphenyl)-5-oxo-4-[(2S)-3,3,3-trifluor-2-hydroxypropyl]-4,5-dihydro-1H-1,2,4-triazol-1-yl}acetamid-Hydrochlorid (Diastereomer 2)

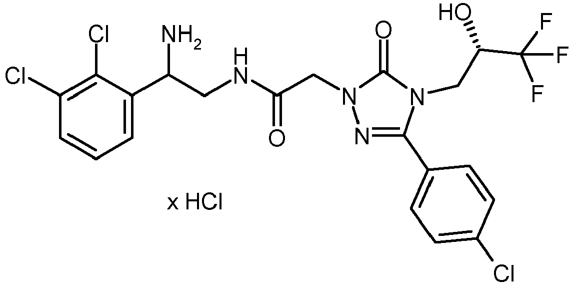

Analog zu Beispiel 54A wurden aus 147 mg (0.23 mmol) der Verbindung aus Beispiel 20 115 mg (87% d. Th.) der Titelverbindung erhalten.
¹H-NMR (400 MHz, DMSO-d₆): δ [ppm] = 3.51 (dt, 1H), 3.68 (dt, 1H), 3.83 (dd, 1H), 3.96 (dd, 1H), 4.22-4.34 (m, 1H), 4.43 (s, 2H), 4.81-4.91 (m, 1H), 6.93 (br. d, 1H), 7.51 (t, 1H), 7.64 (d, 2H), 7.70-7.76 (m, 4H), 8.44 (t, 1H), 8.69 (br. s, 3H).

### Beispiel 56A

### N-{2-Amino-2-[2-(trifluormethyl)phenyl]ethyl}-2-{3-(4-chlorphenyl)-5-oxo-4-[(2S)-3,3,3-trifluor-2-hydroxypropyl]-4,5-dihydro-1H-1,2,4-triazol-1-yl}acetamid-Hydrochlorid (Diastereomer 1)

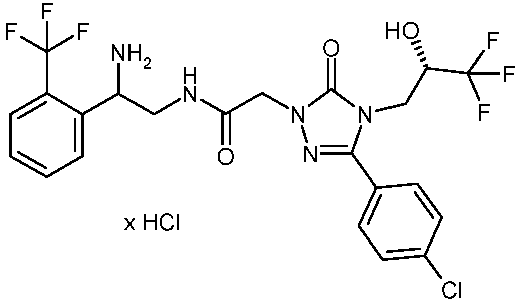

Eine Lösung von 145 mg (0.22 mmol) der Verbindung aus Beispiel 14 in 5 ml Dichlormethan wurde mit 5 ml einer 4 N Lösung von Chlorwasserstoff in Dioxan versetzt und 2 h bei RT gerührt. Anschließend wurden die flüchtigen Bestandteile am Rotationsverdampfer entfernt. Der Rückstand wurde erneut mit 5 ml Dichlormethan versetzt, wiederum am Rotationsverdampfer vom Lösungsmittel befreit und dann im Hochvakuum getrocknet. Man erhielt 130 mg (91% d. Th.) der Titelverbindung (noch etwa 7% Dioxan laut ¹H-NMR enthaltend).
LC/MS [Methode 3]: Rₜ = 0.97 min; m/z = 552 (M+H)⁺
¹H-NMR (400 MHz, DMSO-d₆): δ [ppm] = 3.44-3.55 (m, 1H), 3.64-3.75 (m, 1H), 3.84 (dd, 1H), 3.97 (dd, 1H), 4.21-4.35 (m, 1H), 4.39-4.52 (m [AB], 2H), 4.54-4.67 (m, 1H), 6.91 (d, 1H), 7.61-7.68 (m, 3H), 7.72-7.78 (m, 2H), 7.79-7.88 (m, 2H), 8.00 (d, 1H), 8.50 (t, 1H), 8.76 (br. s, 3H).

### Beispiel 57A

### N-{2-Amino-2-[2-(trifluormethyl)phenyl]ethyl}-2-{3-(4-chlorphenyl)-5-oxo-4-[(2S)-3,3,3-trifluor-2-hydroxypropyl]-4,5-dihydro-1H-1,2,4-triazol-1-yl}acetamid-Hydrochlorid (Diastereomer 2)

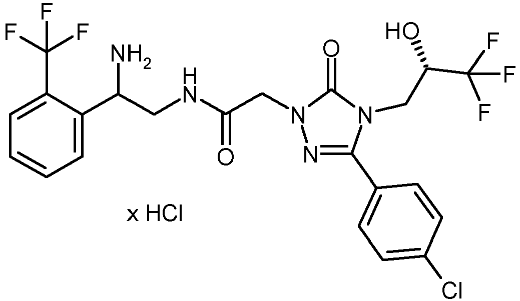

Analog zu Beispiel 56A wurden aus 117 mg (0.18 mmol) der Verbindung aus Beispiel 15 104 mg (93% d. Th.) der Titelverbindung erhalten (noch etwa 5% Dioxan laut ¹H-NMR enthaltend).
LC/MS [Methode 3]: Rₜ = 0.97 min; m/z = 552 (M+H)⁺
¹H-NMR (400 MHz, DMSO-d₆): δ [ppm] = 3.44-3.54 (m, 1H), 3.64-3.74 (m, 1H), 3.83 (dd, 1H), 3.96 (dd, 1H), 4.22-4.34 (m, 1H), 4.39-4.50 (m [AB], 2H), 4.54-4.67 (m, 1H), 6.93 (d, 1H), 7.61-7.69 (m, 3H), 7.74 (d, 2H), 7.78-7.90 (m, 2H), 8.01 (d, 1H), 8.50 (t, 1H), 8.79 (br. s, 3H).

### Beispiel 58A

### N-{2-Amino-2-[3-(trifluormethyl)phenyl]ethyl}-2-{3-(4-chlorphenyl)-5-oxo-4-[(2S)-3,3,3-trifluor-2-hydroxypropyl]-4,5-dihydro-1H-1,2,4-triazol-1-yl}acetamid-Hydrochlorid (Diastereomerengemisch)

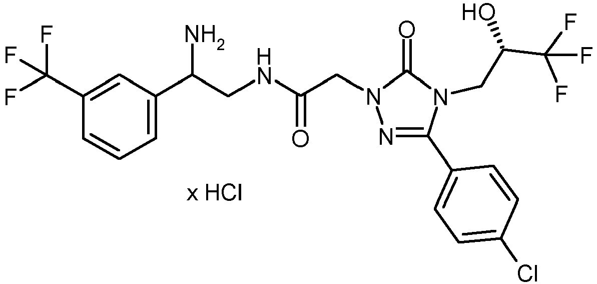

Analog zu Beispiel 56A wurden aus 235 mg (0.36 mmol) der Verbindung aus Beispiel 34 220 mg (99% d. Th.) der Titelverbindung erhalten (noch etwa 5% Dioxan laut ¹H-NMR enthaltend).
LC/MS [Methode 4]: Rₜ = 0.87 min; m/z = 552 (M+H)⁺
¹H-NMR (400 MHz, DMSO-d₆): δ [ppm] = 3.50-3.61 (m, 1H), 3.62-3.73 (m, 1H), 3.83 (dd, 1H), 3.97 (br. d, 1H), 4.22-4.35 (m, 1H), 4.36-4.49 (m, 2H), 4.54 (br. t, 1H), 6.93 (dd, 1H), 7.61-7.71 (m, 3H), 7.72-7.82 (m, 4H), 7.93 (s, 1H), 8.42 (br. t, 1H), 8.60 (br. s, 3H).

### Beispiel 59A

### N-{2-Amino-2-[2-(trifluormethyl)phenyl]ethyl}-2-[3-(4-chlorphenyl)-4-cyclopropyl-5-oxo-4,5-dihydro-1H-1,2,4-triazol-1-yl]acetamid-Hydrochlorid (Racemat)

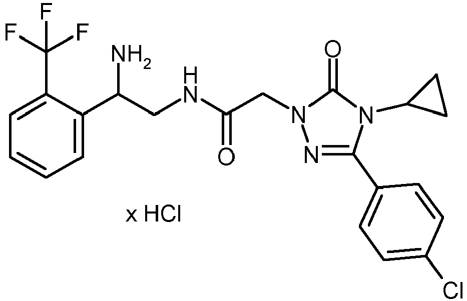

Analog zu Beispiel 56A wurden aus 44 mg (62 µmol) der Verbindung aus Beispiel 65 40 mg (99% d. Th.) der Titelverbindung erhalten (noch etwa 22% Dioxan laut ¹H-NMR enthaltend).
LC/MS [Methode 4]: Rₜ = 0.72 min; m/z = 480 (M+H)⁺
¹H-NMR (400 MHz, DMSO-d₆): δ [ppm] = 0.51-0.62 (m, 2H), 0.86-0.95 (m, 2H), 3.18 (tt, 1H), 3.42-3.53 (m, 1H), 3.62-3.74 (m, 1H), 4.39 (s, 2H), 4.55-4.62 (m, 1H), 7.57-7.69 (m, 3H), 7.75-7.89 (m, 4H), 7.98 (d, 1H), 8.44 (t, 1H), 8.72 (br. s, 3H).

### Beispiel 60A

### N-{2-Amino-2-[2-(trifluormethyl)phenyl]ethyl}-2-[3-(5-chlor-2-thienyl)-4-(2-methoxyethyl)-5-oxo-4,5-dihydro-1H-1,2,4-triazol-1-yl]acetamid-Hydrochlorid (Racemat)

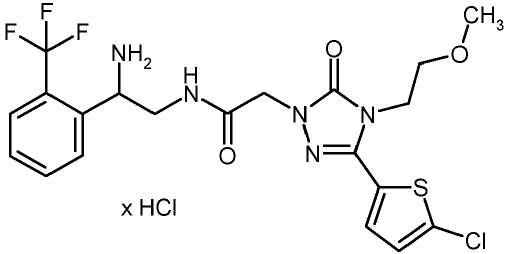

Analog zu Beispiel 56A wurden aus 44 mg (60 µmol) der Verbindung aus Beispiel 66 40 mg (99% d. Th.) der Titelverbindung erhalten (noch etwa 20% Dioxan laut ¹H-NMR enthaltend).
LC/MS [Methode 4]: Rₜ = 0.72 min; m/z = 504 (M+H)⁺
¹H-NMR (400 MHz, DMSO-d₆): δ [ppm] = 3.21 (s, 3H), 3.44-3.52 (m, 1H), 3.52-3.59 (m, 2H), 3.64-3.74 (m, 1H), 3.98 (t, 2H), 4.43 (s, 2H), 4.56-4.62 (m, 1H), 7.28 (d, 1H), 7.57 (d, 1H), 7.62-7.69 (m, 1H), 7.81-7.88 (m, 2H), 8.00 (d, 1H), 8.50 (t, 1H), 8.76 (br. s, 3H).

### Beispiel 61A

### N-{2-Amino-2-[2-(trifluormethyl)phenyl]ethyl}-2-[3-(5-chlor-2-thienyl)-4-(2-fluorbenzyl)-5-oxo-4,5-dihydro-1H-1,2,4-triazol-1-yl]acetamid-Hydrochlorid (Racemat)

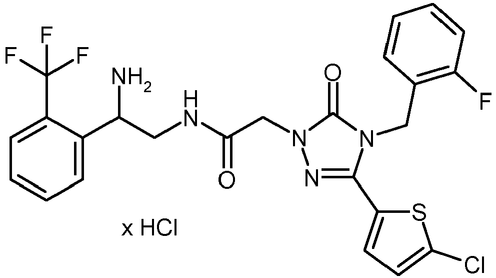

Analog zu Beispiel 56A wurden aus 49 mg (60 µmol) der Verbindung aus Beispiel 67 47 mg (99% d. Th.) der Titelverbindung erhalten (noch etwa 30% Dioxan laut ¹H-NMR enthaltend).
LC/MS [Methode 4]: Rₜ = 0.83 min; m/z = 554 (M+H)⁺
¹H-NMR (400 MHz, DMSO-d₆): δ [ppm] = 3.44-3.54 (m, 1H), 3.64-3.76 (m, 1H), 4.50 (s, 2H), 4.55-4.64 (m, 1H), 5.15 (s, 2H), 7.07 (t, 1H), 7.12-7.28 (m, 4H), 7.37 (q, 1H), 7.66 (t, 1H), 7.80-7.89 (m, 2H), 8.00 (d, 1H), 8.55 (t, 1H), 8.75 (br. s, 3H).

### Ausführungsbeispiele:

### Beispiel 1

### 3-[({3-(4-Chlorphenyl)-5-oxo-4-[(2S)-3,3,3-trifluor-2-hydrolypropyl]-4,5-dihydro-1H-1,2,4-triazol-1-yl}acetyl)amino]-2-[2-(trifluormethyl)phenyl]propylcarbamat (Diastereomerengemisch)

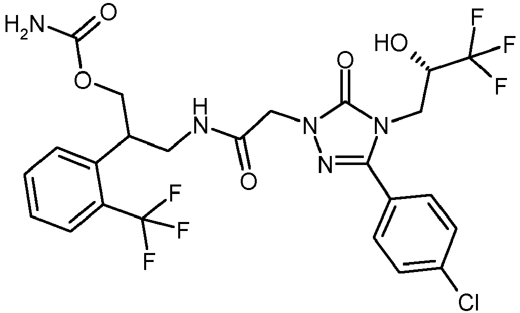

Eine Mischung aus 366 mg (1.00 mmol) der Verbindung aus Beispiel 8A, 299 mg (1.00 mmol) der Verbindung aus Beispiel 13A, 288 mg (1.50 mmol) EDC, 203 mg (1.50 mmol) HOBt und 348 µl (2.0 mmol) *N,N*-Diisopropylethylamin in 25 ml DMF wurde über Nacht bei RT gerührt. Danach wurde das Gemisch mit 1 ml 1 M Salzsäure versetzt und direkt über präparative HPLC [Methode 8] in seine Komponenten aufgetrennt. Die Produktfraktion wurde am Rotationsverdampfer vom Lösungsmittel befreit und der Rückstand im Hochvakuum getrocknet. Man erhielt 340 mg (56% d. Th.) der Titelverbindung als Diastereomerengemisch.
LC/MS [Methode 4]: Rₜ = 1.04 min; MS [ESIpos]: m/z = 610 (M+H)⁺.

Durch präparative HPLC an chiraler Phase [Methode 10a] konnten die beiden Diastereomere getrennt werden, siehe Beispiel 2 und Beispiel 3.

### Beispiel 2

### 3-[({3-(4-Chlorphenyl)-5-oxo-4-[(2S)-3,3,3-trifluor-2-hydrolypropyl]-4,5-dihydro-1H-1,2,4-triazol-1-yl}acetyl)amino]-2-[2-(trifluormethyl)phenyl]propylcarbamat (Diastereomer 1)

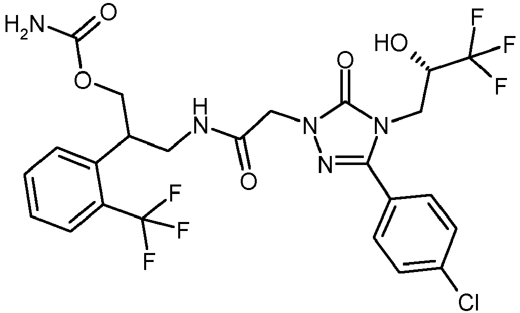

Zuerst eluierendes Diastereomer aus der chromatographischen Trennung von 340 mg der Verbindung aus Beispiel 1 nach Methode 10a. Das so erhaltene Material (170 mg) wurde noch einmal durch präparative HPLC [Methode 8] feingereinigt. Man erhielt 160 mg der sauberen Titelverbindung.
Chirale analytische HPLC [Methode 11]: Rₜ = 2.30 min.
LC/MS [Methode 4]: Rₜ = 1.04 min; MS [ESIpos]: m/z = 610 (M+H)⁺
¹H-NMR (400 MHz, DMSO-d₆): δ [ppm] = ca. 3.26-3.35 (m, 1H, teilweise durch Wasser-Signal verdeckt), 3.43-3.54 (m, 1H), 3.54-3.64 (m, 1H), 3.82 (dd, 1H), 3.95 (dd, 1H), 4.10 (dd, 1H), 4.21-4.33 (m, 2H), 4.35 (s, 2H), 6.32-6.53 (br. s, 2H), 6.92 (d, 1H), 7.45 (t, 1H), 7.57-7.80 (m, 7H), 8.17 (t, 2H).

### Beispiel 3

### 3-[({3-(4-Chlorphenyl)-5-oxo-4-[(2S)-3,3,3-trifluor-2-hydrolypropyl]-4,5-dihydro-1H-1,2,4-triazol-1-yl}acetyl)amino]-2-[2-(trifluormethyl)phenyl]propylcarbamat (Diastereomer 2)

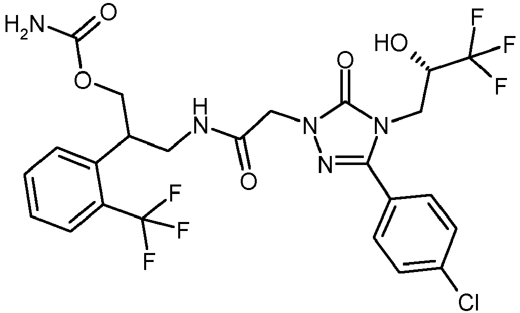

Zuletzt eluierendes Diastereomer aus der chromatographischen Trennung von 340 mg der Verbindung aus Beispiel 1 nach Methode 10a. Das so erhaltene Material (185 mg) wurde noch einmal durch präparative HPLC [Methode 8] feingereinigt. Man erhielt 160 mg der sauberen Titelverbindung.
Chirale analytische HPLC [Methode 11]: Rₜ = 2.98 min.
LC/MS [Methode 2]: Rₜ = 2.16 min; MS [ESIpos]: m/z = 610 (M+H)⁺
¹H-NMR (400 MHz, DMSO-d₆): δ [ppm] = ca. 3.29-3.35 (m, 1H, teilweise durch Wasser-Signal verdeckt), 3.44-3.62 (m, 2H), 3.82 (dd, 1H), 3.95 (dd, 1H), 4.10 (dd, 1H), 4.24 (dd, 1H), 4.23-4.36 (m, 1H), 4.29-4.42 (m [AB], 2H), 6.33-6.53 (br. s, 2H), 6.93 (d, 1H), 7.45 (t, 1H), 7.60-7.78 (m, 7H), 8.19 (t, 1H).

### Beispiel 4

### 2-(2-Chlorphenyl)-3-[({3-(4-chlorphenyl)-5-oxo-4-[(2S)-3,3,3-trifluor-2-hydrolypropyl]-4,5-dihydro-1H-1,2,4-triazol-1-yl}acetyl)amino]propylcarbamat (Diastereomerengemisch)

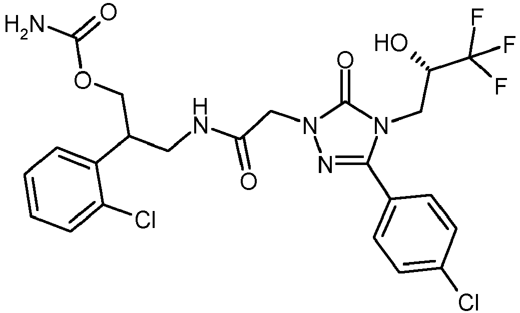

185 mg (0.51 mmol) der Verbindung aus Beispiel 8A, 146 mg (0.76 mmol) EDC und 108 mg (0.76 mmol) HOBt wurden in 5 ml DMF 20 min bei RT gerührt. Anschließend wurde die resultierende Lösung zu einer Lösung von 116 mg (0.51 mmol) der Verbindung aus Beispiel 15A in 15 ml Acetonitril zugetropft. Nach 30 min bei RT wurde das Acetonitril am Rotationsverdampfer entfernt. Die restliche Lösung wurde mit 1 ml 1 M Salzsäure versetzt und direkt durch präparative HPLC [Methode 8] in ihre Komponenten aufgetrennt. Die Produktfraktion wurde am Rotationsverdampfer vom Lösungsmittel befreit und der Rückstand im Hochvakuum getrocknet. Man erhielt 150 mg (49% d. Th.) der Titelverbindung als Diastereomerengemisch.
LC/MS [Methode 4]: Rₜ = 0.99 min; MS [ESIpos]: m/z = 576 (M+H)⁺.

Durch präparative HPLC an chiraler Phase [Methode 5b] konnten die beiden Diastereomere getrennt werden, siehe Beispiel 5 und Beispiel 6.

### Beispiel 5

### 2-(2-Chlorphenyl)-3-[({3-(4-chlorphenyl)-5-oxo-4-[(2S)-3,3,3-trifluor-2-hydrolypropyl]-4,5-dihydro-1H-1,2,4-triazol-1-yl}acetyl)amino]propylcarbamat (Diastereomer 1)

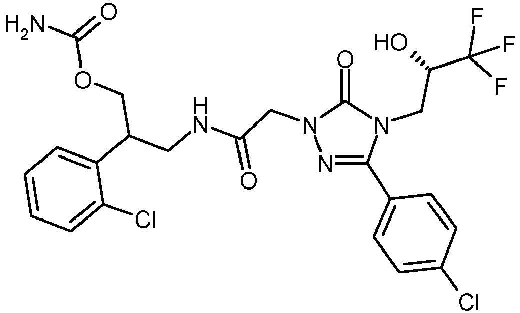

Zuerst eluierendes Diastereomer aus der chromatographischen Trennung von 150 mg der Verbindung aus Beispiel 4 nach Methode 5b. Das so erhaltene Material (58 mg) wurde noch einmal durch präparative HPLC [Methode 8] feingereinigt. Man erhielt 46 mg der sauberen Titelverbindung.
Chirale analytische HPLC [Methode 6b]: Rₜ = 2.51 min.
LC/MS [Methode 4]: Rₜ = 0.99 min; MS [ESIpos]: m/z = 576 (M+H)⁺
¹H-NMR (400 MHz, DMSO-d₆): δ [ppm] = 3.33-3.39 (m, 1H), 3.49 (dt, 1H), 3.66 (quin, 1H), 3.82 (dd, 1H), 3.96 (dd, 1H), 4.12-4.22 (m, 2H), 4.25-4.34 (m, 1H), 4.32-4.42 (m, 2H), 6.30-6.62 (br. s, 2H), 6.93 (d, 1H), 7.26 (td, 1H), 7.31 (td, 1H), 7.40-7.46 (m, 2H), 7.63 (d, 2H), 7.75 (d, 2H), 8.18 (t, 1H).

### Beispiel 6

### 2-(2-Chlorphenyl)-3-[({3-(4-chlorphenyl)-5-oxo-4-[(2S)-3,3,3-trifluor-2-hydrolypropyl]-4,5-dihydro-1H-1,2,4-triazol-1-yl}acetyl)amino]propylcarbamat (Diastereomer 2)

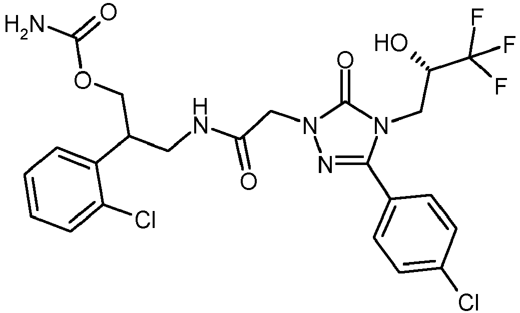

Zuletzt eluierendes Diastereomer aus der chromatographischen Trennung von 150 mg der Verbindung aus Beispiel 4 nach Methode 5b. Das so erhaltene Material (63 mg) wurde noch einmal durch präparative HPLC [Methode 8] feingereinigt. Man erhielt 59 mg der sauberen Titelverbindung.
Chirale analytische HPLC [Methode 6b]: Rₜ = 2.92 min.
LC/MS [Methode 4]: Rₜ = 1.00 min; MS [ESIpos]: m/z = 576 (M+H)⁺
¹H-NMR (400 MHz, DMSO-d₆): δ [ppm] = 3.32-3.40 (m, 1H), 3.47 (dt, 1H), 3.65 (quin, 1H), 3.82 (dd, 1H), 3.96 (dd, 1H), 4.12-4.22 (m, 2H), 4.24-4.35 (m, 1H), 4.31-4.43 (m [AB], 2H), 6.25-6.65 (br. s, 2H), 6.93 (d, 1H), 7.26 (dt, 1H), 7.31 (dt, 1H), 7.39-7.46 (m, 2H), 7.63 (d, 2H), 7.76 (d, 2H), 8.20 (t, 1H).

### Beispiel 7

### 3-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propylcarbamat (Diastereomerengemisch)

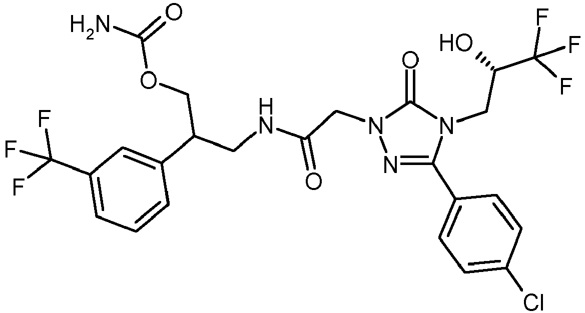

Eine Mischung aus 236 mg (0.64 mmol) der Verbindung aus Beispiel 8A, 385 mg (0.77 mmol) der Verbindung aus Beispiel 18A, 148 mg (0.77 mmol) EDC, 110 mg (0.77 mmol) HOBt und 225 µl (1.29 mmol) *N,N-*Diisopropylethylamin in 10 ml DMF wurde über Nacht bei RT gerührt. Danach wurde das Gemisch mit 2 ml 1 M Salzsäure versetzt und direkt über präparative HPLC [Methode 8] in seine Komponenten aufgetrennt. Das erhaltene Produkt wurde anschließend durch nochmalige präparative HPLC [Methode 9] nachgereinigt. Die Produktfraktion wurde am Rotationsverdampfer vom Lösungsmittel befreit und der Rückstand im Hochvakuum getrocknet. Man erhielt 140 mg (35% d. Th.) der Titelverbindung als Diastereomerengemisch.
LC/MS [Methode 4]: Rₜ = 1.04 min; MS [ESIpos]: m/z = 610 (M+H)⁺.

Durch präparative HPLC an chiraler Phase [Methode 5a] konnten die beiden Diastereomere getrennt werden, siehe Beispiel 8 und Beispiel 9.

### Beispiel 8

### 3-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propylcarbamat (Diastereomer 1)

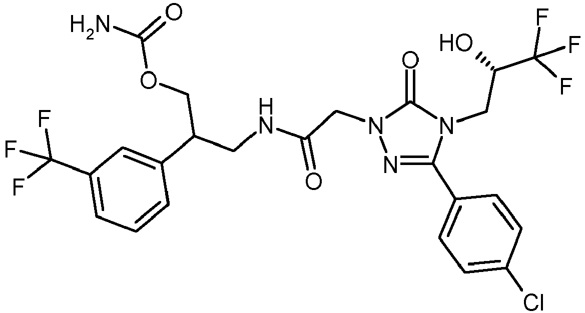

Zuerst eluierendes Diastereomer aus der chromatographischen Trennung von 140 mg der Verbindung aus Beispiel 7 nach Methode 5a. Das so erhaltene Material (82 mg) wurde noch einmal durch präparative HPLC [Methode 8] feingereinigt. Man erhielt 69 mg der sauberen Titelverbindung.
Chirale analytische HPLC [Methode 6a]: Rₜ = 3.54 min.
LC/MS [Methode 4]: Rₜ = 1.04 min; MS [ESIpos]: m/z = 610 (M+H)⁺
¹H-NMR (400 MHz, DMSO-d₆): δ [ppm] = 3.20-3.29 (m, 1H), 3.33-3.42 (m, 1H), 3.42-3.52 (m, 1H), 3.82 (dd, 1H), 3.95 (dd, 1H), 4.17 (d, 2H), 4.23-4.41 (m, 3H), 6.25-6.70 (br. s, 2H), 6.92 (d, 1H), 7.50-7.66 (m, 6H), 7.71-7.77 (m, 2H), 8.16 (t, 1H).

### Beispiel 9

### 3-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propylcarbamat (Diastereomer 2)

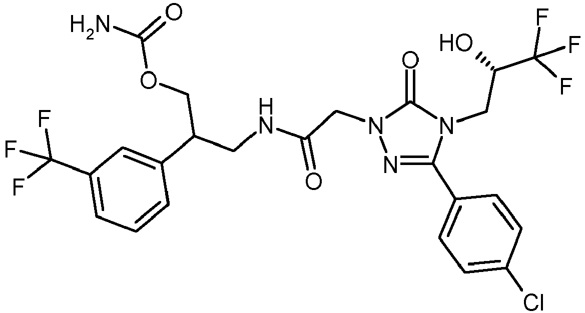

Zuletzt eluierendes Diastereomer aus der chromatographischen Trennung von 140 mg der Verbindung aus Beispiel 7 nach Methode 5a. Das so erhaltene Produkt (83 mg) wurde noch einmal durch präparative HPLC [Methode 8] feingereinigt. Man erhielt 67 mg der sauberen Titelverbindung.
Chirale analytische HPLC [Methode 6a]: Rₜ = 4.29 min.
LC/MS [Methode 4]: Rₜ = 1.04 min; MS [ESIpos]: m/z = 610 (M+H)⁺
¹H-NMR (400 MHz, DMSO-d₆): δ [ppm] = 3.19-3.29 (m, 1H), 3.35-3.50 (m, 2H), 3.82 (dd, 1H), 3.95 (dd, 1H), 4.18 (br. d, 2H), 4.23-4.42 (m, 3H), 6.30-6.65 (br. s, 2H), 6.92 (d, 1H), 7.50-7.67 (m, 6H), 7.71-7.78 (m, 2H), 8.17 (t, 1H).

### Beispiel 10

### 3-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propyl-ethylcarbamat (Diastereomerengemisch)

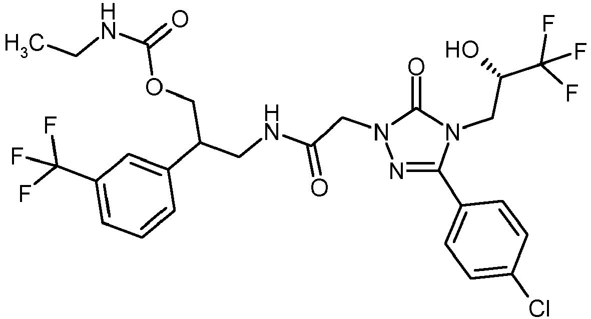

95 mg (0.26 mmol) der Verbindung aus Beispiel 8A, 75 mg (0.39 mmol) EDC und 56 mg (0.39 mmol) HOBt wurden in 2.5 ml DMF 5 min bei RT gerührt. Anschließend wurde die resultierende Lösung zu einer Lösung von 102 mg (0.26 mmol) der Verbindung aus Beispiel 20A in 7.5 ml Acetonitril zugetropft. Nach 30 min bei RT wurde das Acetonitril am Rotationsverdampfer entfernt. Die restliche Lösung wurde mit 1 ml 1 M Salzsäure versetzt und direkt durch präparative HPLC [Methode 8] in ihre Komponenten aufgetrennt. Die Produktfraktion wurde am Rotationsverdampfer vom Lösungsmittel befreit und der Rückstand im Hochvakuum getrocknet. Man erhielt 123 mg (73% d. Th.) der Titelverbindung als Diastereomerengemisch.
LC/MS [Methode 4]: Rₜ = 1.13 min; MS [ESIpos]: m/z = 638 (M+H)⁺.

Durch präparative HPLC an chiraler Phase [Methode 10b] konnten die beiden Diastereomere getrennt werden, siehe Beispiel 11 und Beispiel 12.

### Beispiel 11

### 3-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propyl-ethylcarbamat (Diastereomer 1)

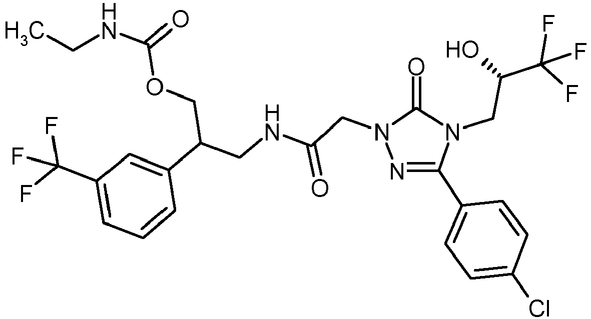

Zuerst eluierendes Diastereomer aus der chromatographischen Trennung von 120 mg der Verbindung aus Beispiel 10 nach Methode 10b. Das so erhaltene Material (62 mg) wurde noch einmal durch präparative HPLC [Methode 8] feingereinigt. Man erhielt 50 mg der sauberen Titelverbindung.
Chirale analytische HPLC [Methode 11]: Rₜ = 1.77 min.
LC/MS [Methode 3]: Rₜ = 1.31 min; MS [ESIpos]: m/z = 638 (M+H)⁺
¹H-NMR (400 MHz, DMSO-d₆): δ [ppm] = 0.95 (t, 3H), 2.94 (quin, 2H), 3.19-3.29 (m, 1H), 3.34-3.52 (m, 2H), 3.82 (dd, 1H), 3.95 (dd, 1H), 4.14-4.42 (m, 5H), 6.92 (d, 1H), 7.07 (t, 1H), 7.50-7.67 (m, 6H), 7.74 (d, 2H), 8.17 (t, 1H).

### Beispiel 12

### 3-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-2-[3-(trifluormethyl)phenyl]propyl-ethylcarbamat (Diastereomer 2)

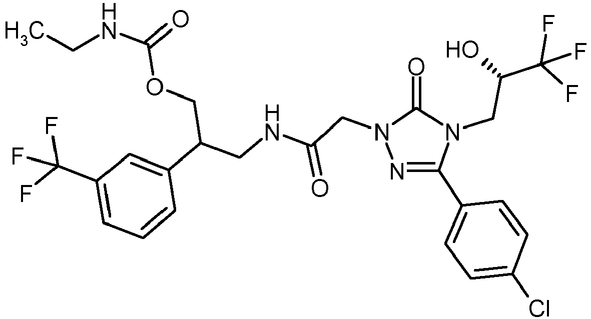

Zuletzt eluierendes Diastereomer aus der chromatographischen Trennung von 120 mg der Verbindung aus Beispiel 10 nach Methode 10b. Das so erhaltene Material (ca. 60 mg) wurde noch einmal durch präparative HPLC [Methode 8] feingereinigt. Man erhielt 52 mg der sauberen Titelverbindung.
Chirale analytische HPLC [Methode 11]: Rₜ = 2.28 min.
LC/MS [Methode 3]: Rₜ = 1.31 min; MS [ESIpos]: m/z = 638 (M+H)⁺
¹H-NMR (400 MHz, DMSO-d₆; Signale des Hauptrotamers): δ [ppm] = 0.95 (t, 3H), 2.89-2.99 (m, 2H), 3.19-3.29 (m, 1H), 3.35-3.53 (m, 2H), 3.83 (dd, 1H), 3.95 (dd, 1H), 4.15-4.42 (m, 5H), 6.92 (d, 1H), 7.07 (t, 1H), 7.49-7.67 (m, 6H), 7.75 (d, 2H), 8.18 (t, 1H).

### Beispiel 13

### tert.-Butyl-{2-[({3-(4-chlorphenyl)-5-oxo-4-[(2S)-3,3,3-trifluor-2-hydroxypropyl]-4,5-dihydro-1H-1,2,4-triazol-1-yl}acetyl)amino]-1-[2-(trifluormethyl)phenyl]ethyl}carbamat (Diastereomerengemisch)

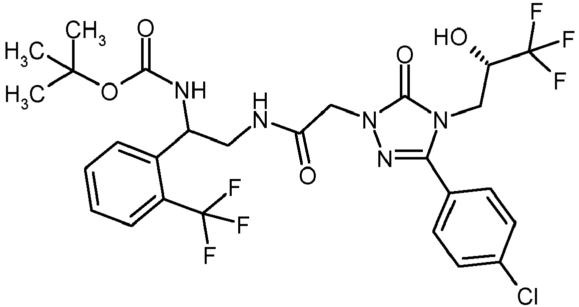

Eine Mischung aus 295 mg (0.81 mmol) der Verbindung aus Beispiel 8A, 270 mg (0.89 mmol) *tert.-*Butyl-{2-amino-1-[2-(trifluormethyl)phenyl]ethyl}carbamat, 216 mg (1.13 mmol) EDC und 153 mg (1.13 mmol) HOBt in 7 ml DMF wurde 1 h bei RT gerührt. Anschließend wurde das Gemisch mit 1 ml 1 M Salzsäure versetzt und direkt über präparative HPLC [Methode 8] in seine Komponenten aufgetrennt. Die Produktfraktion wurde am Rotationsverdampfer vom Lösungsmittel befreit und der Rückstand im Hochvakuum getrocknet. Man erhielt 494 mg (94% d. Th.) der Titelverbindung als Diastereomerengemisch.
LC/MS [Methode 4]: Rₜ = 1.22 min; MS [ESIpos]: m/z = 652 (M+H)⁺.

Durch präparative HPLC an chiraler Phase [Methode 13a] konnten die beiden Diastereomere getrennt werden, siehe Beispiel 14 und Beispiel 15.

### Beispiel 14

### tert.-Butyl-{2-[({3-(4-chlorphenyl)-5-oxo-4-[(2S)-3,3,3-trifluor-2-hydroxypropyl]-4,5-dihydro-1H-1,2,4-triazol-1-yl}acetyl)amino]-1-[2-(trifluormethyl)phenyl]ethyl}carbamat (Diastereomer 1)

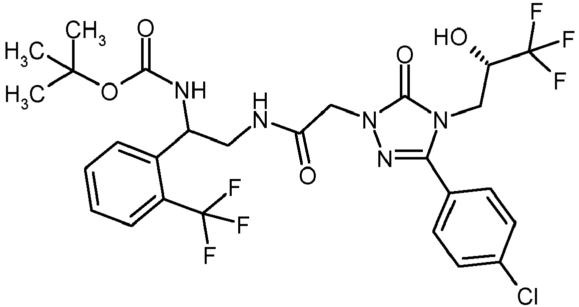

Zuerst eluierendes Diastereomer (145 mg) aus der chromatographischen Trennung von 490 mg der Verbindung aus Beispiel 13 nach Methode 13a.
Chirale analytische HPLC [Methode 14]: Rₜ = 5.25 min.
LC/MS [Methode 4]: Rₜ = 1.22 min; MS [ESIpos]: m/z = 652 (M+H)⁺
¹H-NMR (400 MHz, DMSO-d₆; Signale des Hauptrotamers): δ [ppm] = 1.33 (s, 9H), 3.21-3.41 (m, 2H), 3.83 (dd, 1H), 3.97 (dd, 1H), 4.22-4.33 (m, 1H), 4.35-4.48 (m, 2H), 4.96-5.08 (m, 1H), 6.92 (d, 1H), 7.42-7.53 (m, 2H), 7.60-7.70 (m, 4H), 7.71-7.80 (m, 3H), 8.26 (br. t, 1H).

### Beispiel 15

### tert.-Butyl-{2-[({3-(4-chlorphenyl)-5-oxo-4-[(2S)-3,3,3-trifluor-2-hydroxypropyl]-4,5-dihydro-1H-1,2,4-triazol-1-yl}acetyl)amino]-1-[2-(trifluormethyl)phenyl]ethyl}carbamat (Diastereomer 2)

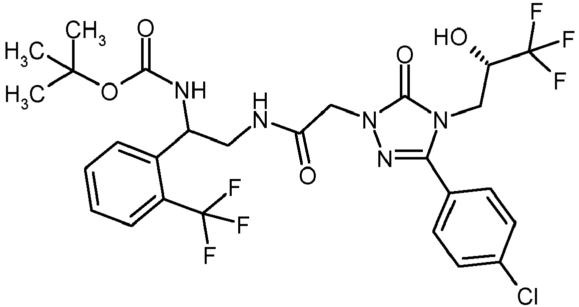

Zuletzt eluierendes Diastereomer (117 mg) aus der chromatographischen Trennung von 490 mg der Verbindung aus Beispiel 13 nach Methode 13a.
Chirale analytische HPLC [Methode 14]: Rₜ = 5.94 min.
LC/MS [Methode 4]: Rₜ = 1.22 min; MS [ESIpos]: m/z = 652 (M+H)⁺
¹H-NMR (400 MHz, DMSO-d₆; Signale des Hauptrotamers): δ [ppm] = 1.33 (br. s, 9H), 3.20-3.41 (m, 2H), 3.84 (dd, 1H), 3.96 (dd, 1H), 4.23-4.34 (m, 1H), 4.41 (br. s, 2H), 4.97-5.07 (m, 1H), 6.93 (d, 1H), 7.43-7.52 (m, 2H), 7.61-7.70 (m, 4H), 7.71-7.79 (m, 3H), 8.21-8.30 (m, 1H).

### Beispiel 16

### 2-{3-(4-Chlorphenyl)-5-oxo-4-[(2S)-3,3,3-trifluor-2-hydroxypropyl]-4,5-dihydro-1H-1,2,4-triazol-1-yl}-N-{2-[(methylsulfonyl)amino]-2-[2-(trifluormethyl)phenyl]ethyl}acetamid (Diastereomer 1)

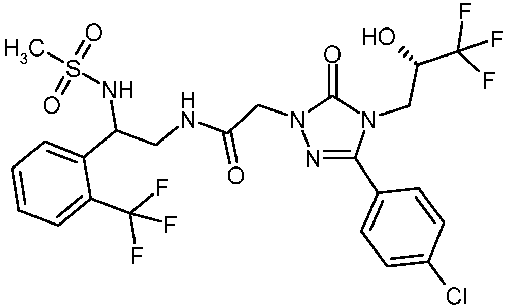

Eine Lösung von 90 mg (0.15 mmol) der Verbindung aus Beispiel 56A in 1.5 ml Pyridin wurde bei RT mit 13 µl Methansulfonsäurechlorid versetzt. Die Mischung wurde 1 h bei RT gerührt und dann nochmals mit 12 µl Methansulfonsäurechlorid versetzt (insgesamt 0.32 mmol, 2.1 eq.). Nach 1 h wurden die flüchtigen Bestandteile am Rotationsverdampfer entfernt. Der Rückstand wurde in wenig DMSO gelöst und über präparative HPLC [Methode 8] gereinigt. Man erhielt 59 mg (61% d. Th.) der Titelverbindung.
LC/MS [Methode 4]: Rₜ = 1.06 min; MS [ESIpos]: m/z = 630 (M+H)⁺
¹H-NMR (400 MHz, DMSO-d₆): δ [ppm] = 2.70 (s, 3H), 3.31-3.46 (m, 2H), 3.84 (dd, 1H), 3.98 (dd, 1H), 4.22-4.35 (m, 1H), 4.37-4.50 (m [AB], 2H), 4.75-4.84 (m, 1H), 6.93 (d, 1H), 7.51 (t, 1H), 7.60-7.66 (m, 2H), 7.68-7.79 (m, 4H), 7.87 (d, 1H), 7.98 (d, 1H), 8.29 (t, 1H).

### Beispiel 17

### 2-{3-(4-Chlorphenyl)-5-oxo-4-[(2S)-3,3,3-trifluor-2-hydroxypropyl]-4,5-dihydro-1H-1,2,4-triazol-1-yl}-N-{2-[(methylsulfonyl)amino]-2-[2-(trifluormethyl)phenyl]ethyl}acetamid (Diastereomer 2)

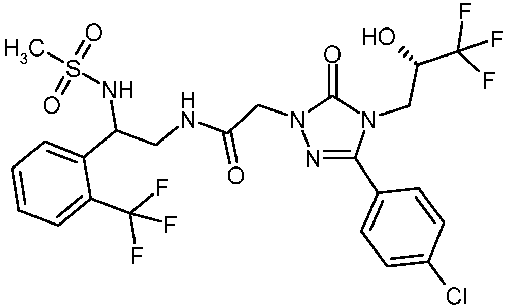

Analog zu Beispiel 16 wurden durch Behandlung von 67 mg (0.114 mmol) der Verbindung aus Beispiel 57A mit Methansulfonsäurechlorid 40 mg (56% d. Th.) der Titelverbindung erhalten.
LC/MS [Methode 4]: Rₜ = 1.06 min; MS [ESIpos]: m/z = 630 (M+H)⁺
¹H-NMR (400 MHz, DMSO-d₆): δ [ppm] = 2.70 (s, 3H), 3.27-3.37 (m, 1H), 3.37-3.47 (m, 1H), 3.84 (dd, 1H), 3.97 (dd, 1H), 4.23-4.35 (m, 1H), 4.44 (s, 2H), 4.76-4.85 (m, 1H), 6.92 (d, 1H), 7.51 (t, 1H), 7.64 (d, 2H), 7.68-7.78 (m, 4H), 7.86 (d, 1H), 7.98 (d, 1H), 8.28 (t, 1H).

### Beispiel 18

### tert.-Butyl-{2-[({3-(4-chlorphenyl)-5-oxo-4-[(2S)-3,3,3-trifluor-2-hydrolypropyl]-4,5-dihydro-1H-1,2,4-triazol-1-yl}acetyl)amino]-1-(2,3-dichlorphenyl)ethyl}carbamat (Diastereomerengemisch)

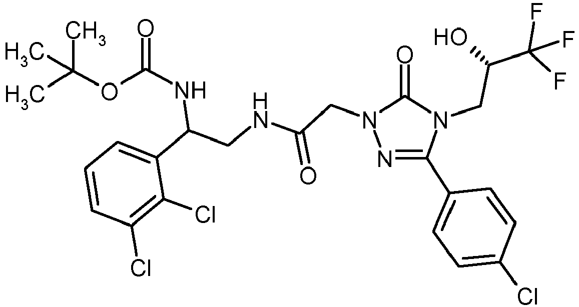

185 mg (0.51 mmol) der Verbindung aus Beispiel 8A und 170 mg (0.56 mmol) der Verbindung aus Beispiel 24A wurden analog zu Beispiel 13 umgesetzt. Man erhielt 300 mg (88% d. Th.) der Titelverbindung als Diastereomerengemisch.
LC/MS [Methode 4]: Rₜ = 1.24 min; MS [ESIpos]: m/z = 652 (M+H)⁺
¹H-NMR (400 MHz, DMSO-d₆): δ [ppm] = 1.34 (s, 9H), 3.27-3.42 (m, 2H), 3.83 (dd, 1H), 3.97 (2 dd, zus. 1H), 4.23-4.48 (m, 3H), 5.03-5.14 (m, 1H), 6.92 (2 d, zus. 1H), 7.36 (t, 1H), 7.41-7.47 (m, 1H), 7.50-7.58 (m, 2H), 7.63 (d, 2H), 7.75 (d, 2H), 8.22 (2 t, zus. 1H).

Durch präparative HPLC an chiraler Phase [Methode 15a] konnten die beiden Diastereomere getrennt werden, siehe Beispiel 19 und Beispiel 20.

### Beispiel 19

### tert.-Butyl-{2-[({3-(4-chlorphenyl)-5-oxo-4-[(2S)-3,3,3-trifluor-2-hydroxypropyl]-4,5-dihydro-1H-1,2,4-triazol-1-yl}acetyl)amino]-1-(2,3-dichlorphenyl)ethyl}carbamat (Diastereomer 1)

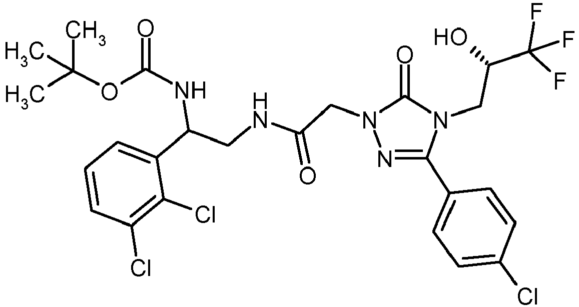

Zuerst eluierendes Diastereomer (150 mg) aus der chromatographischen Trennung von 300 mg der Verbindung aus Beispiel 18 nach Methode 15a.
Chirale analytische HPLC [Methode 16]: Rₜ = 2.15 min.
LC/MS [Methode 3]: Rₜ = 1.43 min; MS [ESIneg]: m/z = 650 (M-H)⁻.

### Beispiel 20

### tert.-Butyl-{2-[({3-(4-chlorphenyl)-5-oxo-4-[(2S)-3,3,3-trifluor-2-hydroxypropyl]-4,5-dihydro-1H-1,2,4-triazol-1-yl}acetyl)amino]-1-(2,3-dichlorphenyl)ethyl}carbamat (Diastereomer 2)

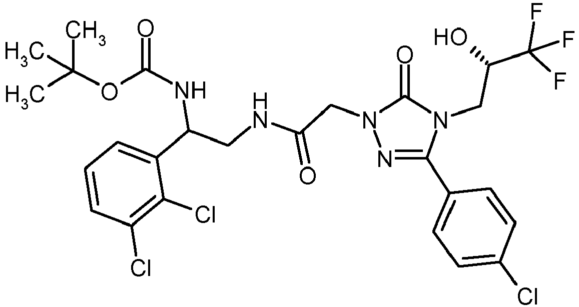

Zuletzt eluierendes Diastereomer (150 mg) aus der chromatographischen Trennung von 300 mg der Verbindung aus Beispiel 18 nach Methode 15a.
Chirale analytische HPLC [Methode 16]: Rₜ = 5.33 min.
LC/MS [Methode 3]: Rₜ = 1.43 min; MS [ESIneg]: m/z = 650 (M-H)⁻.

### Beispiel 21

### 2-{3-(4-Chlorphenyl)-5-oxo-4-[(2S)-3,3,3-trifluor-2-hydroxypropyl]-4,5-dihydro-1H-1,2,4-triazol-1-yl}-N-{2-(2,3-dichlorphenyl)-2-[(methylsulfonyl)amino]ethyl}acetamid (Diastereomer 1)

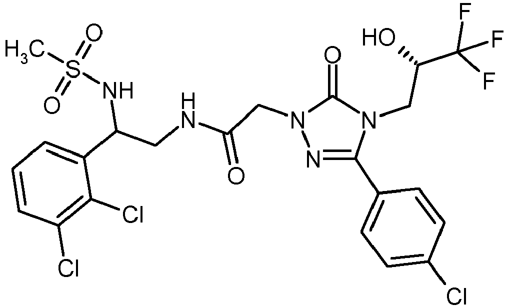

Analog zu Beispiel 16 wurden durch Behandlung von 77 mg (0.131 mmol) der Verbindung aus Beispiel 54A mit Methansulfonsäurechlorid 55 mg (67% d. Th.) der Titelverbindung erhalten.
LC/MS [Methode 4]: Rₜ = 1.08 min; MS [ESIpos]: m/z = 630 (M+H)⁺
¹H-NMR (400 MHz, DMSO-d₆): δ [ppm] = 2.83 (s, 3H), 3.31-3.43 (m, 2H), 3.84 (dd, 1H), 3.98 (dd, 1H), 4.23-4.35 (m, 1H), 4.36-4.49 (m [AB], 2H), 4.94-5.02 (m, 1H), 6.93 (d, 1H), 7.42 (t, 1H), 7.56-7.67 (m, 4H), 7.73-7.80 (m, 2H), 7.98 (d, 1H), 8.30 (t, 1H).

### Beispiel 22

### 2-{3-(4-Chlorphenyl)-5-oxo-4-[(2S)-3,3,3-trifluor-2-hydroxypropyl]-4,5-dihydro-1H-1,2,4-triazol-1-yl}-N-{2-(2,3-dichlorphenyl)-2-[(methylsulfonyl)amino]ethyl}acetamid (Diastereomer 2)

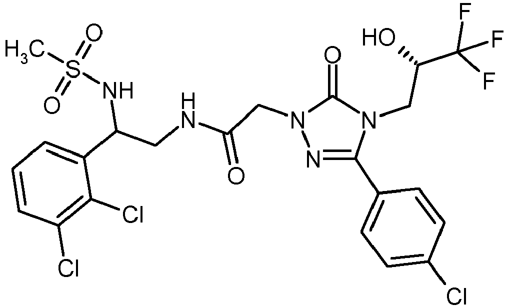

Analog zu Beispiel 16 wurden durch Behandlung von 76 mg (0.13 mmol) der Verbindung aus Beispiel 55A mit Methansulfonsäurechlorid 59 mg (73% d. Th.) der Titelverbindung erhalten.
LC/MS [Methode 4]: Rₜ = 1.08 min; MS [ESIpos]: m/z = 630 (M+H)⁺
¹H-NMR (400 MHz, DMSO-d₆): δ [ppm] = 2.83 (s, 3H), 3.34-3.44 (m, 2H), 3.84 (dd, 1H), 3.97 (dd, 1H), 4.29 (d, 1H), 4.43 (s, 2H), 4.94-5.02 (m, 1H), 6.93 (d, 1H), 7.42 (t, 1H), 7.57-7.62 (m, 2H), 7.62-7.67 (m, 2H), 7.72-7.78 (m, 2H), 7.99 (d, 1H), 8.28 (t, 1H).

### Beispiel 23

### N-[2-(Carbamoylamino)-2-(2,3-dichlorphenyl)ethyl]-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomer 1)

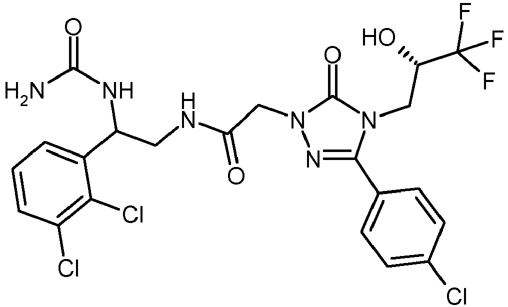

Eine Mischung von 30 mg (51 µmol) der Verbindung aus Beispiel 54A, 1 ml Wasser und 1 ml Methanol wurde mit 12 mg Kaliumcyanat (153 µmol) versetzt und 1.5 h bei 40°C gerührt. Zusätzliche 6 mg (75 µmol) Kaliumcyanat wurden hinzugefügt, und die Reaktionsmischung wurde über Nacht bei RT weiter gerührt. Danach wurden ein paar ml DMSO zugegeben und die gesamte Lösung über präparative HPLC [Methode 8] aufgetrennt. Die Produktfraktion wurde am Rotationsverdampfer vom Lösungsmittel befreit und der Rückstand im Hochvakuum getrocknet. Man erhielt 17 mg (56% d. Th.) der Titelverbindung.
LC/MS [Methode 4]: Rₜ = 0.98 min; MS [ESIpos]: m/z = 595 (M+H)⁺
¹H-NMR (400 MHz, DMSO-d₆): δ [ppm] = 3.28-3.38 (m, 2H), 3.83 (dd, 1H), 3.96 (dd, 1H), 4.24-4.35 (m, 1H), 4.34-4.46 (m [AB], 2H), 5.14 (q, 1H), 5.64 (s, 2H), 6.69 (d, 1H), 6.94 (d, 1H), 7.33-7.40 (m, 2H), 7.50-7.57 (m, 1H), 7.64 (d, 2H), 7.76 (d, 2H), 8.29 (t, 1H).

### Beispiel 24

### N-[2-(Carbamoylamino)-2-(2,3-dichlorphenyl)ethyl]-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomer 2)

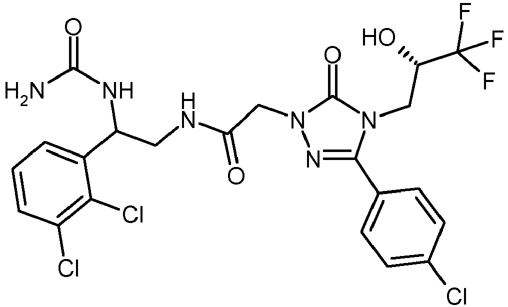

Analog zu Beispiel 23 wurden aus 30 mg (51 µmol) der Verbindung aus Beispiel 55A und Kaliumcyanat 19 mg (63% d. Th.) der Titelverbindung erhalten.
LC/MS [Methode 4]: Rₜ = 0.99 min; MS [ESIpos]: m/z = 595 (M+H)⁺
¹H-NMR (400 MHz, DMSO-d₆): δ [ppm] = 3.27-3.42 (m, 2H), 3.83 (dd, 1H), 3.96 (dd, 1H), 4.24-4.35 (m, 1H), 4.34-4.46 (m [AB], 2H), 5.10-5.18 (m, 1H), 5.63 (s, 2H), 6.69 (d, 1H), 6.92 (d, 1H), 7.32-7.41 (m, 2H), 7.54 (dd, 1H), 7.64 (d, 2H), 7.76 (d, 2H), 8.28 (t, 1H).

### Beispiel 25

### N-{2-(2-Chlorphenyl)-2-[(methylsulfonyl)amino]ethyl}-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomerengemisch)

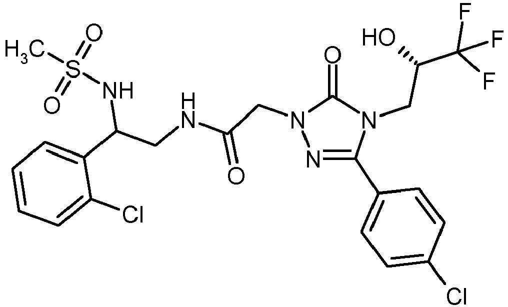

Eine Mischung aus 249 mg (0.68 mmol) der Verbindung aus Beispiel 8A, 186 mg (0.75 mmol) der Verbindung aus Beispiel 27A, 195 mg (1.02 mmol) EDC und 138 mg (1.02 mmol) HOBt in 6.5 ml DMF wurde 2 h bei RT gerührt. Anschließend wurde das Gemisch direkt über präparative HPLC [Methode 8] in seine Komponenten aufgetrennt. Die Produktfraktion wurde am Rotationsverdampfer vom Lösungsmittel befreit und der Rückstand im Hochvakuum getrocknet. Man erhielt 356 mg (83% d. Th.) der Titelverbindung als Diastereomerengemisch.
LC/MS [Methode 4]: Rₜ = 1.01 min; MS [ESIpos]: m/z = 596 (M+H)⁺.

Durch präparative HPLC an chiraler Phase [Methode 17a] konnten die beiden Diastereomere getrennt werden, siehe Beispiel 26 und Beispiel 27.

### Beispiel 26

### N-{2-(2-Chlorphenyl)-2-[(methylsulfonyl)amino]ethyl}-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomer 1)

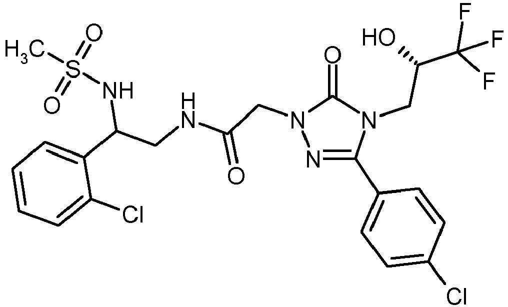

Zuerst eluierendes Diastereomer aus der chromatographischen Trennung von 356 mg der Verbindung aus Beispiel 25 nach Methode 17a. Das so erhaltene Material (150 mg) wurde noch einmal durch präparative HPLC [Methode 8] feingereinigt. Man erhielt 100 mg der sauberen Titelverbindung.
Chirale analytische HPLC [Methode 18a]: Rₜ = 4.10 min.
LC/MS [Methode 4]: Rₜ = 1.01 min; MS [ESIpos]: m/z = 596 (M+H)⁺
¹H-NMR (400 MHz, DMSO-d₆): δ [ppm] = 2.78 (s, 3H), 3.31-3.38 (m, 2H), 3.84 (dd, 1H), 3.98 (dd, 1H), 4.22-4.36 (m, 1H), 4.37-4.49 (m [AB], 2H), 4.89-4.97 (m, 1H), 6.93 (d, 1H), 7.31 (dt, 1H), 7.36-7.46 (m, 2H), 7.60-7.67 (m, 3H), 7.76 (d, 2H), 7.91 (d, 1H), 8.29 (t, 1H).

### Beispiel 27

### N-{2-(2-Chlorphenyl)-2-[(methylsulfonyl)amino]ethyl}-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomer 2)

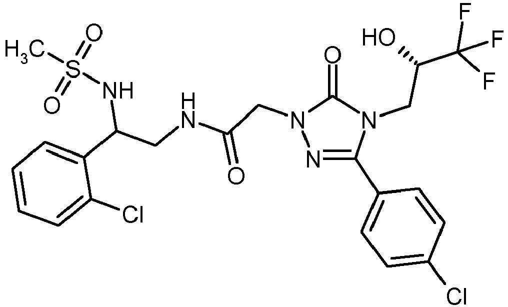

Zuletzt eluierendes Diastereomer aus der chromatographischen Trennung von 356 mg der Verbindung aus Beispiel 25 nach Methode 17a. Das so erhaltene Material (160 mg) wurde noch einmal durch präparative HPLC [Methode 8] feingereinigt. Man erhielt 120 mg der sauberen Titelverbindung.
Chirale analytische HPLC [Methode 18a]: Rₜ = 4.94 min.
LC/MS [Methode 4]: Rₜ = 1.02 min; MS [ESIpos]: m/z = 596 (M+H)⁺
¹H-NMR (400 MHz, DMSO-d₆): δ [ppm] = 2.78 (s, 3H), 3.29-3.42 (m, 2H), 3.84 (dd, 1H), 3.97 (dd, 1H), 4.23-4.35 (m, 1H), 4.43 (s, 2H), 4.89-4.99 (m, 1H), 6.93 (d, 1H), 7.32 (dt, 1H), 7.36-7.46 (m, 2H), 7.61-7.67 (m, 3H), 7.76 (d, 2H), 7.92 (d, 1H), 8.28 (t, 1H).

### Beispiel 28

### N-{2-(2-Chlorphenyl)-2-[(ethylsulfonyl)amino]ethyl}-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomerengemisch)

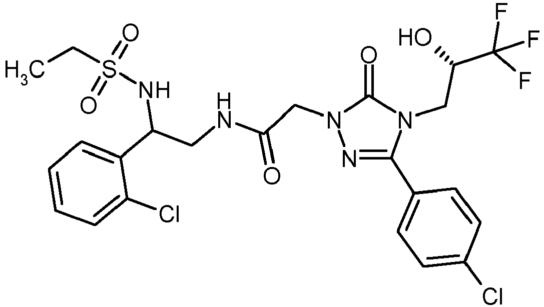

Analog zu Beispiel 25 wurden aus 216 mg (0.59 mmol) der Verbindung aus Beispiel 8A und 190 mg (90% Reinheit, 0.65 mmol) der Verbindung aus Beispiel 29A 274 mg (73% d. Th.) der Titelverbindung erhalten.
LC/MS [Methode 2]: Rₜ = 2.24 min; MS [ESIpos]: m/z = 610 (M+H)⁺.

Durch präparative HPLC an chiraler Phase [Methode 17b] konnten die beiden Diastereomere getrennt werden, siehe Beispiel 29 und Beispiel 30.

### Beispiel 29

### N-{2-(2-Chlorphenyl)-2-[(ethylsulfonyl)amino]ethyl}-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomer 1)

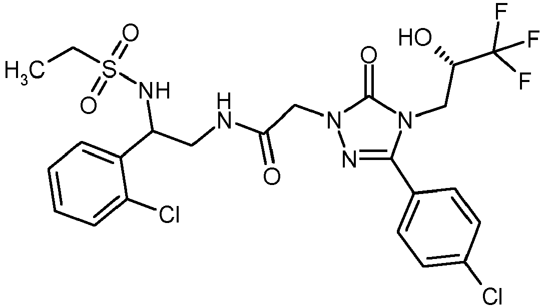

Zuerst eluierendes Diastereomer aus der chromatographischen Trennung von 274 mg der Verbindung aus Beispiel 28 nach Methode 17b. Das so erhaltene Material (123 mg) wurde noch einmal durch präparative HPLC [Methode 19] feingereinigt. Man erhielt 92 mg der sauberen Titelverbindung.
Chirale analytische HPLC [Methode 18a]: Rₜ = 4.27 min.
LC/MS [Methode 4]: Rₜ = 1.04 min; MS [ESIpos]: m/z = 610 (M+H)⁺
¹H-NMR (400 MHz, DMSO-d₆): δ [ppm] = 1.08 (t, 3H), 2.68-2.80 (m, 1H), 2.88 (dq, 1H), 3.30-3.44 (m, 2H), 3.84 (dd, 1H), 3.97 (dd, 1H), 4.24-4.35 (m, 1H), 4.35-4.46 (m [AB], 2H), 4.93 (q, 1H), 6.91 (d, 1H), 7.27-7.34 (m, 1H), 7.36-7.45 (m, 2H), 7.60-7.68 (m, 3H), 7.76 (d, 2H), 7.91 (d, 1H), 8.24 (t, 1H).

### Beispiel 30

### N-{2-(2-Chlorphenyl)-2-[(ethylsulfonyl)amino]ethyl}-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomer 2)

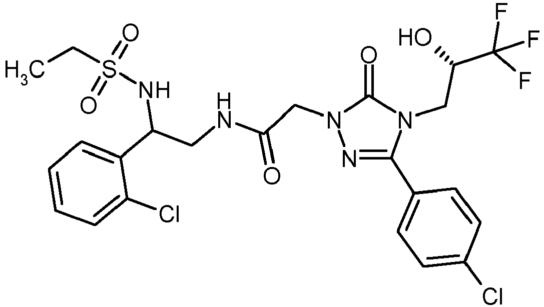

Zuletzt eluierendes Diastereomer aus der chromatographischen Trennung von 274 mg der Verbindung aus Beispiel 28 nach Methode 17b. Das so erhaltene Material (109 mg) wurde noch einmal durch präparative HPLC [Methode 19] feingereinigt. Man erhielt 82 mg der sauberen Titelverbindung.
Chirale analytische HPLC [Methode 18a]: Rₜ = 5.02 min.
LC/MS [Methode 4]: Rₜ = 1.04 min; MS [ESIpos]: m/z = 610 (M+H)⁺
¹H-NMR (500 MHz, DMSO-d₆): δ [ppm] = 1.08 (t, 3H), 2.74 (dq, 1H), 2.88 (dq, 1H), 3.27-3.35 (m, 1H), 3.35-3.44 (m, 1H), 3.84 (dd, 1H), 3.96 (dd, 1H), 4.24-4.34 (m, 1H), 4.35-4.45 (m [AB], 2H), 4.90-4.99 (m, 1H), 6.88 (d, 1H), 7.27-7.34 (m, 1H), 7.36-7.45 (m, 2H), 7.60-7.68 (m, 3H), 7.75 (d, 2H), 7.88 (d, 1H), 8.20 (br. t, 1H).

### Beispiel 31

### N-[2-(Carbamoylamino)-2-(2-chlorphenyl)ethyl]-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomerengemisch)

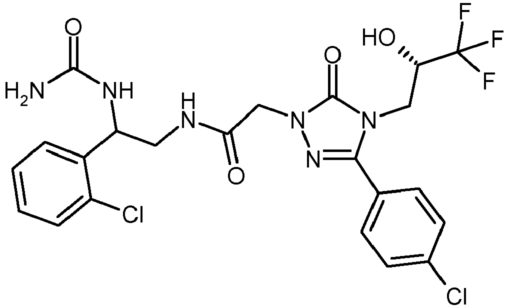

Analog zu Beispiel 25 wurden aus 118 mg (0.32 mmol) der Verbindung aus Beispiel 8A und 94 mg (81% Reinheit, 0.36 mmol) der Verbindung aus Beispiel 31A 138 mg (73% d. Th.) der Titelverbindung erhalten.
LC/MS [Methode 2]: Rₜ = 2.02 min; MS [ESIpos]: m/z = 561 (M+H)⁺.

Durch präparative HPLC an chiraler Phase [Methode 17c] konnten die beiden Diastereomere getrennt werden, siehe Beispiel 32 und Beispiel 33.

### Beispiel 32

### N-[2-(Carbamoylamino)-2-(2-chlorphenyl)ethyl]-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomer 1)

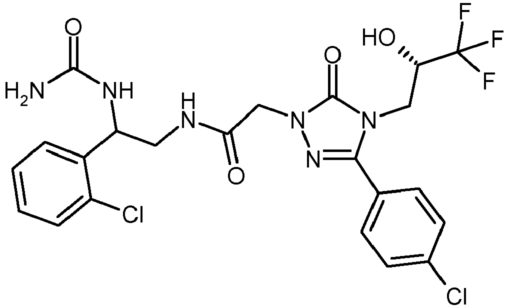

Zuerst eluierendes Diastereomer aus der chromatographischen Trennung von 138 mg der Verbindung aus Beispiel 31 nach Methode 17c. Das so erhaltene Material (31 mg) wurde noch einmal durch präparative HPLC [Methode 19] feingereinigt. Man erhielt 21 mg der sauberen Titelverbindung.
Chirale analytische HPLC [Methode 18b]: Rₜ = 6.80 min.
LC/MS [Methode 4]: Rₜ = 0.94 min; MS [ESIpos]: m/z = 561 (M+H)⁺
¹H-NMR (500 MHz, DMSO-d₆): δ [ppm] = 3.31-3.37 (m, 2H), 3.84 (dd, 1H), 3.96 (dd, 1H), 4.25-4.35 (m, 1H), 4.35-4.45 (m [AB], 2H), 5.13 (q, 1H), 5.56 (s, 2H), 6.58 (d, 1H), 6.89 (d, 1H), 7.25-7.30 (m, 1H), 7.33 (t, 1H), 7.38-7.45 (m, 2H), 7.63 (d, 2H), 7.76 (d, 2H), 8.21 (br. t, 1H).

### Beispiel 33

### N-[2-(Carbamoylamino)-2-(2-chlorphenyl)ethyl]-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomer 2)

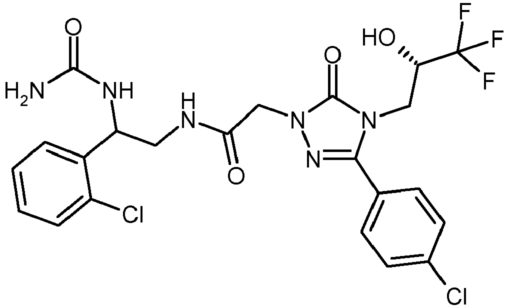

Zuletzt eluierendes Diastereomer aus der chromatographischen Trennung von 138 mg der Verbindung aus Beispiel 31 nach Methode 17c. Das so erhaltene Material (40 mg) wurde noch einmal durch präparative HPLC [Methode 19] feingereinigt. Man erhielt 24 mg der sauberen Titelverbindung.
Chirale analytische HPLC [Methode 18b]: Rₜ = 8.50 min.
LC/MS [Methode 4]: Rₜ = 0.93 min; MS [ESIpos]: m/z = 561 (M+H)⁺
¹H-NMR (500 MHz, DMSO-d₆): δ [ppm] = 3.28-3.37 (m, 2H), 3.83 (dd, 1H), 3.97 (dd, 1H), 4.25-4.35 (m, 1H), 4.35-4.45 (m [AB], 2H), 5.12 (q, 1H), 5.57 (br. s, 2H), 6.58 (d, 1H), 6.90 (d, 1H), 7.25-7.30 (m, 1H), 7.34 (t, 1H), 7.38-7.44 (m, 2H), 7.63 (d, 2H), 7.76 (d, 2H), 8.22 (br. t, 1H).

### Beispiel 34

### tert. -Butyl-{2-[({3-(4-chlorphenyl)-5-oxo-4-[(2S)-3,3,3-trifluor-2-hydrolypropyl]-4,5-dihydro-1H-1,2,4-triazol-1-yl}acetyl)amino]-1-[3-(trifluormethyl)phenyl]ethyl}carbamat (Diastereomerengemisch)

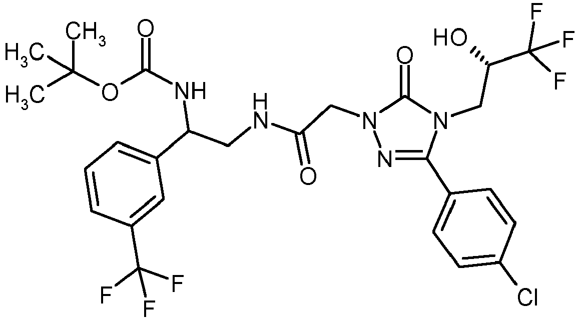

Analog zu Beispiel 25 wurden aus 152 mg (0.42 mmol) der Verbindung aus Beispiel 8A und 150 mg (0.46 mmol) *tert*.-Butyl-{2-amino-1-[3-(trifluormethyl)phenyl]ethyl}carbamat 240 mg (88% d. Th.) der Titelverbindung erhalten.
LC/MS [Methode 4]: Rₜ = 1.23 min; MS [ESIpos]: m/z = 652 (M+H)⁺
¹H-NMR (400 MHz, DMSO-d₆): δ [ppm] = 1.35 (s, 9H), 3.27-3.42 (m, 2H), 3.83 (dd, 1H), 3.91-4.01 (m, 1H), 4.22-4.44 (m, 3H), 4.68-4.78 (m, 1H), 6.92 (2 d, zus. 1H), 7.49-7.68 (m, 7H), 7.72-7.78 (m, 2H), 8.22 (2 t, zus. 1H).

### Beispiel 35

### N-{2-Acetamido-2-[3-(trifluormethyl)phenyl]ethyl}-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomerengemisch)

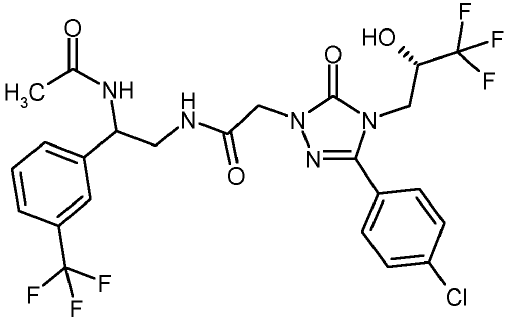

Eine Lösung von 60 mg (0.10 mmol) der Verbindung aus Beispiel 58A in 1 ml Dichlormethan wurde mit 20 µl (0.11 mmol) N,N-Diisopropylethylamin versetzt. Die Mischung wurde auf 0°C gekühlt, dann mit 10 µl (0.10 mmol) Essigsäureanhydrid versetzt und weiter 1 h bei 0°C gerührt. Danach wurden die flüchtigen Bestandteile am Rotationsverdampfer entfernt. Der Rückstand wurde in wenig DMSO gelöst und über präparative HPLC [Methode 8] aufgetrennt. Die produkthaltige Fraktion wurde am Rotationsverdampfer vom Lösungsmittel befreit und der Rückstand im Hochvakuum getrocknet. Man erhielt 50 mg (83% d. Th.) der Titelverbindung.
LC/MS [Methode 2]: Rₜ = 2.19 min; MS [ESIpos]: m/z = 594 (M+H)⁺
¹H-NMR (400 MHz, DMSO-d₆): δ [ppm] = 1.86 (s, 3H), 3.30-3.47 (m, 2H), 3.83 (dd, 1H), 3.96 (br. d, 1H), 4.26-4.43 (m, 3H), 5.02 (q, 1H), 6.91 (d, 1H), 7.51-7.69 (m, 6H), 7.75 (d, 2H), 8.19 (br. t, 1H), 8.39 (d, 1H).

### Beispiel 36

### 2-{3-(4-Chlorphenyl)-5-oxo-4-[(2S)-3,3,3-trifluor-2-hydroxypropyl]-4,5-dihydro-1H-1,2,4-triazol-1-yl}-N-{2-formamido-2-[3-(trifluormethyl)phenyl]ethyl}acetamid (Diastereomerengemisch)

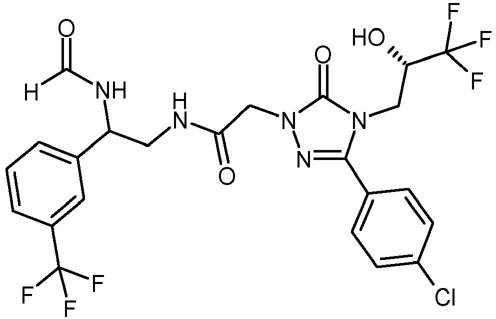

Eine Lösung von 60 mg (102 µmol) der Verbindung aus Beispiel 58A in 1 ml THF wurde mit 20 µl (112 µmol) *N,N*-Diisopropylethylamin versetzt. Die Mischung wurde auf 0°C gekühlt, dann portionsweise mit 18 mg (107 µmol) 4-Nitrophenylformiat versetzt und weiter 1 h bei 0°C gerührt. Da die LC/MS-Analyse der Reaktionsmischung auf die zusätzliche Bildung eines *O*-formylierten Nebenprodukts hinwies, wurden 408 µl einer 1 N Lösung von Lithiumhydroxid in Wasser zum Reaktionsgemisch hinzugefügt. Die Mischung wurde danach über Nacht bei RT weiter gerührt. Anschließend wurden die flüchtigen Bestandteile am Rotationsverdampfer entfernt. Der Rückstand wurde in wenig DMSO gelöst und über präparative HPLC [Methode 8] aufgetrennt. Die produkthaltige Fraktion wurde am Rotationsverdampfer vom Lösungsmittel befreit und der Rückstand im Hochvakuum getrocknet. Man erhielt 35 mg (59% d. Th.) der Titelverbindung.
LC/MS [Methode 2]: Rₜ = 2.17 min; MS [ESIpos]: m/z = 580 (M+H)⁺
¹H-NMR (400 MHz, DMSO-d₆): δ [ppm] = 3.32-3.51 (m, 2H), 3.83 (dd, 1H), 3.96 (br. d, 1H), 4.25-4.46 (m, 3H), 5.11 (q, 1H), 6.90 (d, 1H), 7.53-7.71 (m, 6H), 7.75 (d, 2H), 8.11 (s, 1H), 8.23 (br. t, 1H), 8.65 (d, 1H).

### Beispiel 37

### 2-{3-(4-Chlorphenyl)-5-oxo-4-[(2S)-3,3,3-trifluor-2-hydroxypropyl]-4,5-dihydro-1H-1,2,4-triazol-1-yl}-N-{2-[(methylsulfonyl)amino]-2-[3-(trifluormethyl)phenyl]ethyl}acetamid (Diastereomerengemisch)

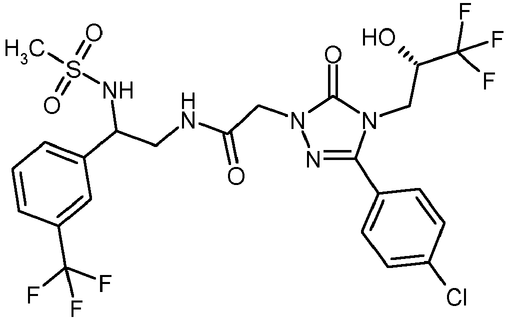

Eine Lösung von 30 mg (51 µmol) der Verbindung aus Beispiel 58A in 0.5 ml Pyridin wurde mit 4 µl (56 µmol) Methansulfonsäurechlorid versetzt und bei RT über Nacht gerührt. Nachdem die HPLC-Analyse noch viel Ausgangsmaterial zeigte, wurden portionsweise weitere Äquivalente an Methansulfonsäurechlorid (insgesamt 3.1 eq.) bis zur vollständigen Umsetzung hinzugefügt. Anschließend wurden jeweils 100 µl Wasser und Methanol zugegeben. Nach 5 min Rühren wurde das Reaktionsgemisch mit ca. 3 ml DMSO verdünnt und über präparative HPLC [Methode 8] aufgetrennt. Die produkthaltige Fraktion wurde am Rotationsverdampfer vom Lösungsmittel befreit und der Rückstand im Hochvakuum getrocknet. Man erhielt 22 mg (68% d. Th.) der Titelverbindung.
LC/MS [Methode 4]: Rₜ = 1.08 min; MS [ESIpos]: m/z = 630 (M+H)⁺
¹H-NMR (400 MHz, DMSO-d₆): δ [ppm] = 2.79 (s, 3H), 3.34-3.44 (m, 2H), 3.84 (dd, 1H), 3.97 (2 dd, zus. 1H), 4.23-4.34 (m, 1H), 4.34-4.47 (m, 2H), 4.57 (q, 1H), 6.92 (d, 1H), 7.55-7.69 (m, 5H), 7.72-7.78 (m, 3H), 7.92 (d, 1H), 8.28 (2 t, zus. 1H).

### Beispiel 38

### N-{2-Acetamido-2-[3-(trifluormethyl)phenyl]propyl}-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomer 1)

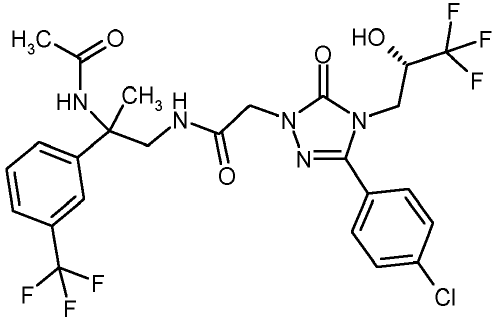

Eine Lösung von 38 mg (62 µmol) der Verbindung aus Beispiel 53A in 0.96 ml Dichlormethan wurde bei RT mit 12 µl (68 µmol) *N,N*-Diisopropylethylamin, dann mit 6 µl (62 µmol) Essigsäureanhydrid versetzt und 1 h gerührt. Anschließend wurden die flüchtigen Bestandteile am Rotationsverdampfer entfernt. Da die LC/MS-Analyse des Rohprodukts auf die zusätzliche Bildung eines *O*-acetylierten Nebenprodukts hinwies, wurde der Rückstand in 2 ml Methanol gelöst und mit 800 µl 2 N Natronlauge versetzt. Nach 72 h wurde die Mischung mit 1 N Salzsäure angesäuert und über präparative HPLC [Methode 8] aufgetrennt. Dabei konnten die beiden Produkt-Diastereomere in separierter Form erhalten werden. Man erhielt 6 mg (16% d. Th.) der Titelverbindung (Diastereomer 1) sowie 7 mg (19% d. Th.) des zweiten Diastereomers (siehe Beispiel 39).
LC/MS [Methode 3]: Rₜ = 1.26 min; MS [ESIpos]: m/z = 608 (M+H)⁺
¹H-NMR (400 MHz, DMSO-d₆): δ [ppm] = 1.50 (s, 3H), 1.84 (s, 3H), 3.57-3.72 (m, 2H), 3.82 (dd, 1H), 3.96 (dd, 1H), 4.21-4.34 (m, 1H), 4.45 (m [AB], 2H), 6.91 (d, 1H), 7.49-7.66 (m, 6H), 7.69-7.75 (m, 2H), 8.12 (s, 1H), 8.16 (t, 1H).

### Beispiel 39

### N-{2-Acetamido-2-[3-(trifluormethyl)phenyl]propyl}-2-{3-(4-chlorphenyl)-5-oxo-4-[(2S)-3,3,3-trifluor-2-hydroxypropyl]-4,5-dihydro-1H-1,2,4-triazol-1-yl}acetamid (Diastereomer 2)

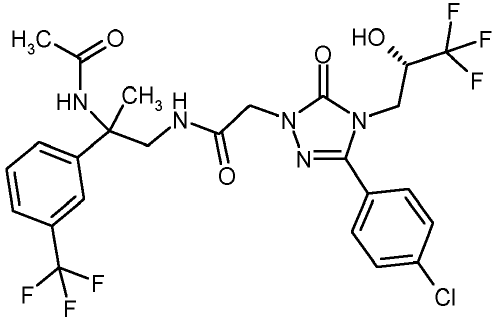

Zweites Diastereomer (7 mg, 19% d. Th.) isoliert aus der Umsetzung der Verbindung aus Beispiel 53A mit Essigsäureanhydrid (siehe unter Beispiel 38).
LC/MS [Methode 3]: Rₜ = 1.27 min; MS [ESIpos]: m/z = 608 (M+H)⁺
¹H-NMR (400 MHz, DMSO-d₆): δ [ppm] = 1.50 (s, 3H), 1.85 (s, 3H), 3.65 (br. d, 2H), 3.82 (dd, 1H), 3.96 (dd, 1H), 4.22-4.35 (m, 1H), 4.40-4.53 (m [AB], 2H), 6.91 (d, 1H), 7.49-7.66 (m, 6H), 7.69-7.77 (m, 2H), 8.12 (s, 1H), 8.15 (t, 1H).

### Beispiel 40

### 2-{3-(4-Chlorphenyl)-5-oxo-4-[(2S)-3,3,3-trifluor-2-hydroxypropyl]-4,5-dihydro-1H-1,2,4-triazol-1-yl}-N-{2-formamido-2-[3-(trifluormethyl)phenyl]propyl}acetamid (Diastereomerengemisch)

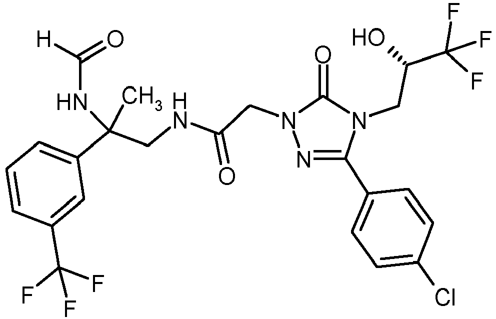

Eine Lösung von 38 mg (62 µmol) der Verbindung aus Beispiel 53A in 1 ml THF wurde bei RT mit 12 µl (68 µmol) *N,N*-Diisopropylethylamin, dann mit 11 mg (65 µmol) 4-Nitrophenylformiat versetzt und bei RT gerührt. Nach 1 h wurden nochmals 10 mg (62 µmol) 4-Nitrophenylformiat zugegeben und die Reaktionsmischung über Nacht weiter gerührt. Da die LC/MS-Analyse auf die zusätzliche Bildung eines *O*-formylierten Nebenprodukts hinwies, wurden 248 µl einer 1 N Lösung von Lithiumhydroxid in Wasser zur Reaktionsmischung hinzugefügt. Nach 1 h wurde das Gemisch mit 1 N Salzsäure angesäuert und über präparative HPLC [Methode 8] aufgetrennt. Man erhielt 30 mg (81% d. Th.) der Titelverbindung als Diastereomerengemisch.
LC/MS [Methode 4]: Rₜ = 1.07 min; MS [ESIpos]: m/z = 594 (M+H)⁺
¹H-NMR (400 MHz, DMSO-d₆): δ [ppm] = 1.56 (s, 3H), 3.58-3.72 (m, 2H), 3.82 (dd, 1H), 3.96 (dd, 1H), 4.22-4.36 (m, 1H), 4.38-4.53 (m, 2H), 6.91 (d, 1H), 7.52-7.67 (m, 6H), 7.68-7.78 (m, 2H), 8.03 (br. s, 1H), 8.15-8.25 (m, 1H), 8.36 (br. d, 1H).

### Beispiel 41

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-1-[2-(trifluormethyl)phenyl]ethylcarbamat (Diastereomerengemisch)

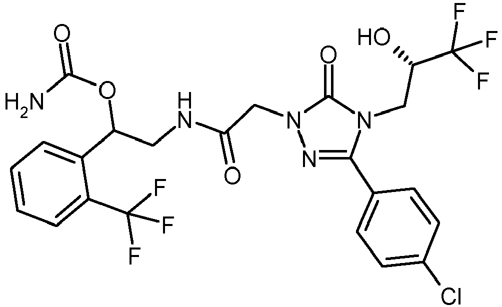

371 mg (1.02 mmol) der Verbindung aus Beispiel 8A, 292 mg (1.52 mmol) EDC und 206 mg (1.52 mmol) HOBt wurden in 10 ml DMF 5 min bei RT gerührt. Anschließend wurde die resultierende Lösung zu einer Lösung von 280 mg (90% Reinheit, 1.02 mmol) der Verbindung aus Beispiel 37A in 40 ml Acetonitril zugetropft. Nach 30 min bei RT wurde das Acetonitril am Rotationsverdampfer entfernt. Die restliche Lösung wurde mit 1 ml 1 M Salzsäure versetzt und direkt durch präparative HPLC [Methode 8] in ihre Komponenten aufgetrennt. Die Produktfraktion wurde am Rotationsverdampfer vom Lösungsmittel befreit und der Rückstand im Hochvakuum getrocknet. Man erhielt 481 mg (80% d. Th.) der Titelverbindung als Diastereomerengemisch.
LC/MS [Methode 4]: Rₜ = 1.03 min; MS [ESIpos]: m/z = 596 (M+H)⁺.

Durch präparative HPLC an chiraler Phase [Methode 15a] konnten die beiden Diastereomere getrennt werden, siehe Beispiel 42 und Beispiel 43.

### Beispiel 42

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-1-[2-(trifluormethyl)phenyl]ethylcarbamat (Diastereomer 1)

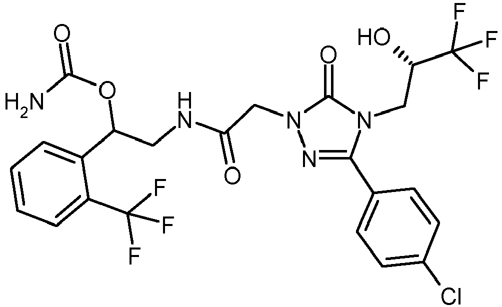

Zuerst eluierendes Diastereomer aus der chromatographischen Trennung von 480 mg der Verbindung aus Beispiel 41 nach Methode 15a. Das so erhaltene Material (254 mg) wurde noch einmal durch präparative HPLC [Methode 8] feingereinigt. Man erhielt 220 mg der sauberen Titelverbindung.
Chirale analytische HPLC [Methode 16]: Rₜ = 2.26 min.
LC/MS [Methode 4]: Rₜ = 1.03 min; MS [ESIpos]: m/z = 596 (M+H)⁺
¹H-NMR (400 MHz, DMSO-d₆): δ [ppm] = 3.40-3.52 (m, 2H), 3.83 (dd, 1H), 3.96 (dd, 1H), 4.23-4.34 (m, 1H), 4.34-4.47 (m [AB], 2H), 5.66 (t, 1H), 6.53-6.90 (br. d, 2H), 6.93 (d, 1H), 7.55-7.71 (m, 6H), 7.76 (d, 2H), 8.35 (t, 1H).

### Beispiel 43

### 2-[({3-(4-Chlorphenyl)-5-oxo-4-[(2S-3,3,3-trifluor-2-hydroxypropyl]-4,5-dihydro-1H-1,2,4-triazol-1-yl}acetyl)amino]-1-[2-(trifluormethyl)phenyl]ethylcarbamat (Diastereomer 2)

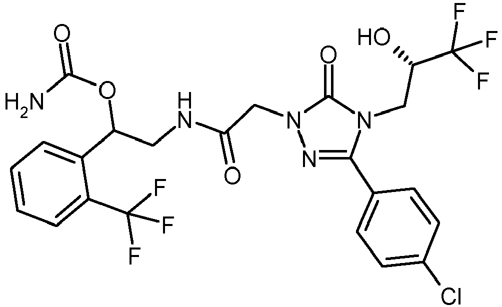

Zuletzt eluierendes Diastereomer aus der chromatographischen Trennung von 480 mg der Verbindung aus Beispiel 41 nach Methode 15a. Das so erhaltene Material (258 mg) wurde noch einmal durch präparative HPLC [Methode 8] feingereinigt. Man erhielt 220 mg der sauberen Titelverbindung.
Chirale analytische HPLC [Methode 16]: Rₜ = 4.33 min.
LC/MS [Methode 4]: Rₜ = 1.03 min; MS [ESIpos]: m/z = 596 (M+H)⁺
¹H-NMR (400 MHz, DMSO-d₆): δ [ppm] = 3.39-3.53 (m, 2H), 3.83 (dd, 1H), 3.96 (dd, 1H), 4.23-4.35 (m, 1H), 4.40 (s, 2H), 5.66 (t, 1H), 6.51-6.90 (br. d, 2H), 6.92 (d, 1H), 7.58-7.71 (m, 6H), 7.76 (d, 2H), 8.35 (t, 1H).

### Beispiel 44

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-1-[3-(trifluormethyl)phenyl]ethylcarbamat (Diastereomerengemisch)

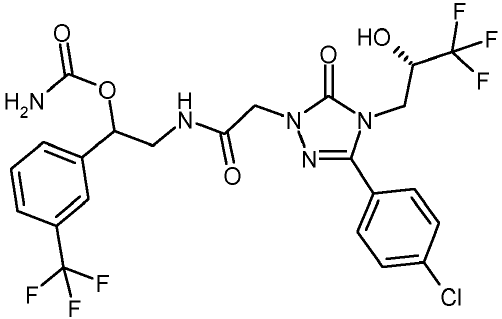

78 mg (0.21 mmol) der Verbindung aus Beispiel 8A, 73 mg (0.26 mmol) der Verbindung aus Beispiel 39A, 43 mg (0.26 mmol) EDC, 36 mg (0.26 mmol) HOBt und 56 µl (0.32 mmol) *N,N*-Diisopropylethylamin wurden in 2 ml DMF 30 min bei RT gerührt. Die Lösung wurde danach mit 1 ml 1 M Salzsäure versetzt und direkt durch präparative HPLC [Methode 8] in ihre Komponenten aufgetrennt. Die Produktfraktion wurde am Rotationsverdampfer vom Lösungsmittel befreit und der Rückstand im Hochvakuum getrocknet. Man erhielt 95 mg (75% d. Th.) der Titelverbindung als Diastereomerengemisch.
LC/MS [Methode 4]: Rₜ = 1.05 min; MS [ESIpos]: m/z = 596 (M+H)⁺.

Durch präparative HPLC an chiraler Phase [Methode 15a] konnten die beiden Diastereomere getrennt werden, siehe Beispiel 45 und Beispiel 46.

### Beispiel 45

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-1-[3-(trifluormethyl)phenyl]ethylcarbamat (Diastereomer 1)

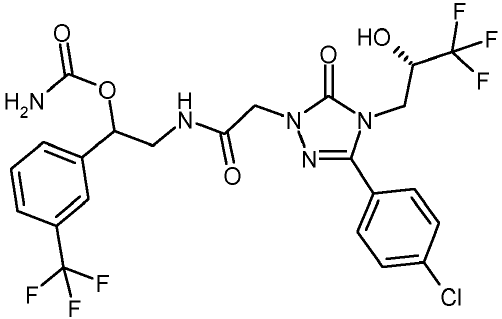

Zuerst eluierendes Diastereomer aus der chromatographischen Trennung von 95 mg der Verbindung aus Beispiel 44 nach Methode 15a. Das so erhaltene Material (44 mg) wurde noch einmal durch präparative HPLC [Methode 8] feingereinigt. Man erhielt 33 mg der sauberen Titelverbindung.
Chirale analytische HPLC [Methode 16]: Rₜ = 2.27 min.
LC/MS [Methode 2]: Rₜ = 2.27 min; MS [ESIpos]: m/z = 596 (M+H)⁺
¹H-NMR (400 MHz, DMSO-d₆): δ [ppm] = 3.39-3.53 (m, 2H), 3.83 (dd, 1H), 3.96 (dd, 1H), 4.23-4.36 (m, 1H), 4.40 (s, 2H), 5.66 (t, 1H), 6.56-6.89 (br. d, 2H), 6.92 (d, 1H), 7.57-7.71 (m, 6H), 7.72-7.79 (m, 2H), 8.34 (t, 1H).

### Beispiel 46

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-1-[3-(trifluormethyl)phenyl]ethylcarbamat (Diastereomer 2)

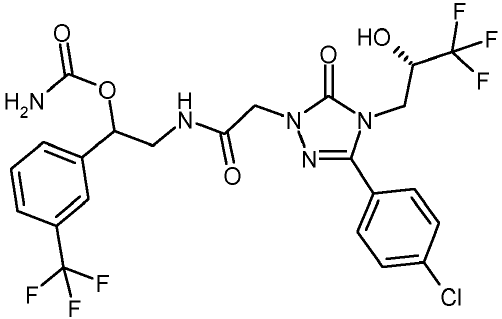

Zuletzt eluierendes Diastereomer aus der chromatographischen Trennung von 95 mg der Verbindung aus Beispiel 44 nach Methode 15a. Das so erhaltene Material (44 mg) wurde noch einmal durch präparative HPLC [Methode 8] feingereinigt. Man erhielt 35 mg der sauberen Titelverbindung.
Chirale analytische HPLC [Methode 16]: Rₜ = 4.33 min.
LC/MS [Methode 3]: Rₜ = 1.21 min; MS [ESIpos]: m/z = 596 (M+H)⁺
¹H-NMR (400 MHz, DMSO-d₆): δ [ppm] = 3.40-3.52 (m, 2H), 3.83 (dd, 1H), 3.96 (dd, 1H), 4.23-4.35 (m, 1H), 4.33-4.48 (m [AB], 2H), 5.66 (t, 1H), 6.55-6.88 (br. d, 2H), 6.92 (d, 1H), 7.57-7.71 (m, 6H), 7.73-7.79 (m, 2H), 8.34 (t, 1H).

### Beispiel 47

### 1-(2-Chlorphenyl)-2-[({3-(4-chlorphenyl)-5-oxo-4-[(2S)-3,3,3-trifluor-2-hydroxypropyl]-4,5-dihydro-1H-1,2,4-triazol-1-yl}acetyl)amino]ethylcarbamat (Diastereomerengemisch)

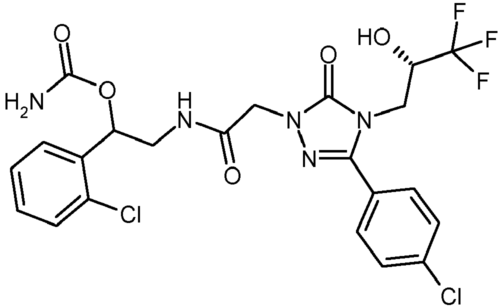

78 mg (0.21 mmol) der Verbindung aus Beispiel 8A, 61 mg (0.32 mmol) EDC und 46 mg (0.32 mmol) HOBt wurden in 2 ml DMF 20 min bei RT gerührt. Anschließend wurde die resultierende Lösung zu einer Lösung von 46 mg (0.21 mmol) der Verbindung aus Beispiel 40A in 8 ml Acetonitril zugetropft. Nach 30 min bei RT wurde das Acetonitril am Rotationsverdampfer entfernt. Die restliche Lösung wurde mit 1 ml 1 M Salzsäure versetzt und direkt durch präparative HPLC [Methode 8] in ihre Komponenten aufgetrennt. Die Produktfraktion wurde am Rotationsverdampfer vom Lösungsmittel befreit und der Rückstand im Hochvakuum getrocknet. Man erhielt 59 mg (49% d. Th.) der Titelverbindung als Diastereomerengemisch.
LC/MS [Methode 2]: Rₜ = 2.10 min; MS [ESIpos]: m/z = 562 (M+H)⁺.

Durch präparative HPLC an chiraler Phase [Methode 15a] konnten die beiden Diastereomere getrennt werden, siehe Beispiel 48 und Beispiel 49.

### Beispiel 48

### 1-(2-Chlorphenyl)-2-[({3-(4-chlorphenyl)-5-oxo-4-[(2S)-3,3,3-trifluor-2-hydroxypropyl]-4,5-dihydro-1H-1,2,4-triazol-1-yl}acetyl)amino]ethylcarbamat (Diastereomer 1)

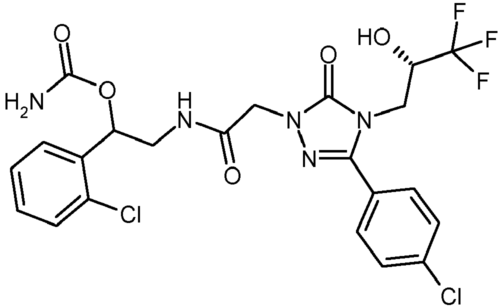

Zuerst eluierendes Diastereomer aus der chromatographischen Trennung von 59 mg der Verbindung aus Beispiel 47 nach Methode 15a. Das so erhaltene Material (28 mg) wurde noch einmal durch präparative HPLC [Methode 8] feingereinigt. Man erhielt 22 mg der sauberen Titelverbindung.
Chirale analytische HPLC [Methode 16]: Rₜ = 2.75 min.
LC/MS [Methode 3]: Rₜ = 1.13 min; MS [ESIpos]: m/z = 562 (M+H)⁺
¹H-NMR (400 MHz, DMSO-d₆): δ [ppm] = 3.35-3.50 (m, 2H), 3.83 (dd, 1H), 3.96 (dd, 1H), 4.23-4.36 (m, 1H), 4.42 (s, 2H), 5.90 (dd, 1H), 6.53-6.89 (br. d, 2H), 6.93 (d, 1H), 7.31-7.42 (m, 2H), 7.42-7.48 (m, 2H), 7.63 (d, 2H), 7.76 (d, 2H), 8.38 (t, 1H).

### Beispiel 49

### 1-(2-Chlorphenyl)-2-[({3-(4-chlorphenyl)-5-oxo-4-[(2S)-3,3,3-trifluor-2-hydroxypropyl]-4,5-dihydro-1H-1,2,4-triazol-1-yl}acetyl)amino]ethylcarbamat (Diastereomer 2)

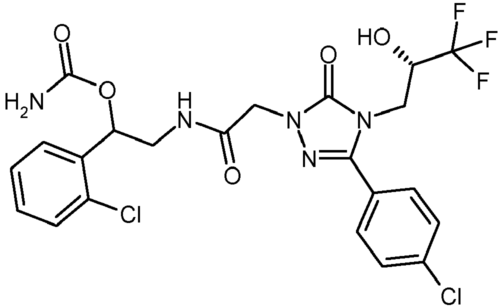

Zuletzt eluierendes Diastereomer aus der chromatographischen Trennung von 59 mg der Verbindung aus Beispiel 47 nach Methode 15a. Das so erhaltene Material (30 mg) wurde noch einmal durch präparative HPLC [Methode 8] feingereinigt. Man erhielt 19 mg der sauberen Titelverbindung.
Chirale analytische HPLC [Methode 16]: Rₜ = 5.11 min.
LC/MS [Methode 4]: Rₜ = 0.98 min; MS [ESIpos]: m/z = 562 (M+H)⁺
¹H-NMR (400 MHz, DMSO-d₆): δ [ppm] = 3.33-3.42 (m, 1H), 3.43-3.53 (m, 1H), 3.83 (dd, 1H), 3.97 (dd, 1H), 4.23-4.35 (m, 1H), 4.35-4.49 (m [AB], 2H), 5.90 (dd, 1H), 6.53-6.87 (br. s, 2H), 6.93 (d, 1H), 7.30-7.42 (m, 2H), 7.42-7.48 (m, 2H), 7.63 (d, 2H), 7.76 (d, 2H), 8.38 (t, 1H).

### Beispiel 50

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-1-(2,3-dichlorphenyl)ethylcarbamat (Diastereomerengemisch)

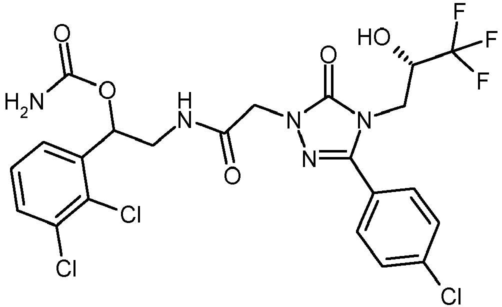

73 mg (0.20 mmol) der Verbindung aus Beispiel 8A, 60 mg (0.24 mmol) der Verbindung aus Beispiel 44A, 46 mg (0.24 mmol) EDC und 34 mg (0.24 mmol) HOBt wurden in 2 ml DMF über Nacht bei RT gerührt. Die Lösung wurde danach mit 1 ml 1 M Salzsäure versetzt und direkt durch präparative HPLC [Methode 8] in ihre Komponenten aufgetrennt. Die Produktfraktion wurde am Rotationsverdampfer vom Lösungsmittel befreit und der Rückstand im Hochvakuum getrocknet. Man erhielt 100 mg (83% d. Th.) der Titelverbindung als Diastereomerengemisch.
LC/MS [Methode 4]: Rₜ = 1.03 min; MS [ESIpos]: m/z = 596 (M+H)⁺.

Durch präparative HPLC an chiraler Phase [Methode 15a] konnten die beiden Diastereomere getrennt werden, siehe Beispiel 51 und Beispiel 52.

### Beispiel 51

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-1-(2,3-dichlorphenyl)ethylcarbamat (Diastereomer 1)

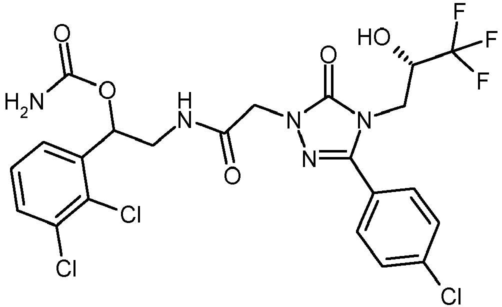

Zuerst eluierendes Diastereomer aus der chromatographischen Trennung von 100 mg der Verbindung aus Beispiel 50 nach Methode 15a. Das so erhaltene Material (47 mg) wurde noch einmal durch präparative HPLC [Methode 8] feingereinigt. Man erhielt 32 mg der sauberen Titelverbindung.
Chirale analytische HPLC [Methode 16]: Rₜ = 3.20 min.
LC/MS [Methode 4]: Rₜ = 1.03 min; MS [ESIpos]: m/z = 596 (M+H)⁺
¹H-NMR (400 MHz, DMSO-d₆): δ [ppm] = 3.44 (t, 2H), 3.83 (dd, 1H), 3.96 (dd, 1H), 4.22-4.35 (m, 1H), 4.41 (s, 2H), 5.91 (t, 1H), 6.59-6.89 (br. d, 2H), 6.93 (d, 1H), 7.38-7.44 (m, 2H), 7.58-7.67 (m, 3H), 7.76 (d, 2H), 8.39 (t, 1H).

### Beispiel 52

### 2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-1-(2,3-dichlorphenyl)ethylcarbamat (Diastereomer 2)

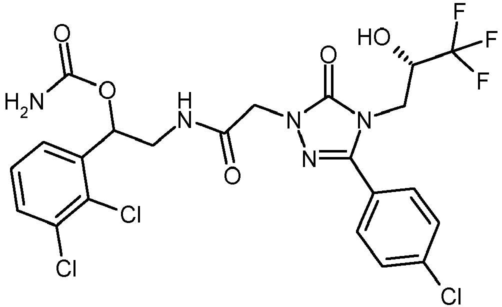

Zuletzt eluierendes Diastereomer aus der chromatographischen Trennung von 100 mg der Verbindung aus Beispiel 50 nach Methode 15a. Das so erhaltene Material (50 mg) wurde noch einmal durch präparative HPLC [Methode 8] feingereinigt. Man erhielt 39 mg der sauberen Titelverbindung.
Chirale analytische HPLC [Methode 16]: Rₜ = 6.05 min.
LC/MS [Methode 4]: Rₜ = 1.03 min; MS [ESIpos]: m/z = 596 (M+H)⁺
¹H-NMR (400 MHz, DMSO-d₆): δ [ppm] = 3.37-3.52 (m, 2H), 3.83 (dd, 1H), 3.96 (dd, 1H), 4.23-4.34 (m, 1H), 4.35-4.48 (m [AB], 2H), 5.90 (dd, 1H), 6.59-6.88 (br. d, 2H), 6.93 (d, 1H), 7.38-7.45 (m, 2H), 7.57-7.67 (m, 3H), 7.76 (d, 2H), 8.39 (t, 1H).

### Beispiel 53

### 2-{3-(4-Chlorphenyl)-5-oxo-4-[(2S)-3,3,3-trifluor-2-hydroxypropyl]-4,5-dihydro-1H-1,2,4-triazol-1-yl}-N-{(2R)-1-hydroxy-3-[3-(trifluormethyl)phenyl]propan-2-yl}acetamid

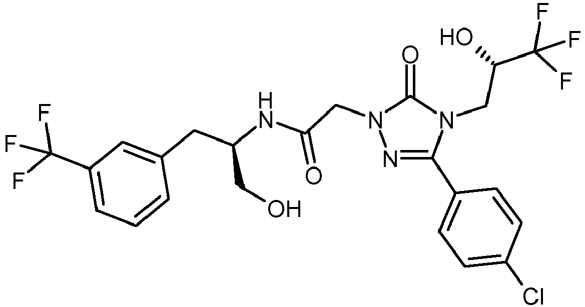

Zu einer Mischung von 50 mg (137 µmol) der Verbindung aus Beispiel 8A, 42 mg (164 µmol) der Verbindung aus Beispiel 45A, 39 mg (205 µmol) EDC und 28 mg (205 µmol) HOBt in 1.36 ml DMF wurden 48 µl *N,N*-Diisopropylethylamin gegeben. Das resultierende Gemisch wurde über Nacht bei RT gerührt und dann direkt über präparative HPLC [Methode 9] in seine Komponenten aufgetrennt. Man erhielt 61 mg (79% d. Th.) der Titelverbindung.
LC/MS [Methode 1]: Rₜ = 2.01 min; MS [ESIpos]: m/z = 567 (M+H)⁺
¹H-NMR (400 MHz, DMSO-d₆): δ [ppm] = 2.75 (dd, 1H), 2.96 (dd, 1H), 3.37 (dq, 2H), 3.83 (dd, 1H), 3.87-4.00 (m, 2H), 4.21-4.32 (m, 1H), 4.31 (d, 1H), 4.43 (s, 1H), 4.90 (t, 1H), 6.90 (d, 1H), 7.45-7.59 (m, 4H), 7.63 (d, 2H), 7.74 (d, 2H), 8.12 (d, 1H).

### Beispiel 54

### 2-{3-(4-Chlorphenyl)-5-oxo-4-[(2S)-3,3,3-trifluor-2-hydroxypropyl]-4,5-dihydro-1H-1,2,4-triazol-1-yl}-N {(2S)-1-hydroxy-3-[3-(trifluormethyl)phenyl]propan-2-yl}acetamid

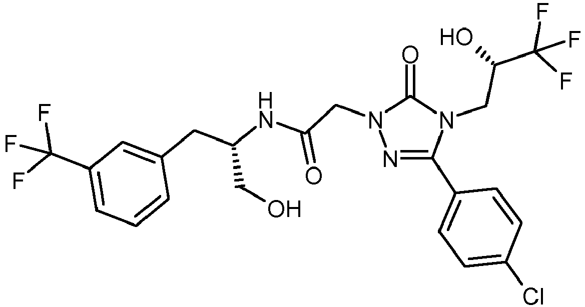

Analog zu Beispiel 53 wurden aus 50 mg (137 µmol) der Verbindung aus Beispiel 8A und 42 mg (164 µmol) der Verbindung aus Beispiel 46A 53 mg (68% d. Th.) der Titelverbindung erhalten.
LC/MS [Methode 3]: Rₜ = 1.26 min; MS [ESIpos]: m/z = 567 (M+H)⁺
¹H-NMR (400 MHz, DMSO-d₆): δ [ppm] = 2.75 (dd, 1H), 2.95 (dd, 1H), 3.38 (dq, 2H), 3.82 (dd, 1H), 3.87-4.00 (m, 2H), 4.22-4.33 (m, 1H), 4.30-4.43 (m, 2H), 4.91 (t, 1H), 6.91 (d, 1H), 7.44-7.59 (m, 4H), 7.60-7.66 (m, 2H), 7.74 (d, 2H), 8.11 (d, 1H).

### Beispiel 55

### (2R)-2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-3-[3-(trifluormethyl)phenyl]propylcarbamat

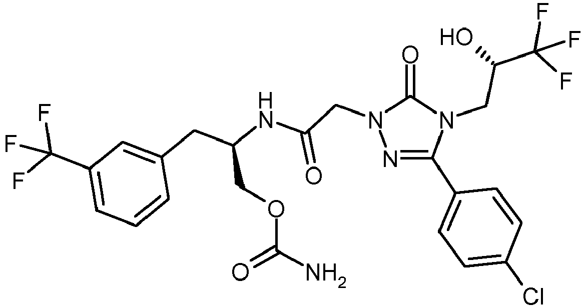

Zu einer Mischung von 38 mg (105 µmol) der Verbindung aus Beispiel 8A, 42 mg (115 µmol) der Verbindung aus Beispiel 47A, 28 mg (147 µmol) EDC und 21 mg (147 µmol) HOBt in 1.39 ml DMF wurden 37 µl (210 µmol) *N,N-*Diisopropylethylamin gegeben. Das resultierende Gemisch wurde 2 h bei RT gerührt, dann mit 1 ml 1 M Salzsäure versetzt und direkt über präparative HPLC [Methode 9] in seine Komponenten aufgetrennt. Man erhielt 61 mg (79% d. Th.) der Titelverbindung.
LC/MS [Methode 1]: Rₜ = 2.01 min; MS [ESIpos]: m/z = 610 (M+H)⁺
¹H-NMR (400 MHz, DMSO-d₆): δ [ppm] = 2.80 (dd, 1H), 2.93 (dd, 1H), 3.78-3.89 (m, 2H), 3.90-4.00 (m, 2H), 4.06-4.18 (m, 1H), 4.22-4.43 (m, 3H), 6.44-6.73 (br. s, 2H), 6.91 (d, 1H), 7.47-7.60 (m, 4H), 7.63 (d, 2H), 7.74 (d, 2H), 8.26 (d, 1H).

### Beispiel 56

### (2S)-2-[({3-(4-Chlorphenyl)-5-oxo-4-[(2S)-3,3,3-trifluor-2-hydroxypropyl]-4,5-dihydro-1H-1,2,4-triazol-1-yl}acetyl)amino]-3-[3-(trifluormethyl)phenyl]propylcarbamat

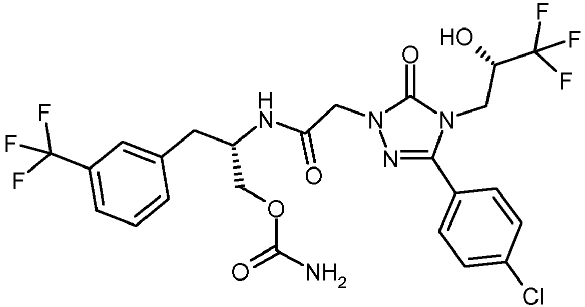

Zu einer Mischung von 70 mg (191 µmol) der Verbindung aus Beispiel 8A, 79 mg (264 µmol) der Verbindung aus Beispiel 48A, 44 mg (230 µmol) EDC und 33 mg (230 µmol) HOBt in 3 ml DMF wurden 67 µl (383 µmol) N,N-Diisopropylethylamin gegeben. Das resultierende Gemisch wurde über Nacht bei RT gerührt, dann mit 1 ml 1 M Salzsäure versetzt und direkt über präparative HPLC [Methode 9] in seine Komponenten aufgetrennt. Man erhielt 64 mg (55% d. Th.) der Titelverbindung.
LC/MS [Methode 1]: Rₜ = 2.03 min; MS [ESIpos]: m/z = 610 (M+H)⁺
¹H-NMR (400 MHz, DMSO-d₆): δ [ppm] = 2.80 (dd, 1H), 2.93 (dd, 1H), 3.78-3.89 (m, 2H), 3.90-4.00 (m, 2H), 4.06-4.18 (m, 1H), 4.22-4.32 (m, 1H), 4.35 (s, 2H), 6.42-6.72 (br. s, 2H), 6.91 (d, 1H), 7.46-7.59 (m, 4H), 7.60-7.66 (m, 2H), 7.72-7.77 (m, 2H), 8.25 (d, 1H).

### Beispiel 57

### 1-(2-Chlorphenyl)-2-({[3-(4-chlorphenyl)-4-(2-fluorbenzyl)-5-oxo-4,5-dihydro-1H-1,2,4-triazol-1-yl]acetyl}amino)ethylcarbamat (Racemat)

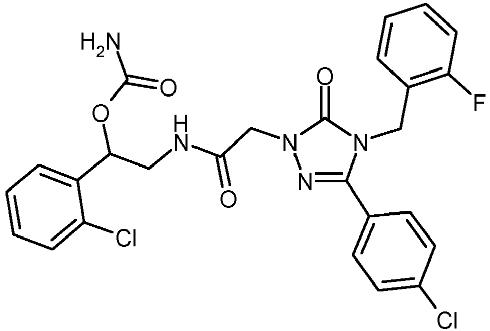

Eine Mischung von 66 mg (182 µmol) [3-(4-Chlorphenyl)-4-(2-fluorbenzyl)-5-oxo-4,5-dihydro-1H-1,2,4-triazol-1-yl]essigsäure [zur Herstellung siehe Beispiel 156A in WO 2007/134862], 47 mg (219 µmol) der Verbindung aus Beispiel 40A, 42 mg (219 µmol) EDC und 35 mg (219 µmol) HOBt in 4 ml DMF wurde über Nacht bei RT gerührt, dann mit 1 ml 1 M Salzsäure versetzt und direkt über präparative HPLC [Methode 23] in ihre Komponenten aufgetrennt. Man erhielt 64 mg (63% d. Th.) der Titelverbindung.
LC/MS [Methode 1]: Rₜ = 1.25 min; MS [ESIpos]: m/z = 558 (M+H)⁺
¹H-NMR (400 MHz, DMSO-d₆): δ [ppm] = 3.36-3.51 (m, 2H), 4.40-4.52 (m [AB], 2H), 5.03 (br. s, 2H), 5.90 (dd, 1H), 6.55-6.91 (2 br. s, 2H), 7.04-7.19 (m, 3H), 7.27-7.42 (m, 3H), 7.42-7.48 (m, 2H), 7.54 (s, 4H), 8.43 (t, 1H).

### Beispiel 58

### 1-(2-Chlorphenyl)-2-[({3-(4-chlorphenyl)-5-oxo-4-[(1E)-3,3,3-trifluorprop-1-en-1-yl]-4,5-dihydro-1H-1,2,4-triazol-1-yl}acetyl)amino]ethylcarbamat (Racemat)

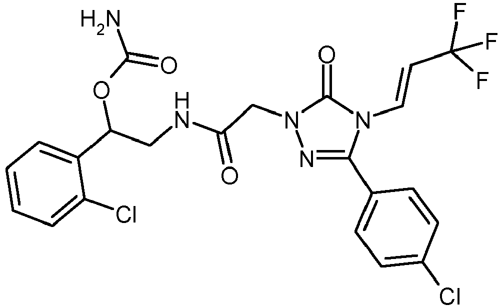

Eine Mischung von 128 mg (369 µmol) der Verbindung aus Beispiel 33A, 95 mg (443 µmol) der Verbindung aus Beispiel 40A, 85 mg (443 µmol) EDC und 71 mg (443 µmol) HOBt in 4 ml DMF wurde über Nacht bei RT gerührt, dann mit 1 ml 1 M Salzsäure versetzt und direkt über präparative HPLC [Methode 23] in ihre Komponenten aufgetrennt. Man erhielt 130 mg (65% d. Th.) der Titelverbindung.
LC/MS [Methode 3]: Rₜ = 1.30 min; MS [ESIpos]: m/z = 544 (M+H)⁺
¹H-NMR (400 MHz, DMSO-d₆): δ [ppm] = 3.36-3.51 (m, 2H), 4.40-4.52 (m [AB], 2H), 5.91 (dd, 1H), 6.50-6.95 (2 br. s, 2H), 6.87 (dq, 1H), 7.18 (dq, 1H), 7.30-7.41 (m, 2H), 7.45 (d, 2H), 7.62-7.72 (m, 4H), 8.44 (t, 1H).

### Beispiel 59

### 1-(2-Chlorphenyl)-2-({[3-(4-chlorphenyl)-5-oxo-4-(3,3,3-trifluorpropyl)-4,5-dihydro-1H-1,2,4-triazol-1-yl]acetyl}amino)ethylcarbamat (Racemat)

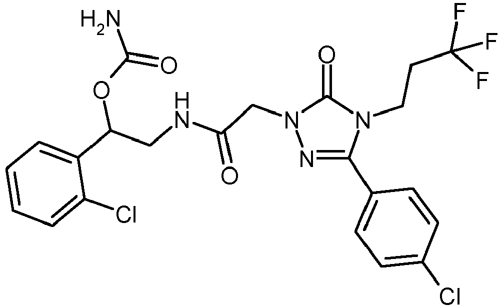

Eine Lösung von 50 mg (92 µmol) der Verbindung aus Beispiel 58 in 20 ml Methanol wurde in einer mit einer 5% Pt/C-Kartusche ausgestatteten Durchfluss-Hydrierapparatur (H-Cube, Firma Thales Nano, Budapest, Modell HC-2-SS) bei einem Durchfluss von 1 ml/min, einer Temperatur von 60°C und bei Normaldruck hydriert. Nach beendeter Umsetzung wurde die Lösung am Rotationsverdampfer vom Methanol befreit und der Rückstand durch präparative HPLC [Methode 23] gereinigt. Es wurden 22 mg (44% d. Th.) der Titelverbindung erhalten.
LC/MS [Methode 3]: Rₜ = 1.20 min; MS [ESIpos]: m/z = 546 (M+H)⁺
¹H-NMR (400 MHz, DMSO-d₆): δ [ppm] = 2.57-2.67 (m, 2H), 3.30-3.50 (m, 2H), 3.99 (t, 2H), 4.36-4.47 (m [AB], 2H), 5.89 (dd, 1H), 6.48-6.89 (2 br. s, 2H), 7.30-7.41 (m, 2H), 7.42-7.48 (m, 2H), 7.61-7.71 (m, 4H), 8.38 (t, 1H).

### Beispiel 60

### 2-({[3-(4-Chlorphenyl)-4-(2-fluorbenzyl)-5-oxo-4,5-dihydro-1H-1,2,4-triazol-1-yl]acety}amino)-1-[2-(trifluormethyl)phenyl]ethylcarbamat (Racemat)

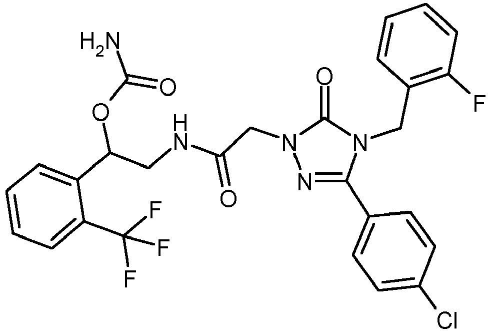

Eine Mischung von 40 mg (111 µmol) [3-(4-Chlorphenyl)-4-(2-fluorbenzyl)-5-oxo-4,5-dihydro-1*H-*1,2,4-triazol-1-yl]essigsäure [zur Herstellung siehe Beispiel 156A in WO 2007/134862], 33 mg (133 µmol) der Verbindung aus Beispiel 37A, 25 mg (133 µmol) EDC und 21 mg (133 µmol) HOBt in 2.4 ml DMF wurde über Nacht bei RT gerührt, dann mit 1 ml 1 M Salzsäure versetzt und direkt über präparative HPLC [Methode 20] in ihre Komponenten aufgetrennt. Man erhielt 53 mg (81% d. Th.) der Titelverbindung.
LC/MS [Methode 4]: Rₜ = 1.04 min; MS [ESIpos]: m/z = 592 (M+H)⁺
¹H-NMR (400 MHz, DMSO-d₆): δ [ppm] = 3.37-3.52 (m, 2H), 4.39-4.52 (m [AB], 2H), 5.03 (s, 2H), 5.92 (dd, 1H), 6.45-6.88 (2 br. s, 2H), 7.03-7.20 (m, 3H), 7.27-7.35 (m, 1H), 7.50-7.57 (m, 5H), 7.66-7.77 (m, 3H), 8.46 (t, 1H).

### Beispiel 61

### 2-[({3-(4-Chlorphenyl)-5-oxo-4-[(1E)-3,3,3-trifluorprop-1-en-1-yl]-4,5-dihydro-1H-1,2,4-triazol-1-yl}acetyl)amino]-1-[2-(trifluormethyl)phenyl]ethylcarbamat (Racemat)

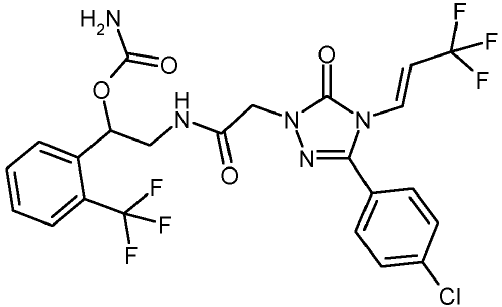

Eine Mischung von 39 mg (111 µmol) der Verbindung aus Beispiel 33A, 33 mg (133 µmol) der Verbindung aus 37A, 25 mg (133 µmol) EDC und 21 mg (133 µmol) HOBt in 1.2 ml DMF wurde über Nacht bei RT gerührt, dann mit 1 ml 1 M Salzsäure versetzt und direkt über präparative HPLC [Methode 20] in ihre Komponenten aufgetrennt. Man erhielt 52 mg (81% d. Th.) der Titelverbindung.
LC/MS [Methode 3]: Rₜ = 1.34 min; MS [ESIpos]: m/z = 578 (M+H)⁺
¹H-NMR (400 MHz, DMSO-d₆): δ [ppm] = 3.36-3.51 (m, 2H), 4.40-4.51 (m [AB], 2H), 5.89-5.96 (m, 1H), 6.47-6.82 (br. s, 2H), 6.86 (dq, 1H), 7.18 (dq, 1H), 7.54 (br. t, 1H), 7.62-7.75 (m, 7H), 8.46 (t, 1H).

### Beispiel 62

### 2-({[3-(4-Chlorphenyl)-5-oxo-4-(3,3,3-trifluorpropyl)-4,5-dihydro-1H-1,2,4-triazol-1-yl]acetyl}-amino)-1-[2-(trifluormethyl)phenyl]ethylcarbamat (Racemat)

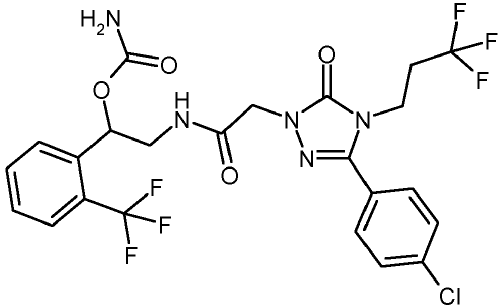

Eine Lösung von 30 mg (52 µmol) der Verbindung aus Beispiel 61 in 15 ml Methanol wurde in einer mit einer 5% Pt/C-Kartusche ausgestatteten Durchfluss-Hydrierapparatur (H-Cube, Firma Thales Nano, Budapest, Modell HC-2-SS) bei einem Durchfluss von 1 ml/min, einer Temperatur von 60°C und bei Normaldruck hydriert. Nach beendeter Umsetzung wurde die Lösung am Rotationsverdampfer vom Methanol befreit und der Rückstand durch präparative HPLC [Methode 23] gereinigt. Es wurden 15 mg (50% d. Th.) der Titelverbindung erhalten.
LC/MS [Methode 3]: Rₜ = 1.24 min; MS [ESIpos]: m/z = 580 (M+H)⁺
¹H-NMR (400 MHz, DMSO-d₆): δ [ppm] = 2.57-2.67 (m, 2H), 3.36-3.50 (m, 2H), 3.98 (t, 2H), 4.34-4.47 (m [AB], 2H), 5.86-5.94 (m, 1H), 6.45-6.86 (br. s, 2H), 7.53 (br. t, 1H), 7.60-7.76 (m, 7H), 8.40 (t, 1H).

### Beispiel 63

### 2-(2-Chlorphenyl)-3-[({3-(4-chlorphenyl)-5-oxo-4-[(1E)-3,3,3-trifluorprop-1-en-1-yl]-4,5-dihydro-1H-1,2,4-triazol-1-yl}acetyl)amino]propylcarbamat (Racemat)

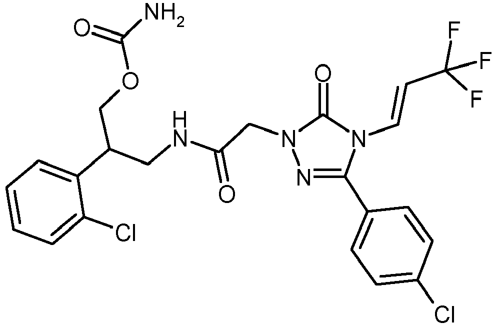

Eine Mischung von 47 mg (135 µmol) der Verbindung aus Beispiel 33A, 34 mg (176 µmol) EDC und 24 mg (176 µmol) HOBt in 1 ml DMF wurde 1 h bei RT gerührt und anschließend mit 34 mg (149 µmol) der Verbindung aus Beispiel 15A versetzt. Das Gemisch wurde 16 h bei RT gerührt und dann direkt über präparative HPLC [Methode 9] in seine Komponenten aufgetrennt. Man erhielt 28 mg (37% d. Th.) der Titelverbindung.
LC/MS [Methode 3]: Rₜ = 1.31 min; MS [ESIpos]: m/z = 558 (M+H)⁺
¹H-NMR (400 MHz, DMSO-d₆): δ [ppm] = 3.30-3.40 (m, 1H), 3.48 (dt, 1H), 3.66 (quin, 1H), 4.12-4.22 (m, 2H), 4.36-4.46 (m, 2H), 6.48 (br. s, 2H), 6.86 (dq, 1H), 7.13-7.21 (m, 1H), 7.23-7.34 (m, 2H), 7.39-7.46 (m, 2H), 7.60-7.71 (m, 4H), 8.24 (t, 1H).

### Beispiel 64

### 2-(2-Chlorphenyl)-3-({[3-(4-chlorphenyl)-4-(2-fluorbenzyl)-5-oxo-4,5-dihydro-1H-1,2,4-triazol-1-yl]acetyl}amino)propylcarbamat (Racemat)

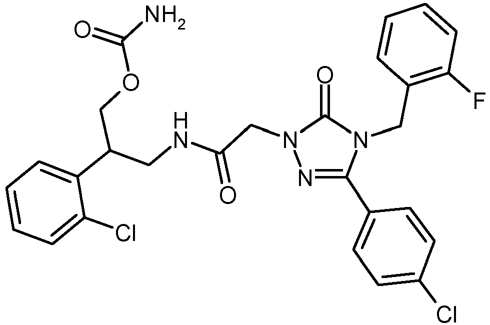

Analog zur Vorschrift von Beispiel 63 wurden 49 mg (135 µmol) [3-(4-Chlorphenyl)-4-(2-fluorbenzyl)-5-oxo-4,5-dihydro-1*H*-1,2,4-triazol-1-yl]essigsäure [zur Herstellung siehe Beispiel 156A in WO 2007/134862] mit 34 mg (149 µmol) der Verbindung aus Beispiel 15A umgesetzt. Man erhielt 27 mg (35% d. Th.) der Titelverbindung.
LC/MS [Methode 3]: Rₜ = 1.26 min; MS [ESIpos]: m/z = 572/574 (M+H)⁺
¹H-NMR (400 MHz, DMSO-d₆): δ [ppm] = 3.31-3.41 (m, 1H), 3.45-3.55 (m, 1H), 3.66 (quin, 1H), 4.12-4.22 (m, 2H), 4.35-4.46 (m, 2H), 5.02 (s, 2H), 6.48 (br. s, 2H), 7.02-7.20 (m, 3H), 7.22-7.35 (m, 3H), 7.39-7.46 (m, 2H), 7.49-7.56 (m, 4H), 8.23 (t, 1H).

### Beispiel 65

### tert.-Butyl-{2-({[3-(4-chlorphenyl)-4-cyclopropyl-5-oxo-4,5-dihydro-1H-1,2,4-triazol-1-yl]acetyl}-amino)-1-[2-(trifluormethyl)phenyl]ethyl}carbamat (Racemat)

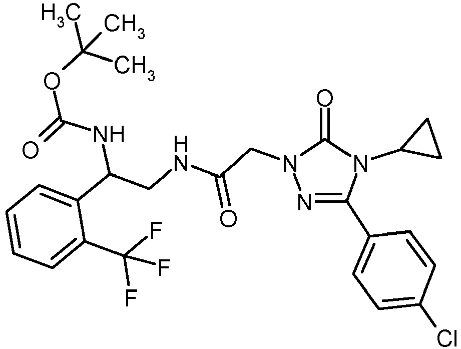

Analog zur Vorschrift von Beispiel 63 wurden 43 mg (146 µmol) [3-(4-Chlorphenyl)-4-cyclopropyl-5-oxo-4,5-dihydro-1*H*-1,2,4-triazol-1-yl]essigsäure [zur Herstellung siehe Beispiel 88A in WO 2007/134862] mit 49 mg (161 µmol) *tert*.-Butyl-{2-amino-1-[2-(trifluormethyl)phenyl]ethyl}-carbamat umgesetzt. Man erhielt 59 mg (69% d. Th.) der Titelverbindung.
LC/MS [Methode 4]: Rₜ = 1.14 min; MS [ESIpos]: m/z = 580 (M+H)⁺
¹H-NMR (400 MHz, DMSO-d₆): δ [ppm] = 0.54-0.61 (m, 2H), 0.87-0.94 (m, 2H), 1.33 (s, 9H), 3.18 (tt, 1H), 3.22-3.33 (m, 2H), 4.29-4.39 (m, 2H), 5.01 (br. s, 1H), 7.47 (q, 2H), 7.60 (d, 2H), 7.63-7.75 (m, 3H), 7.81 (d, 2H), 8.20 (m, 1H).

### Beispiel 66

### tert.-Butyl-{2-({[3-(5-chlor-2-thienyl)-4-(2-methoxyethyl)-5-oxo-4,5-dihydro-1H-1,2,4-triazol-1-yl]-acetyl}amino)-1-[2-(trifluormethyl)phenyl]ethyl}carbamat (Racemat)

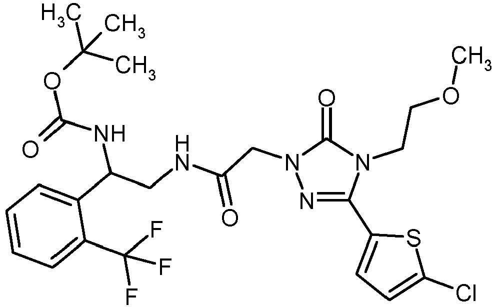

Analog zur Vorschrift von Beispiel 63 wurden 47 mg (146 µmol) der Verbindung aus Beispiel 52A mit 49 mg (161 µmol) *tert*.-Butyl-{2-amino-1-[2-(trifluormethyl)phenyl]ethyl}carbamat umgesetzt. Man erhielt 60 mg (68% d. Th.) der Titelverbindung.
LC/MS [Methode 4]: Rₜ = 1.13 min; MS [ESIpos]: m/z = 604 (M+H)⁺
¹H-NMR (400 MHz, DMSO-d₆): δ [ppm] = 1.35 (s, 9H), 3.21 (s, 3H), 3.23-3.28 (m, 1H), 3.35-3.42 (m, 1H), 3.55 (t, 2H), 3.98 (t, 2H), 4.37 (s, 2H), 5.00 (br. s, 1H), 7.27 (d, 1H), 7.43-7.54 (m, 2H), 7.58 (d, 1H), 7.63-7.76 (m, 3H), 8.26 (m, 1H).

### Beispiel 67

### tert.-Butyl-{2-({[3-(5-chlor-2-thienyl)-4-(2-fluorbenzyl)-5-oxo-4,5-dihydro-1H-1,2,4-triazol-1-yl]-acetyl}amino)-1-[2-(trifluormethyl)phenyl]ethyl}carbamat (Racemat)

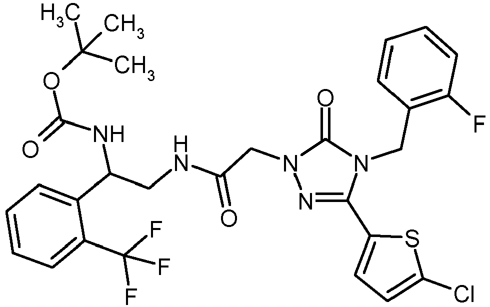

Analog zur Vorschrift von Beispiel 63 wurden 54 mg (146 µmol) [3-(5-Chlor-2-thienyl)-4-(2-fluorbenzyl)-5-oxo-4,5-dihydro-1*H*-1,2,4-triazol-1-yl]essigsäure [zur Herstellung siehe Beispiel 154A in WO 2007/134862] mit 49 mg (161 µmol) *tert*.-Butyl-{2-amino-1-[2-(trifluormethyl)phenyl]-ethyl}carbamat umgesetzt. Man erhielt 64 mg (67% d. Th.) der Titelverbindung.
LC/MS [Methode 3]: Rₜ = 1.52 min; MS [ESIpos]: m/z = 654 (M+H)⁺
¹H-NMR (400 MHz, DMSO-d₆): δ [ppm] = 1.35 (s, 9H), 3.20-3.41 (m, 2H), 4.38-4.49 (m, 2H), 4.96-5.08 (m, 1H), 5.15 (s, 2H), 7.03-7.10 (m, 1H), 7.12-7.28 (m, 4H), 7.31-7.39 (m, 1H), 7.42-7.49 (m, 1H), 7.53 (d, 1H), 7.63-7.78 (m, 3H), 8.28-8.36 (m, 1H).

### Beispiel 68

### 2-[3-(4-Chlorphenyl)-4-cyclopropyl-5-oxo-4,5-dihydro-1H-1,2,4-triazol-1-yl]-N-{2-[(methyl-sulfonyl)amino]-2-[2-(trifluormethyl)phenyl]ethyl}acetamid (Racemat)

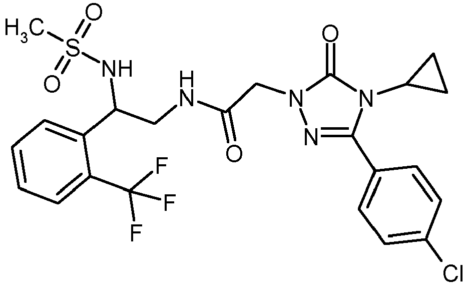

Eine Lösung von 29 mg (48 µmol) der Verbindung aus Beispiel 59A in 0.5 ml Pyridin wurde bei RT mit 4.1 µl Methansulfonsäurechlorid versetzt. Die Mischung wurde 1 h bei RT gerührt und dann erneut mit 4.1 µl Methansulfonsäurechlorid versetzt. Es wurde nochmals 18 h bei RT gerührt und anschließend weitere 12.3 µl Methansulfonsäurechlorid, verteilt über einen Zeitraum von 3 h, zugegeben (insgesamt 265 µmol, 5.5 eq.). Nach 1 h wurden die flüchtigen Bestandteile am Rotationsverdampfer entfernt. Der Rückstand wurde in wenig DMSO gelöst und über präparative HPLC [Methode 9] gereinigt. Man erhielt 17 mg (65% d. Th.) der Titelverbindung.
LC/MS [Methode 3]: Rₜ = 1.19 min; MS [ESIpos]: m/z = 558 (M+H)⁺
¹H-NMR (400 MHz, DMSO-d₆): δ [ppm] = 0.53-0.64 (m, 2H), 0.87-0.95 (m, 2H), 2.70 (s, 3H), 3.18 (tt, 1H), 3.27-3.44 (m, 2H), 4.31-4.42 (m, 2H), 4.74-4.84 (m, 1H), 7.51 (t, 1H), 7.60 (d, 2H), 7.67-7.76 (m, 2H), 7.81 (d, 2H), 7.85 (d, 1H), 7.96 (d, 1H), 8.22 (t, 1H).

### Beispiel 69

### 2-[3-(5-Chlor-2-thienyl)-4-(2-metholyethyl)-5-oxo-4,5-dihydro-1H-1,2,4-triazol-1-yl]-N-{2-[(methylsulfonyl)amino]-2-[2-(trifluormethyl)phenyl]ethyl}acetamid (Racemat)

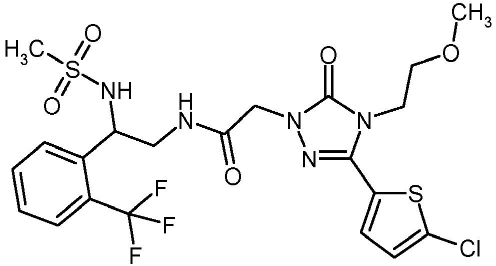

Analog zur Vorschrift von Beispiel 68 wurden 29 mg (46 µmol) der Verbindung aus Beispiel 60A mit Methansulfonsäurechlorid umgesetzt. Man erhielt 25 mg (92% d. Th.) der Titelverbindung.
LC/MS [Methode 3]: Rₜ = 1.18 min; MS [ESIpos]: m/z = 582 (M+H)⁺
¹H-NMR (400 MHz, DMSO-d₆): δ [ppm] = 2.72 (s, 3H), 3.21 (s, 3H), 3.25-3.46 (m, 2H), 3.55 (t, 2H), 3.98 (t, 2H), 4.35-4.45 (m, 2H), 4.75-4.84 (m, 1H), 7.28 (d, 1H), 7.51 (t, 1H), 7.58 (d, 1H), 7.67-7.79 (m, 2H), 7.86 (d, 1H), 7.93-8.05 (m, 1H), 8.30 (t, 1H).

### Beispiel 70

### 2-[3-(5-Chlor-2-thienyl)-4-(2-fluorbenzyl)-5-oxo-4,5-dihydro-1H-1,2,4-triazol-1-yl]-N-{2-[(methylsulfonyl)amino]-2-[2-(trifluormethyl)phenyl]ethyl}acetamid (Racemat)

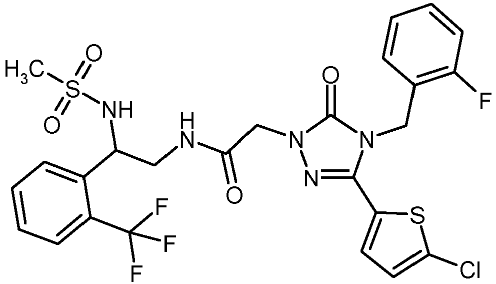

Analog zur Vorschrift von Beispiel 68 wurden 35 mg (52 µmol) der Verbindung aus Beispiel 61A mit Methansulfonsäurechlorid umgesetzt. Man erhielt 14 mg (41% d. Th.) der Titelverbindung.
LC/MS [Methode 3]: Rₜ = 1.33 min; MS [ESIpos]: m/z = 632 (M+H)⁺
¹H-NMR (400 MHz, DMSO-d₆): δ [ppm] = 2.72 (s, 3H), 3.30-3.48 (m, 2H), 4.41-4.53 (m, 2H), 4.76-4.83 (m, 1H), 5.14 (s, 2H), 7.04-7.11 (m, 1H), 7.13-7.28 (m, 4H), 7.31-7.41 (m, 1H), 7.51 (t, 1H), 7.68-7.78 (m, 2H), 7.87 (d, 1H), 8.01 (m, 1H), 8.36 (t, 1H).

### B. Bewertung der pharmakologischen Wirksamkeit

Die pharmakologische Wirkung der erfindungsgemäßen Verbindungen kann in folgenden Assays gezeigt werden:

### Abkürzungen:

- EDTA: Ethylendiamintetraessigsäure
- DMEM: Dulbecco's Modified Eagle Medium
- FCS: Fötales Kälberserum
- HEPES: 4-(2-Hydroxyethyl)-1-piperazinethansulfonsäure
- SmGM: Smooth Muscle Cell Growth Media
- Tris-HCl: 2-Amino-2-(hydrolymethyl)-1,3-propandiol-Hydrochlorid

### B-1. Zellulärer in vitro-Test zur Bestimmung der Vasopressin-Rezeptor-Aktivität

Die Identifizierung von Agonisten und Antagonisten der V1a- und V2-Vasopressin-Rezeptoren von Mensch und Ratte sowie die Quantifizierung der Wirksamkeit der erfindungsgemäßen Verbindungen erfolgt mit Hilfe von rekombinanten Zelllinien. Diese Zellen leiten sich ursprünglich von einer Ovarepithelzelle des Hamsters (Chinese Hamster Ovary, CHO K1, ATCC American Type Culture Collection, Manassas, VA 20108, USA) ab. Die Testzelllinien exprimieren konstitutiv eine modifizierte Form des Calcium-sensitiven Photoproteins Aequorin, das nach Rekonstitution mit dem Co-Faktor Coelenterazin bei Erhöhung der freien Calcium-Konzentrationen Licht emittiert [Rizzuto R, Simpson AW, Brini M, Pozzan T, Nature 358, 325-327 (1992)]. Zusätzlich sind die Zellen stabil transfiziert mit den V1a- oder V2-Rezeptoren des Menschen oder der Ratte. Im Falle der Gskoppelnden V2-Rezeptoren sind die Zellen mit einem weiteren Gen, das für das promiskuitive G_{α16}-Protein kodiert [Amatruda TT, Steele DA, Slepak VZ, Simon MI, Proceedings in the National Academy of Science USA 88, 5587-5591 (1991)], entweder unabhängig oder als Fusionsgen stabil transfiziert. Die resultierenden Vasopressin-Rezeptor-Testzellen reagieren auf Stimulation der rekombinant exprimierten Vasopressin-Rezeptoren mit einer intrazellulären Freisetzung von Calcium-Ionen, die durch die resultierende Aequorin-Lumineszens mit einem geeignetem Luminometer quantifiziert werden kann [Milligan G, Marshall F, Rees S, Trends in Pharmacological Sciences 17, 235-237 (1996)].

### Testablauf:

Die Zellen werden am Tag vor dem Test in Kulturmedium (DMEM, 10% FCS, 2 mM Glutamin, 10 mM HEPES) in 384-Loch-Mikrotiterplatten ausplattiert und in einem Zellinkubator (96% Luftfeuchtigkeit, 5% v/v CO₂, 37°C) gehalten. Am Testtag wird das Kulturmedium durch eine Tyrodelösung (140 mM NaCl, 5 mM KCl, 1 mM MgCl₂, 2 mM CaCl₂, 20 mM Glucose, 20 mM HEPES), das zusätzlich den Co-Faktor Coelenterazin (50 µM) enthält, ausgetauscht und die Mikrotiterplatte anschließend für weitere 3-4 Stunden inkubiert. Die Testsubstanzen werden in verschiedenen Konzentrationen für 10 bis 20 Minuten in den Vertiefungen der Mikrotiterplatte vorgelegt, ehe der Agonist [Arg⁸]-Vasopressin hinzugegeben und das resultierende Lichtsignal sofort im Luminometer gemessen wird. Die Berechnung der IC₅₀-Werte erfolgt mit Hilfe des Computerprogramms GraphPad PRISM (Version 3.02).

In der folgenden Tabelle sind repräsentative IC₅₀-Werte für die erfindungsgemäßen Verbindungen an der mit dem humanen V1a- bzw. V2-Rezeptor transfizierten Zelllinie aufgeführt:

**Tabelle**

| **Beispiel Nr.** | **IC₅₀ hV1a [µM]** | **IC₅₀ hV2 [µM]** |
|---|---|---|
| 2 | 0.001 | 0.016 |
| 6 | 0.012 | 0.001 |
| 9 | 0.063 | 0.005 |
| 10 | 0.024 | 0.009 |
| 13 | 0.004 | 0.070 |
| 19 | 0.013 | 0.011 |
| 25 | 0.008 | 0.005 |
| 33 | 0.017 | 0.008 |
| 36 | 0.019 | 0.013 |
| 39 | 0.025 | 0.010 |
| 42 | 0.008 | 0.003 |
| 47 | 0.005 | 0.002 |
| 52 | 0.007 | 0.002 |
| 53 | 0.004 | 0.015 |
| 57 | 0.010 | 0.022 |
| 59 | 0.014 | 0.048 |
| 66 | 0.051 | 1.2 |
| 67 | 0.018 | 0.30 |
| 69 | 0.13 | 3.5 |

### B-2. Zellulärer in vitro-Test zum Nachweis der Wirkung von Vasopressin-V1a-Rezeptorantagonisten auf die Regulation pro-fibrotischer Gene

Die als Kardiomyozyten-Typ beschriebene Zelllinie H9C2 (American Type Culture Collection ATCC-Nr. CRL-1446), die aus Herzgewebe der Ratte isoliert wurde, exprimiert endogen den Vasopressin-V1a-Rezeptor AVPR1A in hoher Kopienzahl, während die AVPR2-Expression nicht nachweisbar ist. Für Zell-Tests zur Hemmung der AVPR1A-abhängigen Regulation der Genexpression durch Rezeptor-Antagonisten wird wie folgt verfahren:
H9C2-Zellen werden in 1.0 ml Medium Opti-MEM (Invitrogen Corp., Carlsbad CA, USA, Kat.-Nr. 11058-021) mit 2% FCS und 1% Penicillin/Streptomycin-Lösung (Invitrogen, Kat.-Nr. 10378-016) mit einer Zelldichte von 100 000 Zellen/well in 12-well-Mikrotiterplatten für die Zellkultur ausgesät und in einem Zellinkubator (96% Luftfeuchtigkeit, 5% v/v CO₂, 37°C) gehalten. Nach 24 Stunden werden in je drei wells (Triplikate) Vehikel-Lösung (als Negativkontrolle), Vasopressin-Lösung ([Arg⁸]-Vasopressin-Acetat, Fa. Sigma, Kat.-Nr. V9879) oder Testsubstanz (gelöst in Vehikel Wasser mit 20 Vol.-% Ethanol) und Vasopressin-Lösung gegeben. Die finale Vasopressin-Konzentration in der Zellkultur ist 0.05 µM. Die Testsubstanz-Lösung wird in kleinen Volumina zur Zellkultur gegeben, so dass eine finale Konzentration von 0.1% Ethanol im Zelltest nicht überschritten wird. Nach einer Inkubationszeit von 6 Stunden wird der Kulturüberstand abgesaugt, die adhärenten Zellen werden in 250 µl RLT-Puffer (Fa. Qiagen, Ratingen, Kat.-Nr. 79216) lysiert, und die RNA wird aus diesem Lysat mit dem RNeasy-Kit (Qiagen, Kat.-Nr. 74104) isoliert. Anschließend erfolgt der DNAse-Verdau (Invitrogen, Kat.-Nr. 18068-015), die cDNA-Synthese (ImProm-II Reverse Transcription System, Fa. Promega, Kat.-Nr. A3800) und die RTPCR (pPCR MasterMix RT-QP2X-03-075, Fa. Eurogentec, Seraing, Belgien). Alle Prozeduren erfolgen gemäß den Arbeitsprotokollen der Test-Reagenzien-Hersteller. Die Primer-Sets für die RTPCR werden anhand der mRNA-Gensequenzen (NCBI Genbank Entrez Nucleotide Data Base) mit Hilfe des Programms Primer3Plus mit 6-FAM TAMRA-markierten Sonden ausgewählt. Die RTPCR zur Bestimmung der relativen mRNA-Expression in den Zellen der verschiedenen Test-Ansätze erfolgt mit dem Applied Biosystems ABI Prism 7700 Sequence Detector im 96-well- oder 384-well-Mikrotiterplatten-Format gemäß der Betriebsanleitung des Gerätes. Die relative Genexpression wird durch den delta-delta Ct-Wert dargestellt [Fa. Applied Biosystems, User Bulletin No. 2 ABI Prism 7700 SDS December 11, 1997 (update 10/2001)] mit dem Bezug auf die Expressionsstärke des Gens Ribosomales Protein L-32 (Genbank Acc. No. NM_013226) und den Ct-Schwellenwert von 35.

### B-3. In vivo-Test zum Nachweis der kardiovaskulären Wirkung: Blutdruckmessung an narkotisierten Ratten ("Vasopressin-Challenge-Modell")

Bei männlichen Sprague-Dawley-Ratten (250-350 g Körpergewicht) werden in Ketamin/Xylazin/ Pentobarbital-Injektionsnarkose Polyethylenschläuche (PE-50; Intramedic^{®}), die mit Heparinhaltiger (500 I.E./ml) isotoner Natriumchlorid-Lösung vorgefüllt sind, in die *Vena jugularis* und die *Vena femoralis* eingeführt und anschließend eingebunden. Über einen venösen Zugang wird mit Hilfe einer Spritze Arg-Vasopressin injiziert, über den zweiten venösen Zugang wird die Testsubstanz appliziert. Zur Ermittlung des systolischen Blutdrucks wird ein Druckkatheter (Millar SPR-320 2F) in die *A. carotis* eingebunden. Der arterielle Katheter wird an einen Druckwandler angeschlossen, der seine Signale in einen mit einer geeigneten Registrierungssoftware ausgestatteten Messcomputer einspeist. In einem typischen Experiment werden dem Versuchstier 3-4 aufeinanderfolgende Bolusinjektionen im Abstand von 10-15 min mit einer definierten Menge Arg-Vasopressin (30 ng/kg) in isotoner Natriumchlorid-Lösung verabreicht und, nachdem der Blutdruck wieder Ausgangswerte erreicht hat, die zu testende Substanz als Bolus mit anschließender Dauerinfusion in einem geeigneten Lösungsmittel appliziert. Hiernach wird in definierten Abständen (10-15 min) erneut die gleiche Menge Arg-Vasopressin wie zu Anfang verabreicht. Anhand der Blutdruckwerte wird ermittelt, inwieweit die Testsubstanz der blutdrucksteigernden Wirkung des Arg-Vasopressins entgegenwirkt. Kontrolltiere erhalten nur Lösungsmittel statt der Testsubstanz.

Die erfindungsgemäßen Verbindungen bewirken nach intravenöser Applikation im Vergleich zu den Lösungsmittelkontrollen eine Hemmung des durch Arg-Vasopressin verursachten Blutdruckanstiegs.

### B-4. In vivo-Test zum Nachweis der kardiovaskulären Wirkung: Diurese-Untersuchungen an wachen Ratten in Stoffwechselkäfigen

Wistar-Ratten (300-450 g Körpergwicht) werden mit freiem Zugang zu Futter (Altromin) und Trinkwasser gehalten. Während des Versuchs werden die Tiere für 4-8 Stunden einzeln in für Ratten dieser Gewichtsklasse geeigneten Stoffwechselkäfigen (Fa. Tecniplast Deutschland GmbH, D-82383 Hohenpeißenberg) mit freiem Zugang zu Trinkwasser gehalten. Am Versuchsbeginn wird den Tieren die zu prüfende Substanz in einem Volumen von 1-3 ml/kg Körpergewicht eines geeigneten Lösungsmittels mit Hilfe einer Schlundsonde in den Magen verabreicht. Als Kontrolle dienende Tiere erhalten nur Lösungsmittel. Kontrollen und Substanztestungen werden am selben Tag parallel durchgeführt. Kontrollgruppen und Substanzdosisgruppen bestehen aus jeweils 4-8 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschiedenen Natrium- bzw. Kalium-Ionen mittels flammenphotometrischer Standardmethoden gemessen. Um eine ausreichende Urinmenge zu erhalten, wird den Tieren zu Versuchsbeginn per Schlundsonde eine definierte Menge Wasser zugeführt (typischerweise 10 ml pro kg Körpergewicht). Vor Versuchsbeginn und nach Versuchsende wird das Körpergewicht der einzelnen Tiere bestimmt.

Die erfindungsgemäßen Verbindungen bewirken nach oraler Applikation im Vergleich zu Lösungsmittel-Kontrollapplikationen eine gesteigerte Ausscheidung von Urin, die im wesentlichen auf einer gesteigerten Wasserausscheidung (Aquarese) beruht.

### B-5. In vivo-Test zum Nachweis der kardiovaskulären Wirkung: Hämodynamische Untersuchungen an narkotisierten Hunden

Männliche oder weibliche Mischlingshunde (Mongrels, Marshall BioResources, USA) mit einem Gewicht zwischen 20 und 30 kg werden für die operativen Eingriffe und die hämodynamischen und funktionellen Untersuchungstermine jeweils mit Pentobarbital (30 mg/kg i.v., Narcoren^{®}, Fa. Merial, Deutschland) an-narkotisiert. Alcuroniumchlorid (3 mg/Tier i.v., Alloferin^{®}, Fa. ICN Pharmaceuticals, Deutschland) dient dabei zusätzlich als Muskelrelaxans. Die Hunde werden intubiert und mit einem Sauerstoff-Raumluft-Gemisch beatmet (40/60%, etwa 5-6 L/min). Die Beatmung erfolgt mit einem Beatmungsgerät (Fa. Draeger, Sulla 808) und wird überwacht mit einem Kohlendioxid-Analysator (Fa. Engström). Die Narkose wird aufrecht erhalten durch eine ständige Infusion von Pentobarbital (50 µg/kg/min); als Analgetikum dient Fentanyl (10 µg/kg/h). Eine Alternative zu Pentobarbital besteht in der Verwendung von Isofluran (1-2 Vol.-%).

Die Hunde werden in vorbereitenden Eingriffen mit einem Herzschrittmacher ausgestattet. Zum Zeitpunkt 21 Tage vor der ersten Substanz-Testung (= Versuchsbeginn) wird ein Herzschrittmacher (Fa. Biotronik, Logos^{®}) in eine subcutane Hauttasche implantiert und über eine Schrittmacher-Elektrode, die durch die *Vena jugularis externa* unter Durchleuchtung bis in den rechten Ventrikel vorgeschoben wird, mit dem Herzen in Kontakt gebracht.

Zum gleichen Zeitpunkt der Schrittmacher-Implantation erfolgt durch retrogrades Vorschieben einer 7F-Biopsie-Zange (Fa. Cordis) über eine Schleuse (Avanti+^{®}, Fa. Cordis) in der *Arteria femoralis* und nach atraumatischer Passage der Aortenklappe eine definierte Läsion der Mitralklappe unter echokardiographischer und Durchleuchtungskontrolle. Im Anschluss werden alle Zugänge entfernt, und der Hund wacht spontan aus der Narkose auf. Nach 7 weiteren Tagen (= 14 Tage vor der ersten Substanz-Testung) wird der oben beschriebene Schrittmacher aktiviert und das Herz mit einer Frequenz von 220 Schlägen pro Minute stimuliert.

Die eigentlichen Experimente zur Substanz-Testung erfolgen 14 und 28 Tage nach Beginn der Schrittmacher-Stimulation nach folgender Instrumentierung:
- Einführung eines Blasenkatheters zur Blasenentlastung bzw. zur Messung des Urinflusses;
- Anbringung von EKG-Ableitungen an den Extremitäten zur EKG-Messung;
- Einführung eines mit Natriumchlorid-Lösung gefüllten Fluidmedic^{®} PE 300-Schlauches in die *A. femoralis,* welcher mit einem Drucksensor (Fa. Braun Melsungen, Deutschland) verbunden ist zur Messung des systemischen Blutdrucks;
- Einführung eines Millar Tip-Katheters (Typ 350 PC, Fa. Millar Instruments, Houston, USA) über den linken Vorhof oder über eine in der *A. carotis* eingebundene Schleuse zur Messung der Herz-Hämodynamik;
- Einführung eines Swan-Ganz-Katheters (CCOmbo 7.5F, Fa. Edwards, Irvine, USA) über die *V*. *jugularis* in die *A. pulmonalis* zur Messung von Herzzeitvolumen, Sauerstoffsättigung, Pulmonalartiendrücken und zentralvenösem Druck;
- Plazierung einer Braunüle in den *Venae cephalicae* zur Infusion von Pentobarbital, der Flüssigkeitssubstitution und zur Blutentnahme (für die Bestimmung der Substanz-Plasmaspiegel oder sonstiger klinischer Blutwerte);
- Plazierung einer Braunüle in der *Venae saphenae* zur Infusion von Fentanyl und zur Substanzapplikation;
- Infusion von Vasopressin (Fa. Sigma) in aufsteigender Dosierung bis zu einer Dosis von 4 mU/kg/min; unter dieser Dosierung erfolgt dann die Testung pharmakologischer Substanzen.

Die Primärsignale werden eventuell verstärkt (Gould-Verstärker, Fa. Gould Instrument Systems, Valley View, USA bzw. Edwards-Vigilance-Monitor, Fa. Edwards, Irvine, USA) und anschließend zur Auswertung in das Ponemah-System (DataSciences Inc., Minneapolis, USA) eingespeist. Die Signale werden kontinuierlich über den gesamten Versuchszeitraum aufgezeichnet, von dieser Software digital weiterarbeitet und über 30 Sekunden gemittelt.

### C. Ausführunssbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 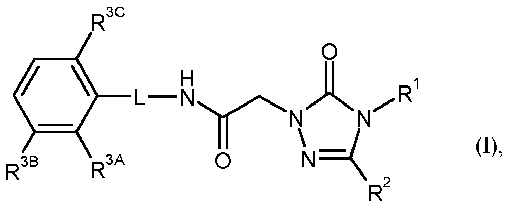 in welcher
R¹ für (C₁-C₆)-Alkyl, (C₂-C₆)-Alkenyl oder (C₂-C₆)-Alkinyl steht, welche jeweils ein- oder zweifach, gleich oder verschieden, mit Resten ausgewählt aus der Reihe Fluor, Chlor, Cyano, Difluormethyl, Trifluormethyl, Oxo, Hydroxy, Difluormethoxy, Trifluormethoxy, (C₁-C₄)-Alkoxy, (C₃-C₆)-Cycloalkyl und Phenyl substituiert sein können,
wobei (C₃-C₆)-Cycloalkyl bis zu zweifach, gleich oder verschieden, mit Resten ausgewählt aus der Reihe Fluor, Trifluormethyl, (C₁-C₄)-Alkyl, Oxo, Hydroxy, Trifluormethoxy und (C₁-C₄)-Alkoxy substituiert sein kann
und
wobei Phenyl bis zu dreifach, gleich oder verschieden, mit Resten ausgewählt aus der Reihe Halogen, Cyano, Difluormethyl, Trifluormethyl, (C₁-C₄)-Alkyl, Hydroxy, Hydroxymethyl, Difluormethoxy, Trifluormethoxy, (C₁-C₄)-Alkoxy, (C₁-C₄)-Alkoxymethyl, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
oder
für (C₃-C₆)-Cycloalkyl steht, welches ein- oder zweifach, gleich oder verschieden, mit Resten ausgewählt aus der Reihe Fluor, Trifluormethyl, (C₁-C₄)-Alkyl, Oxo, Hydroxy, Trifluormethoxy und (C₁-C₄)-Alkoxy substituiert sein kann,
R² für Phenyl oder Thienyl steht, welche ein- oder zweifach, gleich oder verschieden, mit Resten ausgewählt aus der Reihe Halogen, Cyano, Difluormethyl, Trifluormethyl, (C₁-C₄)-Alkyl, Hydroxy, Trifluormethoxy und (C₁-C₄)-Alkoxy substituiert sein können,
R^{3A}, R^{3B} und R^{3C} unabhängig voneinander für Wasserstoff, Fluor, Chlor, Difluormethyl, Trifluormethyl, (C₁-C₄)-Alkyl, Difluormethoxy, Trifluormethoxy oder (C₁-C₄)-Alkoxy stehen,
wobei jedoch mindestens einer der Reste R^{3A}, R^{3B}, R^{3C} von Wasserstoff verschieden ist,
und
L für eine Gruppe der Formel 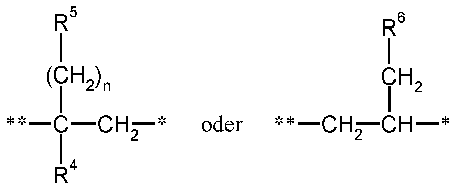 steht, worin
* die Verknüpfungsstelle mit dem angrenzenden N-Atom
und
** die Verknüpfungsstelle mit dem Phenyl-Ring kennzeichnen,
n die Zahl 0, 1 oder 2 bedeutet,
R⁴ Wasserstoff oder Methyl bedeutet,
R⁵ eine Gruppe der Formel -O-C(=O)-NR^{7A}R^{7B}, -NR⁸-C(=O)-NR^{7A}R^{7B}, -NR⁸-SO₂-NR^{7A}R^{7B}, -NR⁸-C(=O)-R⁹, -NR⁸-SO₂-R¹⁰ oder -NR⁸-C(=O)-OR¹⁰ bedeutet, worin
R^{7A} und R^{7B} unabhängig voneinander Wasserstoff, (C₁-C₆)-Alkyl oder (C₃-C₆)-Cycloalkyl darstellen oder zusammen mit dem Stickstoffatom, an das sie beide gebunden sind, einen 4- bis 6-gliedrigen Heterocyclus bilden, der ein weiteres Ring-Heteroatom aus der Reihe N, O oder S enthalten kann und der ein- oder zweifach, gleich oder verschieden, mit Resten ausgewählt aus der Reihe Fluor, Trifluormethyl, (C₁-C₄)-Alkyl, Hydroxy und Oxo substituiert sein kann,
R⁸ Wasserstoff oder (C₁-C₄)-Alkyl darstellt,
R⁹ Wasserstoff, (C₁-C₆)-Alkyl oder (C₃-C₆)-Cycloalkyl darstellt,
und
R¹⁰ (C₁-C₆)-Alkyl oder (C₃-C₆)-Cycloalkyl darstellt,
und
R⁶ die oben angegebene Bedeutung von R⁵ hat oder Hydroxy bedeutet,
sowie ihre Salze, Solvate und Solvate der Salze.

2. Verbindung der Formel (I) nach Anspruch 1, in welcher
R¹ für (C₁-C₄)-Alkyl oder (C₂-C₄)-Alkenyl steht, welche jeweils ein- oder zweifach, gleich oder verschieden, mit Resten ausgewählt aus der Reihe Fluor, Trifluormethyl, Hydroxy, Methoxy und Ethoxy substituiert sein können,
oder
für Benzyl steht, welches im Phenylring mit einem Rest ausgewählt aus der Reihe Fluor, Chlor, Methyl, Trifluormethyl und Methoxy substituiert sein kann,
oder
für Cyclopropyl steht,
R² für Phenyl oder Thienyl steht, welche mit einem Rest ausgewählt aus der Reihe Fluor und Chlor substituiert sind,
R^{3A} und R^{3B} unabhängig voneinander für Wasserstoff, Fluor, Chlor, Methyl, Trifluormethyl, Methoxy oder Trifluormethoxy stehen,
wobei jedoch mindestens einer der Reste R^{3A} und R^{3B} von Wasserstoff verschieden ist,
R^{3C} für Wasserstoff steht,
und
L für eine Gruppe der Formel 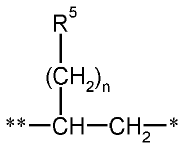 steht, worin
* die Verknüpfungsstelle mit dem angrenzenden N-Atom
und
** die Verknüpfungsstelle mit dem Phenyl-Ring kennzeichnen,
n die Zahl 0 oder 1 bedeutet,
und
R⁵ eine Gruppe der Formel -O-C(=O)-NHR^{7B}, -NH-C(=O)-NHR^{7B}, -NH-C(=O)-R⁹, -NH-SO₂-R¹⁰ oder -NH-C(=O)-OR¹⁰ bedeutet, worin
R^{7B} Wasserstoff oder (C₁-C₄)-Alkyl darstellt,
R⁹ Wasserstoff oder (C₁-C₄)-Alkyl darstellt,
und
R¹⁰ (C₁-C₄)-Alkyl darstellt,
sowie ihre Salze, Solvate und Solvate der Salze.

3. Verbindung der Formel (I) nach Anspruch 1 oder 2, in welcher
R¹ für 3,3,3-Trifluor-2-hydroxypropyl, 3,3,3-Trifluorpropyl oder 3,3,3-Trifluorprop-1-en-1-yl steht,
R² für *p*-Chlorphenyl steht,
R^{3A} und R^{3B} unabhängig voneinander für Wasserstoff, Chlor oder Trifluormethyl stehen,
wobei jedoch mindestens einer der Reste R^{3A} und R^{3B} von Wasserstoff verschieden ist,
R^{3C} für Wasserstoff steht,
und
L für eine Gruppe der Formel 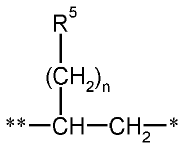 steht, worin
* die Verknüpfungsstelle mit dem angrenzenden N-Atom
und
** die Verknüpfungsstelle mit dem Phenyl-Ring kennzeichnen,
n die Zahl 0 oder 1 bedeutet,
und
R⁵ eine Gruppe der Formel -O-C(=O)-NH₂, -NH-C(=O)-NH₂ oder -NH-SO₂-R¹⁰ bedeutet, worin
R¹⁰ Methyl oder Ethyl darstellt,
sowie ihre Salze, Solvate und Solvate der Salze.

4. Verfahren zur Herstellung von Verbindungen der Formel (I), wie in den Ansprüchen 1 bis 3 definiert, **dadurch gekennzeichnet, dass** man eine Verbindung der Formel (II) 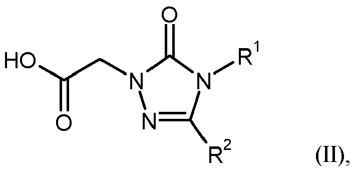 in welcher R¹ und R² die in den Ansprüchen 1 bis 3 angegebenen Bedeutungen haben,
in einem inerten Lösungsmittel unter Aktivierung der Carbonsäure-Funktion mit einer Verbindung der Formel (III) 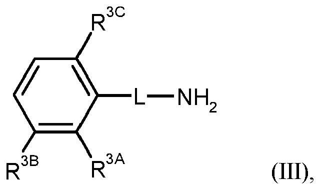 in welcher L, R^{3A}, R^{3B} und R^{3C} die in den Ansprüchen 1 bis 3 angegebenen Bedeutungen haben,
kuppelt und die so erhaltenen Verbindungen der Formel (I) gegebenenfalls in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

5. Verbindung der Formel (I), wie in einem der Ansprüche 1 bis 3 definiert, zur Behandlung und/oder Prävention von Krankheiten.

6. Verbindung der Formel (I), wie in einem der Ansprüche 1 bis 3 definiert, zur Verwendung in einem Verfahren zur Behandlung und/oder Prävention von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

7. Verwendung einer Verbindung der Formel (I), wie in einem der Ansprüche 1 bis 3 definiert, zur Herstellung eines Arzneimittels zur Behandlung und/oder Prävention von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

8. Arzneimittel enthaltend eine Verbindung der Formel (I), wie in einem der Ansprüche 1 bis 3 definiert, in Kombination mit einem oder mehreren inerten, nicht-toxischen, pharmazeutisch geeigneten Hilfsstoffen.

9. Arzneimittel enthaltend eine Verbindung der Formel (I), wie in einem der Ansprüche 1 bis 3 definiert, in Kombination mit einem oder mehreren weiteren Wirkstoffen ausgewählt aus der Gruppe bestehend aus Diuretika, Angiotensin All-Antagonisten, ACE-Hemmer, beta-Rezeptoren-Blocker, Mineralocorticoid-Rezeptor-Antagonisten, organische Nitrate, NO-Donatoren und positiv-inotrop wirksame Substanzen.

10. Arzneimittel nach Anspruch 8 oder 9 zur Behandlung und/oder Prävention von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

## Claims

1. Compound of the formula (I) 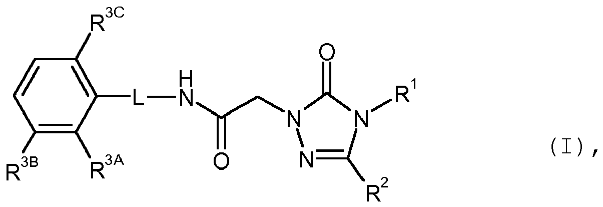 in which
R¹ represents (C₁-C₆)-alkyl, (C₂-C₆)-alkenyl or (C₂-C₆)-alkynyl, each of which may be mono- or disubstituted by identical or different radicals selected from the group consisting of fluorine, chlorine, cyano, difluoromethyl, trifluoromethyl, oxo, hydroxy, difluoromethoxy, trifluoromethoxy, (C₁-C₄)-alkoxy, (C₃-C₆)-cycloalkyl and phenyl,
where (C₃-C₆)-cycloalkyl may be substituted up to two times by identical or different radicals selected from the group consisting of fluorine, trifluoromethyl, (C₁-C₄)-alkyl, oxo, hydroxy, trifluoromethoxy and (C₁-C₄)-alkoxy
and
where phenyl may be substituted up to three times by identical or different radicals selected from the group consisting of halogen, cyano, difluoromethyl, trifluoromethyl, (C₁-C₄)-alkyl, hydroxy, hydroxymethyl, difluoromethoxy, trifluoromethoxy, (C₁-C₄)-alkoxy, (C₁-C₄)-alkoxymethyl, hydroxycarbonyl, (C₁-C₄)-alkoxycarbonyl, aminocarbonyl, mono-(C₁-C₄)-alkylaminocarbonyl and di-(C₁-C₄)-alkyl-aminocarbonyl,
or
represents (C₃-C₆)-cycloalkyl which may be mono- or disubstituted by identical or different radicals selected from the group consisting of fluorine, trifluoromethyl, (C₁-C₄)-alkyl, oxo, hydroxy, trifluoromethoxy and (C₁-C₄)-alkoxy,
R² represents phenyl or thienyl which may be mono- or disubstituted by identical or different radicals selected from the group consisting of halogen, cyano, difluoromethyl, trifluoromethyl, (C₁-C₄)-alkyl, hydroxy, trifluoromethoxy and (C₁-C₄)-alkoxy,
R^{3A}, R^{3B} and R^{3C} independently of one another represent hydrogen, fluorine, chlorine, difluoromethyl, trifluoromethyl, (C₁-C₄)-alkyl, difluoromethoxy, trifluoromethoxy or (C₁-C₄)-alkoxy,
but where at least one of the radicals R^{3A}, R^{3B}, R^{3C} is different from hydrogen,
and
L represents a group of the formula 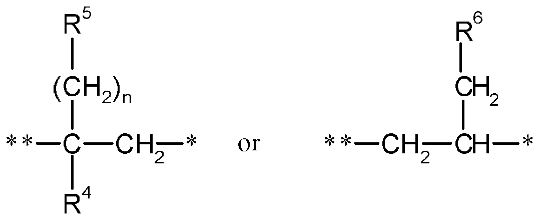 in which
* represents the point of attachment to the adjacent nitrogen atom
and
** represents the point of attachment to the phenyl ring,
n represents the number 0, 1 or 2,
R⁴ represents hydrogen or methyl,
R⁵ represents a group of the formula -O-C (=O) -NR^{7A}R^{7B}, -NR⁸-C (=O) -NR^{7A}R^{7B}, -NR⁸-SO₂-NR^{7A}R^{7B}, -NR⁸-C (=O)-R⁹, -NR⁸-SO₂-R¹⁰ or -NR⁸-C (=O) -OR¹⁰ in which
R^{7A} and R^{7B} independently of one another represent hydrogen, (C₁-C₆)-alkyl or (C₃-C₆)-cycloalkyl or together with the nitrogen atom to which both are attached form a 4- to 6-membered heterocycle which may contain a further ring heteroatom from the group consisting of N, O and S and which may be mono- or disubstituted by identical or different radicals selected from the group consisting of fluorine, trifluoromethyl, (C₁-C₄)-alkyl, hydroxy and oxo,
R⁸ represents hydrogen or (C₁-C₄)-alkyl,
R⁹ represents hydrogen, (C₁-C₆)-alkyl or (C₃-C₆)-cycloalkyl,
and
R¹⁰ represents (C₁-C₆)-alkyl or (C₃-C₆)-cycloalkyl,
and
R⁶ has the meaning of R⁵ given above or represents hydroxy,
and its salts, solvates and solvates of the salts.

2. Compound of the formula (I) according to Claim 1 in which
R¹ represents (C₁-C₄)-alkyl or (C₂-C₄)-alkenyl, each of which may be mono- or disubstituted by identical or different radicals selected from the group consisting of fluorine, trifluoromethyl, hydroxy, methoxy and ethoxy,
or
represents benzyl which may be substituted in the phenyl ring by a radical selected from the group consisting of fluorine, chlorine, methyl, trifluoromethyl and methoxy,
or
represents cyclopropyl,
R² represents phenyl or thienyl which are substituted by a radical selected from the group consisting of fluorine and chlorine,
R^{3A} and R^{3B} independently of one another represent hydrogen, fluorine, chlorine, methyl, trifluoromethyl, methoxy or trifluoromethoxy,
but where at least one of the radicals R^{3A} and R^{3B} is different from hydrogen,
R^{3C} represents hydrogen,
and
L represents a group of the formula 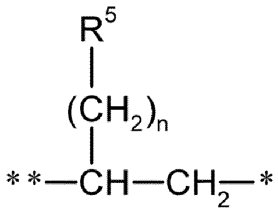 in which
* represents the point of attachment to the adjacent nitrogen atom
and
** represents the point of attachment to the phenyl ring,
n represents the number 0 or 1,
and
R⁵ represents a group of the formula -O-C (=O) -NHR^{7B}, -NH-C (=O)-NHR^{7B}, -NH-C(=O)-R⁹, -NH-SO₂-R¹⁰ or -NH-C(=O)-OR¹⁰ in which
R^{7B} represents hydrogen or (C₁-C₄)-alkyl,
R⁹ represents hydrogen or (C₁-C₄)-alkyl,
and
R¹⁰ represents (C₁-C₄)-alkyl,
and its salts, solvates and solvates of the salts.

3. Compound of the formula (I) according to Claim 1 or 2 in which
R¹ represents 3,3,3-trifluoro-2-hydroxypropyl, 3,3,3-trifluoropropyl or 3,3,3-trifluoroprop-1-en-1-yl,
R² represents *p*-chlorophenyl,
R^{3A} and R^{3B} independently of one another represent hydrogen, chlorine or trifluoromethyl,
but where at least one of the radicals R^{3A} and R^{3B} is different from hydrogen,
R^{3C} represents hydrogen,
and
L represents a group of the formula 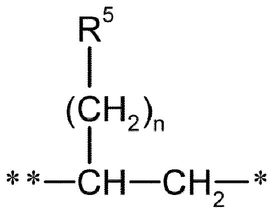 in which
* represents the point of attachment to the adjacent nitrogen atom
and
** represents the point of attachment to the phenyl ring,
n represents the number 0 or 1,
and
R⁵ represents a group of the formula -O-C(=O)-NH₂, -NH-C(=O)-NH₂ or -NH-SO₂-R¹⁰ in which
R¹⁰ represents methyl or ethyl,
and its salts, solvates and solvates of the salts.

4. Process for preparing compounds of the formula (I) as defined in Claims 1 to 3, **characterized in that** a compound of the formula (II) 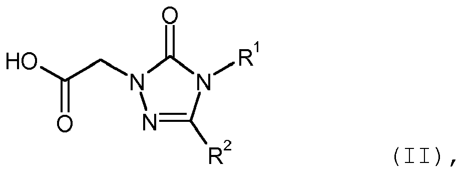 in which R¹ and R² have the meanings given in Claims 1 to 3,
is coupled in an inert solvent with activation of the carboxylic acid function with a compound of the formula (III) 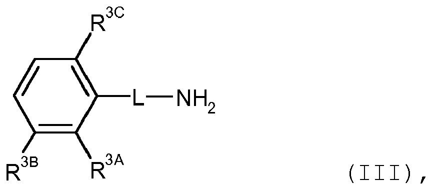 in which L, R^{3A}, R^{3B} and R^{3C} have the meanings given in Claims 1 to 3,
and the resulting compounds of the formula (I) are optionally separated into their enantiomers and/or diastereomers and/or converted with the appropriate (i) solvents and/or (ii) acids or bases into their solvates, salts and/or solvates of the salts.

5. Compound of the formula (I) as defined in any of Claims 1 to 3 for the treatment and/or prevention of diseases.

6. Compound of the formula (I) as defined in any of Claims 1 to 3 for use in a method for the treatment and/or prevention of acute and chronic heart failure, hypervolaemic and euvolaemic hyponatraemia, cirrhosis of the liver, ascites, oedema and the syndrome of inadequate ADH secretion (SIADH).

7. Use of a compound of the formula (I) as defined in any of Claims 1 to 3 for preparing a medicament for the treatment and/or prevention of acute and chronic heart failure, hypervolaemic and euvolaemic hyponatraemia, cirrhosis of the liver, ascites, oedema and the syndrome of inadequate ADH secretion (SIADH).

8. Medicament comprising a compound of the formula (I) as defined in any of Claims 1 to 3 in combination with an one or more inert non-toxic pharmaceutically suitable auxiliaries.

9. Medicament comprising a compound of the formula (I) as defined in any of Claims 1 to 3 in combination with one or more further active substances selected from the group consisting of diuretics, angiotensin All antagonists, ACE inhibitors, beta-receptor blockers, mineralocorticoid receptor antagonists, organic nitrates, NO donors and positive-inotropic active substances.

10. Medicament according to Claim 8 or 9 for the treatment and/or prevention of acute and chronic heart failure, hypervolaemic and euvolaemic hyponatraemia, cirrhosis of the liver, ascites, oedema and the syndrome of inadequate ADH secretion (SIADH).

## Revendications

1. Composé de formule (I) 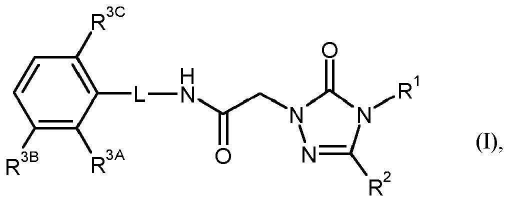 dans laquelle
R¹ représente (C₁-C₆)-alkyle, (C₂-C₆)-alcényle ou (C₂-C₆)-alcynyle, qui peuvent être à chaque fois monosubstitués ou disubstitués, de manière identique ou différente, par des radicaux choisis dans la série fluor, chlore, cyano, difluorométhyle, trifluorométhyle, oxo, hydroxy, difluorométhoxy, trifluorométhoxy, (C₁-C₄)-alcoxy, (C₃-C₆)-cycloalkyle et phényle,
(C₃-C₆)-cycloalkyle pouvant être jusqu'à disubstitué, de manière identique ou différente, par des radicaux choisis dans la série fluor, trifluorométhyle, (C₁-C₄)-alkyle, oxo, hydroxy, trifluorométhoxy et (C₁-C₄)-alcoxy et
phényle pouvant être jusqu'à trisubstitué, de manière identique ou différente, par des radicaux choisis dans la série halogène, cyano, difluorométhyle, trifluorométhyle, (C₁-C₄)-alkyle, hydroxy, hydroxyméthyle, difluorométhoxy, trifluorométhoxy, (C₁-C₄)-alcoxy, (C₁-C₄)-alcoxyméthyle, hydroxycarbonyle, (C₁-C₄)-alcoxycarbonyle, aminocarbonyle, mono-(C₁-C₄)-alkylaminocarbonyle et di-(C₁-C₄)-alkylaminocarbonyle, ou
représente (C₃-C₆)-cycloalkyle, qui peut être monosubstitué ou disubstitué, de manière identique ou différente, par des radicaux choisis dans la série fluor, trifluorométhyle, (C₁-C₄)-alkyle, oxo, hydroxy, trifluorométhoxy et (C₁-C₄)-alcoxy et
R² représente phényle ou thiényle, qui peuvent être monosubstitués ou disubstitués, de manière identique ou différente, par des radicaux choisis dans la série halogène, cyano, difluorométhyle, trifluorométhyle, (C₁-C₄)-alkyle, hydroxy, trifluorométhoxy et (C₁-C₄)-alcoxy,
R^{3A}, R^{3B} et R^{3C} représentent, indépendamment les uns des autres, hydrogène, fluor, chlore, difluorométhyle, trifluorométhyle, (C₁-C₄)-alkyle, difluorométhoxy, trifluorométhoxy ou (C₁-C₄)-alcoxy, où cependant au moins un des radicaux R^{3A}, R^{3B}, R^{3C} est différent d'hydrogène,
et
L représente un groupe de formule 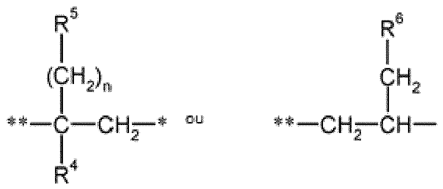 dans laquelle
* représente le site de liaison avec l'atome N adjacent et
** représente le site de liaison avec le cycle phényle,
n vaut le nombre 0, 1 ou 2,
R⁴ signifie hydrogène ou méthyle,
R⁵ signifie un groupe de formule -O-C(=O)-NR^{7A}R^{7B}, -NR⁸-C (=O) -NR^{7A}R^{7B}, -NR⁸-SO₂-NR^{7A}R^{7B}, -NR⁸-C (=O)-R⁹, -NR⁸-SO₂-R¹⁰ ou -NR⁸-C (=O)-OR¹⁰, dans laquelle
R^{7A} et R^{7B} représentent, indépendamment l'un de l'autre, hydrogène, (C₁-C₆)-alkyle ou (C₃-C₆)-cycloalkyle ou, ensemble avec l'atome d'azote auquel ils sont tous les deux liés, un hétérocycle de 4 à 6 chaînons, qui peut contenir un autre hétéroatome de cycle de la série N, O ou S et qui peut être monosubstitué ou disubstitué, de manière identique ou différente, par des radicaux choisis dans la série fluor, trifluorométhyle, (C₁-C₄)-alkyle, hydroxy et oxo,
R⁸ représente hydrogène ou (C₁-C₄)-alkyle,
R⁹ représente hydrogène, (C₁-C₆)-alkyle ou (C₃-C₆)-cycloalkyle, et
R¹⁰ représente (C₁-C₆)-alkyle ou (C₃-C₆)-cycloalkyle,
et
R⁶ présente la signification indiquée ci-dessus de R⁵ ou hydroxy,
ainsi que ses sels, solvates et solvates des sels.

2. Composé de formule (I) selon la revendication 1, dans laquelle
R¹ représente (C₁-C₄)-alkyle ou (C₂-C₄)-alcényle, qui peuvent être à chaque fois monosubstitués ou disubstitués, de manière identique ou différente, par des radicaux choisis dans la série fluor, trifluorométhyle, hydroxy, méthoxy et éthoxy, ou représente benzyle, qui peut être substitué, dans le cycle phényle, par un radical choisi dans la série fluor, chlore, méthyle, trifluorométhyle et méthoxy, ou
représente cyclopropyle,
R² représente phényle ou thiényle, qui sont substitués par un radical choisi dans la série fluor et chlore,
R^{3A} et R^{3B} représentent, indépendamment l'un de l'autre, hydrogène, fluor, chlore, méthyle, trifluorométhyle, méthoxy ou trifluorométhoxy, où cependant au moins un des radicaux R^{3A} et R^{3B} est différent d'hydrogène,
R^{3C} représente hydrogène et
L représente un groupe de formule 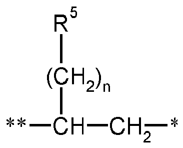 dans laquelle
* représente le site de liaison avec l'atome N adjacent et
** représente le site de liaison avec le cycle phényle,
n vaut le nombre 0 ou 1, et
R⁵ signifie un groupe de formule -O-C(=O)-NHR^{7B}, -NH-C(=O)-NHR^{7B}, -NH-C(=O)-R⁹, -NH-SO₂-R¹⁰ ou -NH-C(=O)-OR¹⁰, dans laquelle
R^{7B} représente hydrogène ou (C₁-C₄) -alkyle,
R⁹ représente hydrogène ou (C₁-C₄)-alkyle, et
R¹⁰ représente (C₁-C₄) -alkyle,
ainsi que ses sels, solvates et solvates des sels.

3. Composé de formule (I) selon la revendication 1 ou 2, dans laquelle
R¹ représente 3,3,3-trifluoro-2-hydroxypropyle, 3,3,3-trifluoropropyle ou 3,3,3-trifluoroprop-1-én-1-yle,
R² représente *p*-chlorophényle,
R^{3A} et R^{3B} représentent, indépendamment l'un de l'autre, hydrogène, chlore ou trifluorométhyle,
où cependant au moins un des radicaux R^{3A} et R^{3B} est différent d'hydrogène,
R^{3C} représente hydrogène et
L représente un groupe de formule 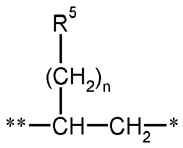 dans laquelle
* représente le site de liaison avec l'atome N adjacent et
** représente le site de liaison avec le cycle phényle,
n vaut le nombre 0 ou 1, et
R⁵ signifie un groupe de formule -O-C(=O)-NH₂, -NH-C(=O)-NH₂ ou -NH-SO₂-R¹⁰, dans laquelle
R¹⁰ représente méthyle ou éthyle,
ainsi que ses sels, solvates et solvates des sels.

4. Procédé pour la préparation de composés de formule (I) tels que définis dans les revendications 1 à 3, **caractérisé en ce qu'**on couple un composé de formule (II) 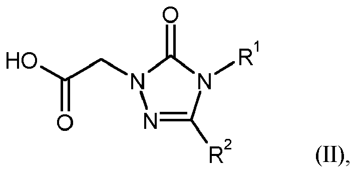 dans laquelle R¹ et R² présentent les significations indiquées dans les revendications 1 à 3,
dans un solvant inerte, en activant la fonction acide carboxylique, avec un composé de formule (III) 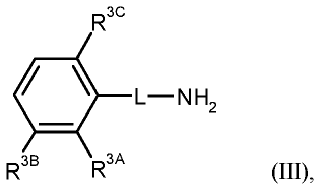 dans laquelle L, R^{3A}, R^{3B} et R^{3C} présentent à chaque fois les significations indiquées dans les revendications 1 à 3,
et on sépare les composés de formule (I) ainsi obtenus le cas échéant en leurs énantiomères et/ou diastéréoisomères et/ou on les transforme avec (i) des solvants et/ou (ii) des bases ou acides correspondants en leurs solvates, sels et/ou solvates des sels.

5. Composé de formule (I), tel que défini dans l'une quelconque des revendications 1 à 3, destiné au traitement et/ou à la prévention de maladies.

6. Composé de formule (I), tel que défini dans l'une quelconque des revendications 1 à 3, pour une utilisation dans un procédé pour le traitement et/ou la prévention de l'insuffisance cardiaque aiguë et chronique, de l'hyponatrémie hypervolémique et euvolémique, d'une cirrhose du foie, des ascites, des oedèmes et du syndrome d'une sécrétion inadéquate d'ADH (SIADH).

7. Utilisation d'un composé de formule (I), tel que défini dans l'une quelconque des revendications 1 à 3, pour la préparation d'un médicament destiné au traitement et/ou à la prévention de l'insuffisance cardiaque aiguë et chronique, de l'hyponatrémie hypervolémique et euvolémique, d'une cirrhose du foie, des ascites, des oedèmes et du syndrome d'une sécrétion inadéquate d'ADH (SIADH).

8. Médicament contenant un composé de formule (I), tel que défini dans l'une quelconque des revendications 1 à 3, en combinaison avec un ou plusieurs adjuvants inertes, non toxiques, pharmaceutiquement appropriés.

9. Médicament contenant un composé de formule (I), tel que défini dans l'une quelconque des revendications 1 à 3, en combinaison avec une ou plusieurs autres substances actives choisies dans le groupe constitué par les diurétiques, les antagonistes de l'angiotensine AII, les inhibiteurs d'ACE, les bloquants des récepteurs bêta, les antagonistes des récepteurs des minéralocorticoïdes, les nitrates organiques, les donneurs de NO et les substances à activité inotrope positive.

10. Médicament selon la revendication 8 ou 9 pour le traitement et/ou la prévention de l'insuffisance cardiaque aiguë et chronique, de l'hyponatrémie hypervolémique et euvolémique, de la cirrhose du foie, des ascites, des oedèmes et du syndrome d'une sécrétion inadéquate d'ADH (SIADH).
